(19) Europäisches Patentamt
European Patent Office
Office européen des brevets

(11) Veröffentlichungsnummer: **0 377 893 B1**

(12) # EUROPÄISCHE PATENTSCHRIFT

(45) Veröffentlichungstag der Patentschrift: **06.04.94**

(51) Int. Cl.[5]: **C07D 207/38**, C07D 405/12, A01N 43/36

(21) Anmeldenummer: **89123895.8**

(22) Anmeldetag: **23.12.89**

(54) **3-Aryl-pyrrolidin-2,4-dion-Derivate.**

(30) Priorität: **07.01.89 DE 3900301**
**18.08.89 DE 3927222**

(43) Veröffentlichungstag der Anmeldung:
**18.07.90 Patentblatt 90/29**

(45) Bekanntmachung des Hinweises auf die Patenterteilung:
**06.04.94 Patentblatt 94/14**

(84) Benannte Vertragsstaaten:
**BE CH DE ES FR GB IT LI NL**

(56) Entgegenhaltungen:
**EP-A- 0 262 399**

**LIEBIGS ANN. CHEM., 1985, Seiten 1095-1098, VCH Verlagsgesellschaft mbH,Weinheim, DE; R. SCHMIERER et al.: "Cyclisierung von N-Acylalanin- und N-Acylglycinestern"**

(73) Patentinhaber: **BAYER AG**

**D-51368 Leverkusen(DE)**

(72) Erfinder: **Fischer, Reiner, Dr.**
**Nelly-Sachs-Strasse 23**
**D-4019 Monheim(DE)**
Erfinder: **Baasner, Bernd, Dr.**
**Wagner-Strasse 23**
**D-5060 Bergisch-Gladbach 2(DE)**
Erfinder: **Hagemann, Hermann, Dr.**
**Kandinsky-Strasse 52**
**D-5090 Leverkusen 1(DE)**
Erfinder: **Krebs, Andreas, Dr.**
**Im Gartenfeld 70**
**D-5068 Odenthal-Holz(DE)**
Erfinder: **Marhold, Albrecht, Dr.**
**Carl-Duisberg-Strasse 329**
**D-5090 Leverkusen(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Lürssen, Klaus, Dr.**
**August-Kierspel-Strasse 145**
**D-5060 Bergisch-Gladbach 2(DE)**
Erfinder: **Becker, Benedikt, Dr.**
**Steinmannhof**
**I-39050 Pineta di Laives Bolzano(IT)**
Erfinder: **Schaller, Klaus, Dr.**
**Am Sonnenschein 38**
**D-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Strang, Harry, Dr.**
**Heiderweg 53**
**D-4000 Düsseldorf 31(DE)**

## Beschreibung

Die Erfindung betrifft neue 3-Aryl-pyrrolidin-2,4-dion-Derivate, mehrere Verfahren zu ihrer Herstellung und ihre Verwendung als Insektizide, Akarizide und Herbizide.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenyl-pyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist.

Es wurden nun neue 3-Aryl-pyrrolidin-2,4-dion-Derivate gefunden, die durch die Formel (I) dargestellt sind,

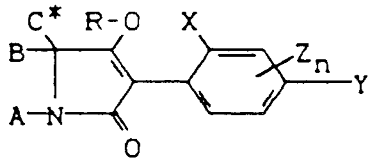

(I)

in welcher

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für 0 oder 1 steht,

R für Wasserstoff (Ia) oder für die Gruppen der Formel

$-CO-R^1$ (Ib) oder $-CO-O-R^2$ (Ic)

steht, in welchen

$R^1$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,
für gegebenenfalls durch Halogen-, Nitro-, $C_1$-$C_6$-Alkyl-, $C_1$-$C_6$-Alkoxy-, $C_1$-$C_6$-Halogenalkyl-, $C_1$-$C_6$-Halogenalkoxy-substituiertes Phenyl;
für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht,

$R^2$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen-, Nitro-, $C_1$-$C_6$-Alkyl-, $C_1$-$C_6$-Alkoxy-, $C_1$-$C_6$-Halogenalkylsubstituiertes Phenyl oder Cycloalkyl mit 3-8 Ringatomen steht,

A für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann oder gegebenenfalls durch Halogen, $C_1$-$C_6$-Alkyl-$C_1$-$C_6$-Haloalkyl-, $C_1$-$C_6$-Alkoxy-, Nitro substituiertes Aryl-$C_1$-$C_6$-alkyl steht,

B, C unabhängig voneinander für Wasserstoff, geradkettiges oder verzweigtes $C_1$-$C_{12}$-Alkyl, $C_1$-$C_8$-Alkoxyalkyl steht,
sowie die enantiomerenreinen Formen von Verbindungen der Formel (I).

Im folgenden seien die folgenden Untergruppen definiert:

(Ia): Verbindungen der Formel (I) worin R = Wasserstoff,

(Ib): Verbindungen der Formel (I) worin R = $COR^1$,

(Ic): Verbindungen der Formel (I) worin R = $COOR^2$,

Weiterhin wurde gefunden, daß man 3-Aryl-pyrrolidin-2,4-dione bzw. deren Enole der Formel (Ia)

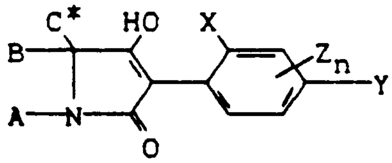

(Ia)

in welcher A, B, C*, X, Y, Z und n die oben angegebene Bedeutung haben, erhält, wenn man

(A)

N-Acylaminosäureester der Formel (II)

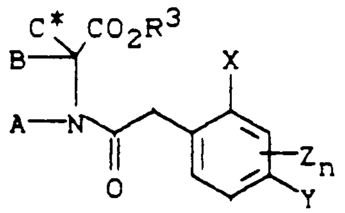

(II)

in welcher
A, B, C*, X, Y, Z und n die oben angegebene Bedeutung haben
und

$R^3$ für Alkyl steht,

in Gegenwart eines Verdünnungsmittels und in Gegenwart einer Base intramolekular kondensiert.

(B)

Außerdem wurde gefunden, daß man Verbindungen der Formel (Ib)

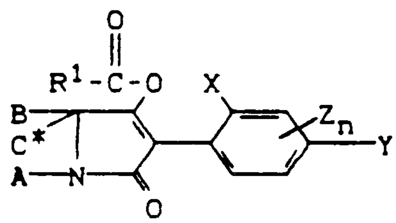

(Ib)

in welcher A, B, C, X, Y, Z, $R^1$ und n die oben angegebene Bedeutung haben, erhält, wenn man Verbindungen der Formel (Ia),

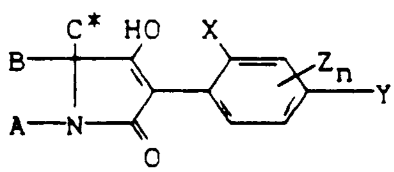

(Ia)

in welcher
A, B, C*, X, Y, Z und n die oben angegebene Bedeutung haben,

α) mit Säurehalogeniden der allgemeinen Formel (III)

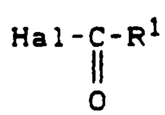

(III)

in welcher

$R^1$ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels,
oder
β) mit Carbonsäureanhydriden der allgemeinen Formel (IV)

$R^1$-CO-O-CO-$R^1$ (IV)

in welcher
$R^1$ die oben angegebene Bedeutung hat,
gegebenenfalls in Gegenwart eines Verdünnungsmittels und gegebenenfalls in Gegenwart eines Säurebindemittels,
umsetzt.
(C)
Ferner wurde gefunden, daß man Verbindungen der Formel (Ic)

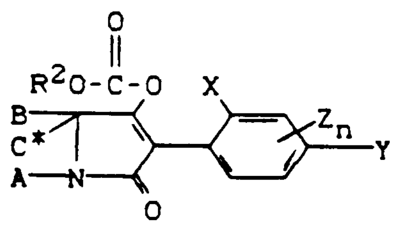
(Ic)

in welcher
A, B, C*, X, Y, Z, $R^2$ und n die oben angegebene Bedeutung haben,
erhält, wenn man Verbindungen der Formel (Ia)

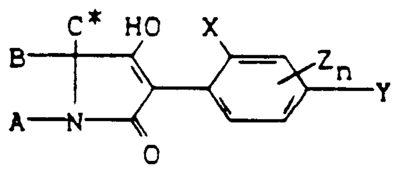
(Ia)

in welcher
A, B, C*, X, Y, Z und n die oben angegebene Bedeutung haben,
mit Chlorameisensäureester der allgemeinen Formel (V)

$R^2$-O-CO-Cl (V)

in welcher
$R^2$ die oben angegebene Bedeutung hat,
gegebenenfals in Gegenwart eines Verdünnungsmittels und gegebenenfalls in Gegenwart eines Säurebindemittels umsetzt.

Überraschenderweise wurde gefunden, daß die neuen 3-Arylpyrrolidin-2,4-dion-Derivate der Formel (I) sich durch hervorragende insektizide, akarizide, herbizide und antimykotische Wirkungen auszeichnen.

Bevorzugt sind 3-Aryl-pyrrolidin-2,4-dion-Derivate der Formel (I), in welcher
X für $C_1$-$C_6$-Alkyl, Halogen, $C_1$-$C_6$-Alkoxy steht,
Y für Wasserstoff, $C_1$-$C_6$-Alkyl, Halogen, $C_1$-$C_6$-Alkoxy, $C_1$-$C_3$-Halogenalkyl steht,
Z für $C_1$-$C_6$-Alkyl, Halogen, $C_1$-$C_6$-Alkoxy steht,
n für 0 oder 1 steht,
R für Wasserstoff (Ia) oder für die Gruppen der Formel

-CO-$R^1$ (Ib) oder -CO-O-$R^2$ (Ic)

steht, in welchen

| | |
|---|---|
| $R^1$ | für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht, für gegebenenfalls durch Halogen-, Nitro-, $C_1$-$C_6$-Alkyl-, $C_1$-$C_6$-Alkoxy-, $C_1$-$C_6$-Halogenalkyl-, $C_1$-$C_6$-Halogenalkoxy-substituiertes Phenyl; für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht, |
| $R^2$ | für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht, für gegebenenfalls durch Halogen-, Nitro-, $C_1$-$C_6$-Alkyl-, $C_1$-$C_6$-Alkoxy-, $C_1$-$C_6$-Halogenalkylsubstituiertes Phenyl oder Cycloalkyl mit 3-8 Ringatomen steht, |
| A | für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann oder gegebenenfalls durch Halogen, $C_1$-$C_6$-Alkyl-$C_1$-$C_6$-Haloalkyl-, $C_1$-$C_6$-Alkoxy-, Nitro substituiertes Aryl-$C_1$-$C_6$-alkyl steht, |
| B, C | unabhängig voneinander für Wasserstoff, geradkettiges oder verzweigtes $C_1$-$C_{12}$-Alkyl, $C_1$-$C_8$-Alkoxyalkyl steht, |

sowie die enantiomerenreinen Formen von Verbindungen der Formel (I).

Besonders bevorzugt sind Verbindungen der Formel (I) in welcher

| | |
|---|---|
| X | für $C_1$-$C_4$-Alkyl, Halogen, $C_1$-$C_4$-Alkoxy steht, |
| Y | für Wasserstoff, $C_1$-$C_6$-Alkyl, Halogen, $C_1$-$C_4$-Alkoxy, $C_1$-$C_2$-Halogenalkyl steht, |
| Z | für $C_1$-$C_4$-Alkyl, Halogen, $C_1$-$C_4$-Alkoxy steht, |
| n | für 0 oder 1 steht, |
| R | für Wasserstoff (Ia) oder für die Gruppen der Formel |

$-CO-R^1$ (Ib) oder $-CO-O-R^2$ (Ic)

steht, in welchen

| | |
|---|---|
| $R^1$ | für gegebenenfalls durch Halogen substituiertes; $C_1$-$C_{16}$-Alkyl, $C_2$-$C_{16}$-Alkenyl, $C_1$-$C_6$-Alkoxy-$C_2$-$C_6$-alkyl, $C_1$-$C_6$-Alkylthio-$C_2$-$C_6$-alkyl, $C_1$-$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann steht, für gegebenenfalls durch Halogen-, Nitro-, $C_1$-$C_4$-Alkyl-, $C_1$-$C_4$-Alkoxy-, $C_1$-$C_3$-Halogenalkyl-, $C_1$-$C_3$-Halogenalkoxy-substituiertes Phenyl steht, für gegebenenfalls durch Halogen-, $C_1$-$C_4$-Alkyl-, $C_1$-$C_4$-Alkoxy-, $C_1$-$C_3$-Halogenalkyl-, $C_1$-$C_3$-Halogenalkoxy-substituiertes Phenyl-$C_1$-$C_4$-alkyl steht, |
| $R^2$ | für gegebenenfalls durch Halogen substituiertes; $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht, für gegebenenfalls durch Halogen, Nitro-, $C_1$-$C_4$-Alkyl, $C_1$-$C_3$-Alkoxy-, $C_1$-$C_3$-Halogenalkylsubstituiertes Phenyl oder Cycloalkyl mit 3-7 Ringatomen steht, |
| A | für gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen, das durch 1-2 Sauerstoff- und/oder Schwefelatomen unterbrochen sein kann oder gegebenenfalls durch Halogen-, $C_1$-$C_4$-Alkyl-, $C_1$-$C_4$-Haloalkyl-$C_1$-$C_4$-Alkoxy-, Nitro substituiertes Aryl-$C_1$-$C_4$-alkyl steht, |
| B, C | unabhängig voneinander für Wasserstoff, geradkettiges oder verzweigtes $C_1$-$C_{10}$-Alkyl, $C_1$-$C_6$-Alkoxyalkyl steht, |

sowie die enantiomerenreinen Formen von Verbindungen der Formel (I).

Ganz besonders bevorzugt sind Verbindungen der Formel (I) in welcher

| | |
|---|---|
| X | für Methyl, Ethyl, Propyl, i-Propyl, Fluor, Chlor, Brom, Methoxy und Ethoxy steht, |
| Y | für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht, |
| Z | für Methyl, Ethyl, i-Propyl, Butyl, i-Butyl, tert.-Butyl, Fluor, Chlor, Brom, Methoxy und Ethoxy steht, |
| n | für 0 oder 1 steht, |
| R | für Wasserstoff (Ia) oder für die Gruppen der Formel |

$-CO-R^1$ (Ib) oder $-CO-O-R^2$ (Ic)

steht, in welcher

$R^1$ für gegebenenfalls durch Fluor oder Chlor substituiertes; $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann steht,
für gegebenenfalls durch Fluor-, Chlor, Brom-, Methyl-, Ethyl-, Propyl, i-Propyl, Methoxy, Ethoxy, Trifluormethyl-, Trifluormethoxy-, Nitro- substituiertes Phenyl steht,
für gegebenenfalls durch Fluor-, Chlor-, Brom-, Methyl-, Ethyl-, Propyl-, i-Propyl, Methoxy, Ethoxy, Trifluormethyl, Trifluormethoxysubstituiertes Phenyl-$C_1$-$C_3$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Cycloalkyl mit 3-6 Ringatomen steht,

A für gegebenenfalls durch Halogen substituiertes geradkettiges oder verzweigtes $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen, das durch 1-2 Sauerstoff- und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro susbtituiertes Aryl-$C_1$-$C_3$-alkyl steht,

B, C unabhängig voneinander für Wasserstoff, geradkettiges oder verzweigtes $C_1$-$C_8$-Alkyl, $C_1$-$C_4$-Alkoxyalkyl steht,

sowie die enantiomerenreinen Formen von Verbindungen der Formel I.

Verwendet man gemäß Verfahren (A) N-2,6-Dichlorphenylacetyl-N-methyl-alaninethylester, so kann der Verlauf des erfindungsgemäßen Verfahrens durch folgendes Reaktionsschema wiedergegeben werden:

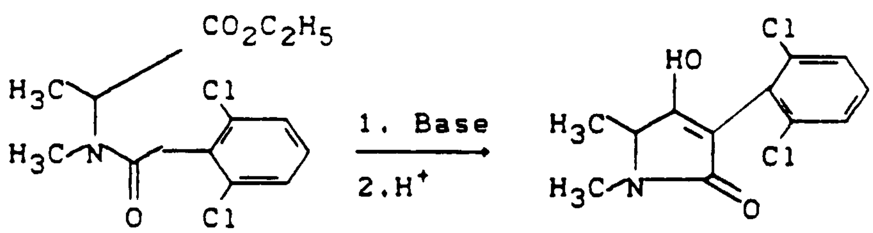


Verwendet man gemäß Verfahren (B) (Variante α) 3-(2,4,6-Trimethylphenyl)-1-isopropyl-pyrrolidin-2,4-dion und Pivaloylchlorid als Ausgangsstoff, so kann der Verlauf des erfindungsgemäßen Verfahrens durch folgendes Reaktionsschema wiedergegeben werden.

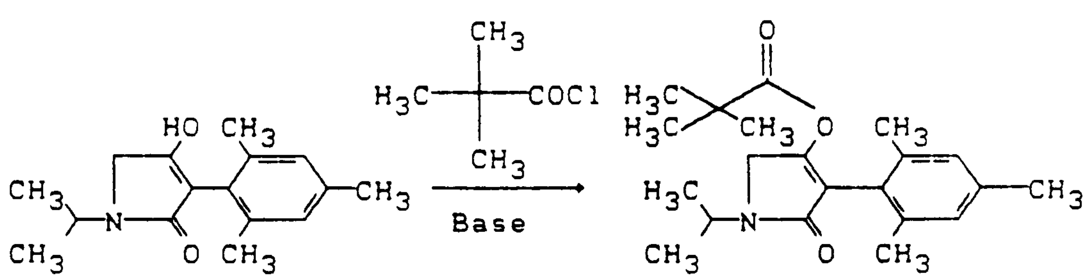


Verwendet man gemäß Verfahren B (Variante ß) 3-(2,4,6-Trimethylphenyl)-1-cyclopentyl-pyrrolidin-2,4-dion und Acetanhydrid, so kann der Verlauf des erfindungsgemäßen Verfahrens durch folgendes Reaktionsschema wiedergegeben werden.

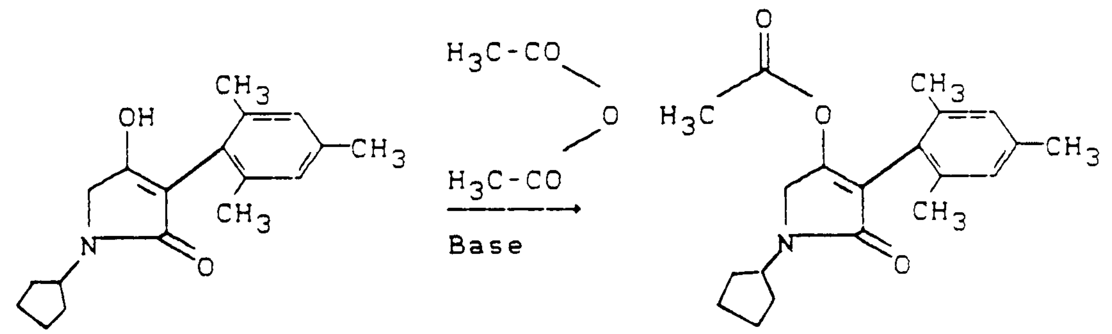


Verwendet man gemäß Verfahren C 3-(2,4-6-Trimethylphenyl)-1-methoxyethyl-5-methyl-pyrrolidin-2,4-dion und Chlorameisensäureethoxyethylester, so kann der Verlauf des erfindungsgemäßen Verfahrens durch folgendes Reaktionsschema wiedergegeben werden.

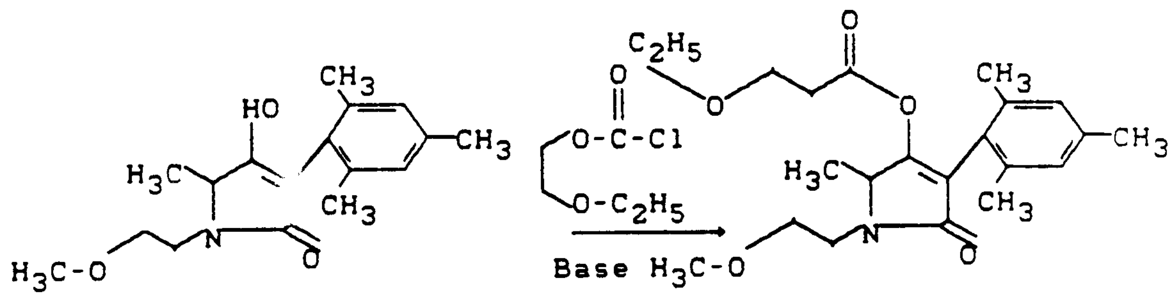


Die bei dem obigen Verfahren (A) als Ausgangsstoffe benötigten Verbindungen der Formel (II)

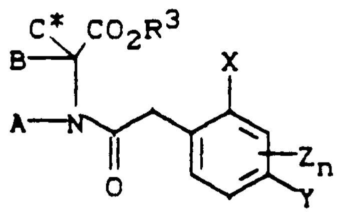

(II)

in welcher
A, B, C*, X, Y, Z, n und $R^3$ die oben angegebene Bedeutung haben sind teilweise bekannt oder lassen sich nach im Prinzip bekannten Methoden in einfacher Weise herstellen. So erhält man z.B. Acyl-aminosäureester der Formel (II), wenn man

a) Aminosäureester der Formel (VI),

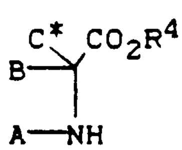

(VI)

in welcher

$R^4$ für Wasserstoff (VIa) und Alkyl (VIb) steht

und

A, B und C* die oben angegebene Bedeutung haben mit Phenylessigsäurehalogeniden der Formel (VII)

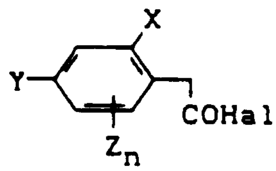

(VII)

in welcher

X, Y, Z und n die oben angegebene Bedeutung haben

und

Hal für Chlor oder Brom steht,

acyliert (Chem. Reviews 52 237-416 (1953);

oder wenn man Acylaminosäuren der Formel (IIa),

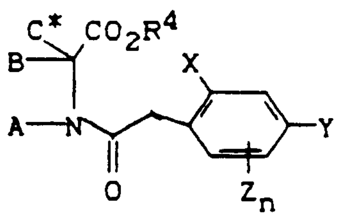

(IIa)

in welcher

A, B, C*, X, Y, Z und n die oben angegebene Bedeutung haben

und

$R^4$ für Wasserstoff steht,

verestert (Chem. Ind. (London) 1568 (1968)).

Beispielhaft seien folgende Verbindungen der Formel (II) genannt:

1. N-Isopropyl-N-(2,4-Dichlorphenyl-acetyl)-glycin-ethylester
2. N-Isopropyl-N-(2,6-Dichlorphenyl-acetyl)-glycin-ethylester
3. N-(2,6-Dichlorphenyl-acetyl)-sarkosin-methylester
4. N-Isopropyl-N-(2,6-dichlorphenyl-acetyl)-alanin-ethylester
5. N-Methoxyethyl-N-(2,6-dichlorphenyl-acetyl)-glycin-ethylester
6. N-Methoxyethyl-N-(2,6-dichlorphenyl-acetyl)-alanin-ethylester
7. N-tert.-Butyl-N-(2,6-dichlorphenyl-acetyl)-glycin-ethylester
8. N-Methyl-N-(2,6-dichlorphenyl-acetyl)-alanin-ethylester
9. N-2-(2,4,4-trimethyl-pentyl)-N-(2,6-dichlorphenyl-acetyl)-glycin-ethylester
10. N-(2,4,6-trimethylphenyl-acetyl)-sarkosin-methylester
11. N-Ethyl-N-(2,4,6-trimethylphenyl-acetyl)-glycin-ethylester
12. N-Isopropyl-N-(2,4,6-trimethylphenyl-acetyl)-glycin-ethylester
13. N-tert.-Butyl-N-(2,4,6-trimethylphenyl-acetyl)-glycin-ethylester
14. N-iso-Butyl-N-(2,4,6-trimethylphenyl-acetyl)-glycin-ethylester
15. N-sec-Butyl-N-(2,4,6-trimethylphenyl-acetyl)-glycin-ethylester
16. N-neo-Pentyl-N-(2,4,6-trimethylphenyl-acetyl)-glycin-ethylester
17. N-2-(2,3-Dimethyl-butyl)-N-(2,4,6-trimethylphenyl-acetyl)-glycin-ethylester
18. N-2-(2,2,3-Trimethyl-butyl)-N-(2,4,6-trimethylphenyl-acetyl)-glycin-ethylester
19. N-Cyclopropyl-N-(2,4,6-trimethylphenyl-acetyl)-glycin-ethylester
20. N-Cyclopentyl-N-(2,4,6-trimethylphenyl-acetyl)-glycin-ethylester
21. N-Cyclohexyl-N-(2,4,6-trimethylphenyl-acetyl)-glycin-ethylester
22. N-Alkyl-N-(2,4,6-trimethylphenyl-acetyl)-glycin-ethylester
23. N-Benzyl-N-(2,4,6-trimethylphenyl-acetyl)-glycin-ethylester
24. N-2-(2,4,4-trimethyl-pentyl)-N-(2,4,6-Trimethylphenyl-acetyl)-glycin-ethylester
25. N-Methoxyethyl-N-(2,4,6-trimethylphenyl-acetyl)-glycin-ethylester
26. N-Methoxypropyl-N-(2,4,6-trimethylphenyl-acetyl)-glycin-ethylester
27. N-Methoxy-2-methyl-propyl-N-(2,4,6-trimethylphenyl-acetyl)-glycin-ethylester
28. N-2-(Ethoxy-butyl)-N-(2,4,6-trimethylphenyl-acetyl)-glycin-ethylester
29. N-2-(Methoxy-propyl)-N-(2,4,6-trimethylphenyl-acetyl)-glycin-ethylester

30. N-Ethyl-mercaptoethyl-N-(2,4,6-trimethylphenyl-acetyl)-glycin-ethylester
31. N-Methyl-N-(2,4,6-trimethylphenyl-acetyl)-alanin-ethylester
32. N-Ethyl-N-(2,4,6-trimethylphenyl-acetyl)-alanin-ethylester
33. N-Isopropyl-N-(2,4,6-trimethylphenyl-acetyl)-alanin-ethylester
34. N-Isobutyl-N-(2,4,6-trimethylphenyl-acetyl)-alanin-ethylester
35. N-sec-Butyl-N-(2,4,6-trimethylphenyl-acetyl)-alanin-ethylester
36. N-Cyclopropyl-N-(2,4,6-trimethylphenyl-acetyl)-alanin-ethylester
37. N-Cyclopentyl-N-(2,4,6-trimethylphenyl-acetyl)-alanin-ethylester
38. N-Cyclohexyl-N-(2,4,6-trimethylphenyl-acetyl)-alanin-ethylester
39. N-Methoxyethyl-N-(2,4,6-trimethylphenyl-acetyl)-alanin-ethylester
40. N-Methoxypropyl-N-(2,4,6-trimethylphenyl-acetyl)-alanin-ethylester
41. N-Methyl-N-(2,4,6-trimethylphenyl-acetyl)-2-amino-buttersäure-ethylester
42. N-Ethyl-N-(2,4,6-trimethylphenyl-acetyl)-2-amino-buttersäure-ethylester
43. N-Methyl-N-(2,4,6-trimethylphenyl-acetyl)-2-amino-valeriansäure-ethylester
44. N-Methyl-N-(2,4,6-trimethylphenyl-acetyl)-2-amino-iso-valeriansäure-ethylester
45. N-Ethyl-N-(2,4,6-trimethylphenyl-acetyl)-2-amino-valeriansäure-ethylester
46. N-Ethyl-N-(2,4,6-trimethylphenyl-acetyl)-2-amino-iso-valeriansäure-ethylester
47. N-Methyl-N-(2,4,6-trimethylphenyl-acetyl)-2-methylalanin-ethylester
48. N-Ethyl-N-(2,4,6-trimethylphenyl-acetyl)-2-methylalanin-ethylester
49. N-Isopropyl-N-(2,4,6-trimethylphenyl-acetyl)-2-methylalanin-ethylester

Beispielhaft sind folgende Verbindungen der Formel (IIa) genannt:

1. N-(2,4-Dichlorphenyl-acetyl)-sarkosin
2. N-(2,6-Dichlorphenyl-acetyl)-sarkosin
3. N-(2,4,6-trimethylphenyl-acetyl)-sarkosin

Verbindungen der Formel (IIa) sind beispielsweise aus den Phenylessigsäurehalogeniden der Formel (VII) und Aminosäuren der Formel (VIa) nach Schotten-Baumann (Organikum 9, Auflage 446 (1970) VEB Deutscher Verlag der Wissenschaften, Berlin) erhältlich.

Verbindungen der Formel VI, in welchen A, B, C und $R^4$ die oben angegebene Bedeutung haben, sind nach literaturbekannten Verfahren aus $\alpha$-Halogencarbonsäuren bzw. -estern und Aminen erhältlich (Advanced Organic Chemistry, J. March S. 377, Mc Graw-Hill Inc. 1977).

Das Verfahren (A) ist dadurch gekennzeichnet, daß Verbindungen der Formel (II) in welcher A, B, C, X, Y, Z, m, n und $R^3$ die oben angegebene Bedeutung haben in Gegenwart von Basen einer intramolekularen Kondensation unterwirft.

Als Verdünnungsmittel können bei dem erfindungsgemäßen Verfahren (A) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid und N-Methylpyrrolidon.

Als Deprotonierungsmittel können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natriummethylat und Kalium-tert.-butylat einsetzbar. Adogen 464 = Methyltrialkyl($C_8$-$C_{10}$)ammoniumchlorid TDA 1 = Tris-(methoxyethoxylethyl)-amin

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0 °C und 250 °C, vorzugsweise zwischen 50 °C und 150 °C.

Das erfindungsgemäße Verfahren (A) wird im allgemeinen unter Normaldruck durchgeführt.

Adogen 464 = Methyltrialkyl($C_8$-$C_{10}$)ammoniumchlorid
TDA 1 = Tris-(methoxyethoxylethyl)-amin

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, , Diazabicyclooctan (DABCO), Diazabicycloundecen (DBU), Diazabicyclononben (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet.

Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können auch bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern der Formel (V) umsetzt.

Verwendet man die entsprechenden Chlorameisensäureester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20 °C und +100 °C, vorzugsweise zwischen 0 °C und 50 °C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende (Chlorameisensäureester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beispiel 1:

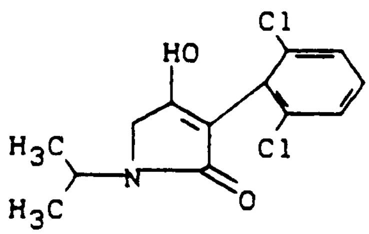

3,9 g (0,13 Mol) Natriumhydrid (80%ig) werden in 70 ml abs. Toluol vorgelegt. Nach Zutropfen von 36,2 g (0,107 Mol) N-2,6-Dichlorphenylacetyl-N-isopropyl-glycinethylester in 160 ml abs. Toluol, erhitzt man 6 h unter Rückfluß. Unter Eisbadkühlung werden 20 ml Ethanol zugetropft, der Ansatz im Vakuum einrotiert, der Rückstand in 1 N NaOH gelöst und das 3-(2,6-Dichlorphenyl)-1-isopropyl-pyrrolidin-2,4-dion bei 0-20 °C mit konzentrierter Salzsäure gefällt. Das Produkt wird zur Reinigung mit Chloroform ausgekocht, anschließend wird n-Hexan zugesetzt und das ausgefallene, farblose Produkt abgesaugt.

Ausbeute: 25,42 g (83 % d. Theorie) Fp. >230 °C.

Beispiel 2:

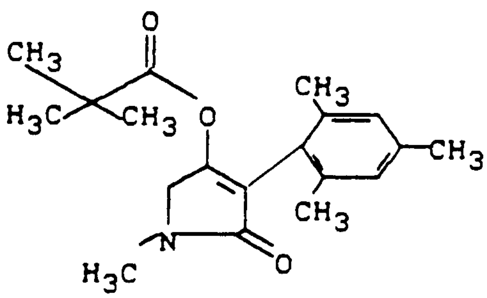

3,42 g (15 mmol) 3-(2,4,6-Trimethylphenyl)-1-methyl-pyrrolidin-2,4-dionwerden in 50 ml abs. Tetrahydrofuran (THF) suspendiert und mit 1,22 ml (15 mmol) abs. Pyridin und 2,54 ml (15 mmol) Ethyl-diisopropylamin versetzt. Dazu tropft man bei 0 °-10 °C 1,88 ml (15 mmol) Pivaloylchlorid gelöst in 5 ml abs. THF und rührt 30 Min. nach. Der Niederschlag wird abfiltriert, die Lösung im Vakuum einrotiert und der Rückstand an Kieselgel mit Cyclohexan/Essigester 1:1 chromatographiert.

Durch Kristallisation aus Ether/n-Hexan erhält man 3,8 g (80,4 % der Theorie) 4-(Pivaloyloxy)-3-(2,4,6-trimethylphenyl)-1-methyl-3-pyrrolin-2-on von Schmp. 75 °C.

Beispiel 3

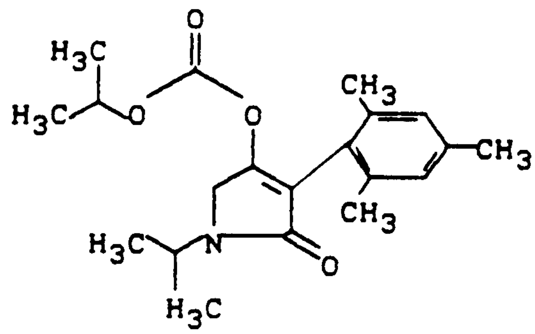

5,18 g (20 mmol) 3-(2,4,6-trimethylphenyl)-1-isopropyl-pyrrolidin-2,4-dion werden in 70 ml tert.-Butylmethylether (MTB-Ether) suspendiert. Nach Zugabe von 1,63 ml (20 mmol) abs, Pyridin und 3,4 ml (20 mmol) Ethyl-diisopropylamin tropft man bei 0 °C - 10 °C 2,45 g (20 mmol) Chlorameisensäure-isopropylester, gelöst in 5 ml MTB-Ether, zu, rührt 30 Minuten nach, filtriert ab und rotiert ein. Der Rückstand wird an Kieselgel mit Cyclohexan/Essigester 1 : 1 chromatographiert. Durch Kristallisation aus n-Hexan erhält man 4,67 g (67,6% der Theorie) 4-Isopropoxy-carbonyloxy-3-(2,4,6-trimethylphenyl)-1-isopropyl-3-pyrrolin-2-on vom Schmelzpunkt 81 °C.

In entsprechender Weise zu den Herstellungsbeispielen und gemäß den allgemeinen Angaben zur Herstellung erhält man die in den nachfolgenden Tabellen 1-3 formelgmäßig aufgeführten 3-Aryl-pyrrolidin-2,4-dion(e)-Derivate der Formel (Ia) - (Ic).

Tabelle 1

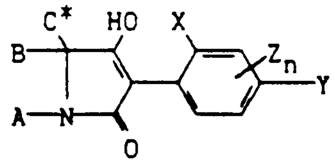

(Ia)

| Bsp. Nr. | X | Y | $Z_n$ | A | B | C* | Fp °C |
|---|---|---|---|---|---|---|---|
| 4 | Cl | Cl | H | $(CH_3)_2CH$- | H | H | 198 |
| 5 | Cl | H | 6-Cl | $CH_3$- | H | H | 230 |
| 6 | Cl | H | 6-Cl | $CH_3$- | $CH_3$- | H | 221 |
| 7 | Cl | H | 6-Cl | $(CH_3)_2CH$- | $CH_3$- | $CH_3$- | 180 |
| 8 | Cl | H | 6-Cl | $(CH_3)_3C$- | H | H | > 230 |
| 9 | Cl | H | 6-Cl | $(CH_3)_3C-CH_2-C(CH_3)_2$- | H | H | > 235 |
| 10 | Cl | H | 6-Cl | $(CH_3O-(CH_2)_2$- | H | H | > 230 |
| 11 | Cl | H | 6-Cl | $CH_3-O-(CH_2)_2$- | H | H | 128 |
| 12 | $CH_3$ | $CH_3$ | 6-$CH_3$ | $CH_3$- | H | H | > 230 |
| 13 | $CH_3$ | $CH_3$ | 6-$CH_3$ | $CH_3$- | $CH_3$- | H | > 230 |
| 14 | $CH_3$ | $CH_3$ | 6-$CH_3$ | $CH_3$- | $C_2H_5$ | H | 210 |
| 15 | $CH_3$ | $CH_3$ | 6-$CH_3$ | $CH_3$- | $C_3H_7$ | H | |
| 16 | $CH_3$ | $CH_3$ | 6-$CH_3$ | $CH_3$- | $(CH_3)_2CH$- | H | |
| 17 | $CH_3$ | $CH_3$ | 6-$CH_3$ | $CH_3$- | $CH_3$- | $CH_3$- | |
| 18 | $CH_3$ | $CH_3$ | 6-$CH_3$ | $C_2H_5$ | H | H | > 230 |
| 19 | $CH_3$ | $CH_3$ | 6-$CH_3$ | $C_2H_5$ | $CH_3$- | H | 227 |
| 20 | $CH_3$ | $CH_3$ | 6-$CH_3$ | $C_2H_5$ | $C_2H_5$- | H | 184 |
| 21 | $CH_3$ | $CH_3$ | 6-$CH_3$ | $C_2H_5$ | $C_3H_7$- | H | |
| 22 | $CH_3$ | $CH_3$ | 6-$CH_3$ | $C_2H_5$ | $(CH_3)_2CH$- | H | |
| 23 | $CH_3$ | $CH_3$ | 6-$CH_3$ | $C_2H_5$ | $CH_3$- | $CH_3$- | |

Tabelle 1 (Fortsetzung)

| Bsp. Nr. | X | Y | $Z_n$ | A | B | C* | Fp °C |
|---|---|---|---|---|---|---|---|
| 24 | $CH_3$ | $CH_3$ | 6-$CH_3$ | $C_3H_7$ | H | H | |
| 25 | $CH_3$ | $CH_3$ | 6-$CH_3$ | $C_3H_7$ | $CH_3$- | H | |
| 26 | $CH_3$ | $CH_3$ | 6-$CH_3$ | $C_3H_7$ | $C_2H_5$- | H | |
| 27 | $CH_3$ | $CH_3$ | 6-$CH_3$ | $C_3H_7$ | $C_3H_7$ | H | |
| 28 | $CH_3$ | $CH_3$ | 6-$CH_3$ | $C_3H_7$ | $(CH_3)_2CH$- | H | |
| 29 | $CH_3$ | $CH_3$ | 6-$CH_3$ | $C_3H_7$ | $CH_3$- | $CH_3$- | |
| 30 | $CH_3$ | $CH_3$ | 6-$CH_3$ | $(CH_3)_2CH$- | H | H | $>$ 220 |
| 31 | $CH_3$ | $CH_3$ | 6-$CH_3$ | $(CH_3)_2CH$- | $CH_3$- | H | 228 |
| 32 | $CH_3$ | $CH_3$ | 6-$CH_3$ | $(CH_3)_2CH$- | $C_2H_5$ | H | |
| 33 | $CH_3$ | $CH_3$ | 6-$CH_3$ | $(CH_3)_2CH$- | $C_3H_7$ | H | |
| 34 | $CH_3$ | $CH_3$ | 6-$CH_3$ | $(CH_3)_2CH$- | $(CH_3)_2CH$- | H | |
| 35 | $CH_3$ | $CH_3$ | 6-$CH_3$ | $(CH_3)_2CH$- | $CH_3$- | $CH_3$- | |
| 36 | $CH_3$ | $CH_3$ | 6-$CH_3$ | $C_4H_9$ | H | H | |
| 37 | $CH_3$ | $CH_3$ | 6-$CH_3$ | $C_4H_9$ | $CH_3$- | H | |
| 38 | $CH_3$ | $CH_3$ | 6-$CH_3$ | $(CH_3)_2CH-CH_2$- | H | H | 209 |
| 39 | $CH_3$ | $CH_3$ | 6-$CH_3$ | $(CH_3)_2CH-CH_2$- | $CH_3$- | H | 189 |
| 40 | $CH_3$ | $CH_3$ | 6-$CH_3$ | 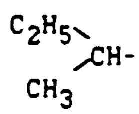 | H | H | 262 |
| 41 | $CH_3$ | $CH_3$ | 6-$CH_3$ | 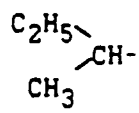 | $CH_3$- | H | 205 |

Tabelle 1 (Fortsetzung)

| Bsp. Nr. | X | Y | $Z_n$ | A | B | C* | Fp °C |
|---|---|---|---|---|---|---|---|
| 42 | $CH_3$ | $CH_3$ | 6-$CH_3$ | $(CH_3)_3C$- | H | H | > 230 |
| 43 | $CH_3$ | $CH_3$ | 6-$CH_3$ | $(CH_3)_3C$-$CH_2$- | H | H | > 230 |
| 44 | $CH_3$ | $CH_3$ | 6-$CH_3$ | $(CH_3)_2CH$-$CH(CH_3)$- | H | H | > 230 |
| 45 | $CH_3$ | $CH_3$ | 6-$CH_3$ | $(CH_3)_3C$-$CH(CH_3)$- | H | H | > 230 |
| 46 | $CH_3$ | $CH_3$ | 6-$CH_3$ | $(CH_3)_3C$-$CH_2$-$C(CH_3)_2$- | H | H | > 230 |
| 47 | $CH_3$ | $CH_3$ | 6-$CH_3$ | $CH_2$=CH-$CH_2$- | H | H | 212 |
| 48 | $CH_3$ | $CH_3$ | 6-$CH_3$ | $CH_2$=CH-$CH_2$- | $CH_3$- | H | |
| 49 | $CH_3$ | $CH_3$ | 6-$CH_3$ | 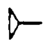 | H | H | > 230 |
| 50 | $CH_3$ | $CH_3$ | 6-$CH_3$ | 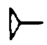 | $CH_3$- | H | > 230 |
| 51 | $CH_3$ | $CH_3$ | 6-$CH_3$ | 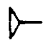 | $C_2H_5$- | H | |
| 52 | $CH_3$ | $CH_3$ | 6-$CH_3$ | 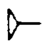 | $C_3H_7$- | H | |
| 53 | $CH_3$ | $CH_3$ | 6-$CH_3$ | 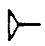 | $(CH_3)_2CH$- | H | |
| 54 | $CH_3$ | $CH_3$ | 6-$CH_3$ | 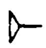 | $CH_3$- | $CH_3$- | |
| 55 | $CH_3$ | $CH_3$ | 6-$CH_3$ | 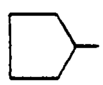 | H | H | > 230 |
| 56 | $CH_3$ | $CH_3$ | 6-$CH_3$ | 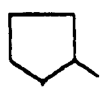 | $CH_3$- | H | 223 |
| 57 | $CH_3$ | $CH_3$ | 6-$CH_3$ | 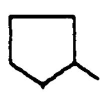 | $C_2H_5$- | H | |
| 58 | $CH_3$ | $CH_3$ | 6-$CH_3$ | 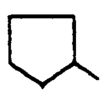 | $C_3H_7$- | H | |

Tabelle 1 (Fortsetzung)

| Bsp. Nr. | X | Y | $Z_n$ | A | B | C* | Fp °C |
|---|---|---|---|---|---|---|---|
| 59 | $CH_3$ | $CH_3$ | 6-$CH_3$ | 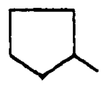 | $(CH_3)_2CH$- | H | |
| 60 | $CH_3$ | $CH_3$ | 6-$CH_3$ | 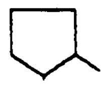 | $CH_3$- | $CH_3$- | |
| 61 | $CH_3$ | $CH_3$ | 6-$CH_3$ | 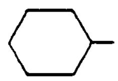 | H | H | > 230 |
| 62 | $CH_3$ | $CH_3$ | 6-$CH_3$ | 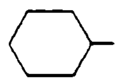 | $CH_3$- | H | > 230 |
| 63 | $CH_3$ | $CH_3$ | 6-$CH_3$ | 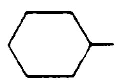 | $C_2H_5$- | H | |
| 64 | $CH_3$ | $CH_3$ | 6-$CH_3$ | 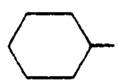 | $C_3H_7$- | H | |
| 65 | $CH_3$ | $CH_3$ | 6-$CH_3$ | 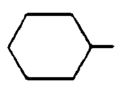 | $(CH_3)_2CH$- | H | |
| 66 | $CH_3$ | $CH_3$ | 6-$CH_3$ | 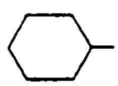 | $CH_3$- | $CH_3$- | |
| 67 | $CH_3$ | $CH_3$ | 6-$CH_3$ | 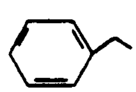 | H | H | > 230 |
| 68 | $CH_3$ | $CH_3$ | 6-$CH_3$ | 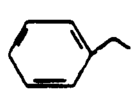 | $CH_3$- | H | |

Tabelle 1 (Fortsetzung)

| Bsp. Nr. | X | Y | $Z_n$ | A | B | C* | Fp °C |
|---|---|---|---|---|---|---|---|
| 69 | $CH_3$ | $CH_3$ | 6-$CH_3$ | $CH_3$-O-$(CH_2)_2$- | H | H | 179 |
| 70 | $CH_3$ | $CH_3$ | 6-$CH_3$ | $CH_3$-O-$(CH_2)_2$- | $CH_3$- | H | 165 |
| 71 | $CH_3$ | $CH_3$ | 6-$CH_3$ | $CH_3$-O-$CH_2$-$CH(CH_3)$- | H | H | 220 |
| 72 | $CH_3$ | $CH_3$ | 6-$CH_3$ | $CH_3$-O-$CH_2$-$CH(CH_3)$- | $CH_3$- | H | |
| 73 | $CH_3$ | $CH_3$ | 6-$CH_3$ | $CH_3$-O-$(CH_2)_3$- | H | H | 190 |
| 74 | $CH_3$ | $CH_3$ | 6-$CH_3$ | $CH_3$-O-$(CH_2)_3$- | $CH_3$- | H | 175 |
| 75 | $CH_3$ | $CH_3$ | 6-$CH_3$ | $C_2H_5$-O-$(CH_2)_2$-$CH(CH_3)$- | H | H | 220 |
| 76 | $CH_3$ | $CH_3$ | 6-$CH_3$ | $C_2H_5$-O-$(CH_2)_2$-$CH(CH_3)$- | $CH_3$- | H | |
| 77 | $CH_3$ | $CH_3$ | 6-$CH_3$ | $CH_3$-O-$CH_2$-$CH(CH_3)$-$CH_2$- | H | H | 156 |
| 78 | $CH_3$ | $CH_3$ | 6-$CH_3$ | $C_2H_5$-S-$(CH_2)_2$- | H | H | 165 |

Tabelle 2

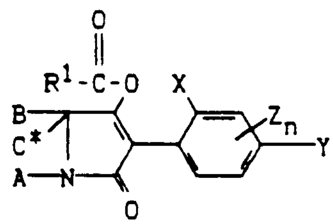

(Ib)

| Bsp. Nr. | X | Y | $Z_n$ | A | B | C* | $R^1$ | Fp °C |
|---|---|---|---|---|---|---|---|---|
| 79 | Cl | Cl | H | $(CH_3)_2CH-$ | H | H | $CH_3-$ | 128 |
| 80 | Cl | H | 6-Cl | $CH_3-$ | $CH_3-$ | H | $CH_3-$ | 125 |
| 81 | Cl | H | 6-Cl | $CH_3-$ | $CH_3-$ | H | $(CH_3)_2CH-$ | Öl |
| 82 | Cl | H | 6-Cl | $CH_3-$ | $CH_3-$ | H | $(CH_3)_3C-$ | 68 |
| 83 | Cl | H | 6-Cl | $(CH_3)_2CH-$ | H | H | $CH_3-$ | 113 |
| 84 | Cl | H | 6-Cl | $(CH_3)_2CH-$ | H | H | $(CH_3)_2CH-$ | 105 |
| 85 | Cl | H | 6-Cl | $(CH_3)_2CH-$ | H | H | $(CH_3)_3C-$ | 122 |
| 86 | Cl | H | 6-Cl | $(CH_3)_2CH-$ | H | H | $(CH_3)_2CH-C(CH_3)_2-$ | 112 |
| 87 | Cl | H | 6-Cl | $(CH_3)_3C-$ | H | H | $CH_3-$ | 113 |
| 88 | Cl | H | 6-Cl | $(CH_3)_3C-$ | H | H | $(CH_3)_2CH-$ | 117 |
| 89 | Cl | H | 6-Cl | $(CH_3)_3C-$ | H | H | $(CH_3)_3C-$ | 158 |
| 90 | $CH_3$ | $CH_3$ | 6-$CH_3$ | $CH_3-$ | H | H | $CH_3-$ | Öl |
| 91 | $CH_3$ | $CH_3$ | 6-$CH_3$ | $CH_3-$ | H | H | $(CH_3)_2CH-$ | Öl |
| 92 | $CH_3$ | $CH_3$ | 6-$CH_3$ | $CH_3-$ | H | H | $(CH_3)_2CH-C(CH_3)_2-$ | 45 |
| 93 | $CH_3$ | $CH_3$ | 6-$CH_3$ | $CH_3-$ | $CH_3-$ | H | $CH_3-$ | 75 |
| 94 | $CH_3$ | $CH_3$ | 6-$CH_3$ | $CH_3-$ | $CH_3-$ | H | $(CH_3)_2CH-$ | Öl |
| 95 | $CH_3$ | $CH_3$ | 6-$CH_3$ | $CH_3-$ | $CH_3-$ | H | $(CH_3)_3C-$ | Öl |

<u>Tabelle 2</u> (Fortsetzung)

| Bsp. Nr. | X | Y | $Z_n$ | A | B | C* | $R^1$ | Fp °C |
|---|---|---|---|---|---|---|---|---|
| 96 | $CH_3$ | $CH_3$ | 6-$CH_3$ | $CH_3$- | $CH_3$- | H | $(CH_3)_2CH-C(CH_3)_2$- | |
| 97 | $CH_3$ | $CH_3$ | 6-$CH_3$ | $CH_3$- | $CH_3$- | H | $(CH_3)_3C-CH_2$- | Öl |
| 98 | $CH_3$ | $CH_3$ | 6-$CH_3$ | $CH_3$- | $CH_3$- | H | $CH_3O-CH_2-C(CH_3)_2$- | Öl |
| 99 | $CH_3$ | $CH_3$ | 6-$CH_3$ | $CH_3$- | $CH_3$- | H | $ClCH_2-C(CH_3)_2$- | Öl |
| 100 | $CH_3$ | $CH_3$ | 6-$CH_3$ | $CH_3$- | $CH_3$- | H | 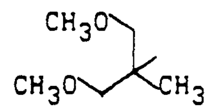 | |
| 101 | $CH_3$ | $CH_3$ | 6-$CH_3$ | $CH_3$- | $CH_3$- | H | $(CH_3)_2C=CH$- | |
| 102 | $CH_3$ | $CH_3$ | 6-$CH_3$ | $CH_3$- | $CH_3$- | H | 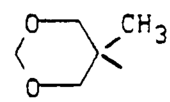 | |
| 103 | $CH_3$ | $CH_3$ | 6-$CH_3$ | $CH_3$- | $C_2H_5$- | H | $CH_3$- | |
| 104 | $CH_3$ | $CH_3$ | 6-$CH_3$ | $CH_3$- | $C_2H_5$- | H | $(CH_3)_2CH$- | |
| 105 | $CH_3$ | $CH_3$ | 6-$CH_3$ | $CH_3$- | $C_2H_5$- | H | $(CH_3)_3C$- | |
| 106 | $CH_3$ | $CH_3$ | 6-$CH_3$ | $CH_3$- | $C_2H_5$- | H | $(CH_3)_2CH-C(CH_3)_2$- | |
| 107 | $CH_3$ | $CH_3$ | 6-$CH_3$ | $CH_3$- | $C_2H_5$- | H | $(CH_3)_3C-CH_2$- | |
| 108 | $CH_3$ | $CH_3$ | 6-$CH_3$ | $CH_3$- | $C_2H_5$- | H | $CH_3O-CH_2-C(CH_3)_2$- | |
| 109 | $CH_3$ | $CH_3$ | 6-$CH_3$ | $CH_3$- | $C_2H_5$- | H | $ClCH_2-C(CH_3)_2$- | |
| 110 | $CH_3$ | $CH_3$ | 6-$CH_3$ | $CH_3$- | $C_2H_5$- | H | 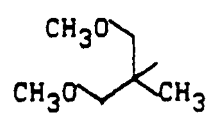 | |
| 111 | $CH_3$ | $CH_3$ | 6-$CH_3$ | $CH_3$- | $C_2H_5$- | H | $(CH_3)_2C=CH$- | |
| 112 | $CH_3$ | $CH_3$ | 6-$CH_3$ | $CH_3$- | $C_2H_5$. | H | 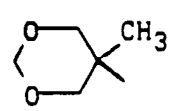 | |

Tabelle 2 (Fortsetzung)

| Bsp. Nr. | X | Y | $Z_n$ | A | B | C* | $R^1$ | Fp °C |
|---|---|---|---|---|---|---|---|---|
| 113 | $CH_3$ | $CH_3$ | 6-$CH_3$ | $CH_3$- | $C_3H_7$- | H | $CH_3$- | |
| 114 | $CH_3$ | $CH_3$ | 6-$CH_3$ | $CH_3$- | $C_3H_7$- | H | $(CH_3)_2CH$- | |
| 115 | $CH_3$ | $CH_3$ | 6-$CH_3$ | $CH_3$- | $C_3H_7$- | H | $(CH_3)_3C$- | |
| 116 | $CH_3$ | $CH_3$ | 6-$CH_3$ | $CH_3$- | $C_3H_7$- | H | $(CH_3)_2CH$-$C(CH_3)_2$- | |
| 117 | $CH_3$ | $CH_3$ | 6-$CH_3$ | $CH_3$- | $(CH_3)_2CH$- | H | $CH_3$- | |
| 118 | $CH_3$ | $CH_3$ | 6-$CH_3$ | $CH_3$- | $(CH_3)_2CH$- | H | $(CH_3)_2CH$- | |
| 119 | $CH_3$ | $CH_3$ | 6-$CH_3$ | $CH_3$- | $(CH_3)_2CH$- | H | $(CH_3)_3C$- | |
| 120 | $CH_3$ | $CH_3$ | 6-$CH_3$ | $CH_3$- | $(CH_3)_2CH$- | H | $(CH_3)_2CH$-$C(CH_3)_2$- | |
| 121 | $CH_3$ | $CH_3$ | 6-$CH_3$ | $CH_3$- | $CH_3$- | $CH_3$- | $CH_3$- | |
| 122 | $CH_3$ | $CH_3$ | 6-$CH_3$ | $CH_3$- | $CH_3$- | $CH_3$- | $(CH_3)_2CH$- | |
| 123 | $CH_3$ | $CH_3$ | 6-$CH_3$ | $CH_3$- | $CH_3$- | $CH_3$- | $(CH_3)_3C$- | |
| 124 | $CH_3$ | $CH_3$ | 6-$CH_3$ | $CH_3$- | $CH_3$- | $CH_3$- | $(CH_3)_2$-CH-$C(CH_3)_2$- | |
| 125 | $CH_3$ | $CH_3$ | 6-$CH_3$ | $C_2H_5$ | H | H | $CH_3$- | 85 |
| 126 | $CH_3$ | $CH_3$ | 6-$CH_3$ | $C_2H_5$ | H | H | $(CH_3)_2CH$- | |
| 127 | $CH_3$ | $CH_3$ | 6-$CH_3$ | $C_2H_5$ | H | H | $(CH_3)_3C$- | 99 |
| 128 | $CH_3$ | $CH_3$ | 6-$CH_3$ | $C_2H_5$ | H | H | $(CH_3)_2$-CH-$C(CH_3)_2$- | |
| 129 | $CH_3$ | $CH_3$ | 6-$CH_3$ | $C_2H_5$ | H | H | $(CH_3)_3C$-$CH_2$- | |
| 130 | $CH_3$ | $CH_3$ | 6-$CH_3$ | $C_2H_5$ | H | H | $CH_3O$-$CH_2$-$C(CH_3)_2$- | |
| 131 | $CH_3$ | $CH_3$ | 6-$CH_3$ | $C_2H_5$ | H | H | $ClCH_2$-$C(CH_3)_2$- | |
| 132 | $CH_3$ | $CH_3$ | 6-$CH_3$ | $C_2H_5$ | H | H | 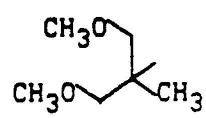 | |

Tabelle 2 (Fortsetzung)

| Bsp. Nr. | X | Y | $Z_n$ | A | B | C* | $R^1$ | Fp °C |
|---|---|---|---|---|---|---|---|---|
| 133 | $CH_3$ | $CH_3$ | 6-$CH_3$ | $C_2H_5$- | H | H | $(CH_3)_2C{=}CH$- | |
| 134 | $CH_3$ | $CH_3$ | 6-$CH_3$ | $C_2H_5$- | H | H | 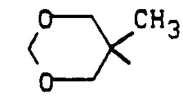 | |
| 135 | $CH_3$ | $CH_3$ | 6-$CH_3$ | $C_2H_5$- | $CH_3$- | H | $CH_3$- | Öl |
| 136 | $CH_3$ | $CH_3$ | 6-$CH_3$ | $C_2H_5$- | $CH_3$- | H | $(CH_3)_2CH$- | |
| 137 | $CH_3$ | $CH_3$ | 6-$CH_3$ | $C_2H_5$- | $CH_3$- | H | $(CH_3)_3C$- | Öl |
| 138 | $CH_3$ | $CH_3$ | 6-$CH_3$ | $C_2H_5$- | $CH_3$- | H | $(CH_3)_2$-CH-C$(CH_3)_2$- | |
| 139 | $CH_3$ | $CH_3$ | 6-$CH_3$ | $C_2H_5$- | $CH_3$- | H | $(CH_3)_3C$-$CH_2$- | Öl |
| 140 | $CH_3$ | $CH_3$ | 6-$CH_3$ | $C_2H_5$- | $CH_3$- | H | $CH_3O$-$CH_2$-C$(CH_3)_2$- | |
| 141 | $CH_3$ | $CH_3$ | 6-$CH_3$ | $C_2H_5$- | $CH_3$- | H | $ClCH_2$-C$(CH_3)_2$- | |
| 142 | $CH_3$ | $CH_3$ | 6-$CH_3$ | $C_2H_5$- | $CH_3$- | H | 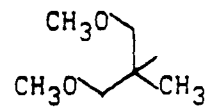 | |
| 143 | $CH_3$ | $CH_3$ | 6-$CH_3$ | $C_2H_5$- | $CH_3$- | H | $(CH_3)_2C{=}CH$- | |
| 144 | $CH_3$ | $CH_3$ | 6-$CH_3$ | $C_2H_5$- | $CH_3$- | H | 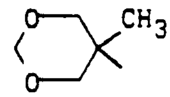 | |
| 145 | $CH_3$ | $CH_3$ | 6-$CH_3$ | $C_2H_5$- | $C_2H_5$- | H | $CH_3$- | |
| 146 | $CH_3$ | $CH_3$ | 6-$CH_3$ | $C_2H_5$- | $C_2H_5$- | H | $(CH_3)_2CH$- | |
| 147 | $CH_3$ | $CH_3$ | 6-$CH_3$ | $C_2H_5$- | $C_2H_5$- | H | $(CH_3)_3C$- | |
| 148 | $CH_3$ | $CH_3$ | 6-$CH_3$ | $C_2H_5$- | $C_2H_5$- | H | $(CH_3)_2$-CH-C$(CH_3)_2$- | |
| 149 | $CH_3$ | $CH_3$ | 6-$CH_3$ | $C_2H_5$- | $C_2H_5$- | H | $(CH_3)_3C$-$CH_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 150 | $CH_3$ | $CH_3$ | 6-$CH_3$ | $C_2H_5$- | $C_2H_5$- | H | $CH_3O$-$CH_2$-$C(CH_3)_2$- | |
| 151 | $CH_3$ | $CH_3$ | 6-$CH_3$ | $C_2H_5$- | $C_2H_5$- | H | $ClCH_2$-$C(CH_3)_2$- | |
| 152 | $CH_3$ | $CH_3$ | 6-$CH_3$ | $C_2H_5$- | $C_2H_5$- | H | 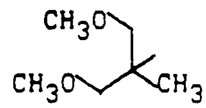 | |
| 153 | $CH_3$ | $CH_3$ | 6-$CH_3$ | $C_2H_5$- | $C_2H_5$- | H | $(CH_3)_2C$=CH- | |
| 154 | $CH_3$ | $CH_3$ | 6-$CH_3$ | $C_2H_5$- | $C_2H_5$- | H | 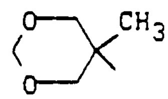 | |
| 155 | $CH_3$ | $CH_3$ | 6-$CH_3$ | $C_2H_5$- | $C_3H_7$- | H | $CH_3$- | |
| 156 | $CH_3$ | $CH_3$ | 6-$CH_3$ | $C_2H_5$- | $C_3H_7$- | H | $(CH_3)_2CH$- | |
| 157 | $CH_3$ | $CH_3$ | 6-$CH_3$ | $C_2H_5$- | $C_3H_7$- | H | $(CH_3)_3C$- | |
| 158 | $CH_3$ | $CH_3$ | 6-$CH_3$ | $C_2H_5$- | $C_3H_7$- | H | $(CH_3)_2CH$-$C(CH_3)_2$- | |
| 159 | $CH_3$ | $CH_3$ | 6-$CH_3$ | $C_2H_5$- | $(CH_3)_2CH$- | H | $CH_3$- | |
| 160 | $CH_3$ | $CH_3$ | 6-$CH_3$ | $C_2H_5$- | $(CH_3)_2CH$- | H | $(CH_3)_2CH$- | |
| 161 | $CH_3$ | $CH_3$ | 6-$CH_3$ | $C_2H_5$- | $(CH_3)_2CH$- | H | $(CH_3)_3C$- | |
| 162 | $CH_3$ | $CH_3$ | 6-$CH_3$ | $C_2H_5$- | $(CH_3)_2CH$- | H | $(CH_3)_2CH$-$C(CH_3)_2$- | |
| 163 | $CH_3$ | $CH_3$ | 6-$CH_3$ | $C_2H_5$- | $CH_3$- | $CH_3$- | $CH_3$- | |
| 164 | $CH_3$ | $CH_3$ | 6-$CH_3$ | $C_2H_5$- | $CH_3$- | $CH_3$- | $(CH_3)_2CH$- | |
| 165 | $CH_3$ | $CH_3$ | 6-$CH_3$ | $C_2H_5$- | $CH_3$- | $CH_3$- | $(CH_3)_3C$- | |
| 166 | $CH_3$ | $CH_3$ | 6-$CH_3$ | $C_2H_5$- | $CH_3$- - | $CH_3$- | $(CH_3)_2CH$-$C(CH_3)_2$- | |
| 167 | $CH_3$ | $CH_3$ | 6-$CH_3$ | $C_3H_7$ | H | H | $CH_3$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 168 | $CH_3$ | $CH_3$ | 6-$CH_3$ | $C_3H_7$- | H | H | $(CH_3)_2CH$- | |
| 169 | $CH_3$ | $CH_3$ | 6-$CH_3$ | $C_3H_7$- | H | H | $(CH_3)_3C$- | |
| 170 | $CH_3$ | $CH_3$ | 6-$CH_3$ | $C_3H_7$- | H | H | $(CH_3)_2$-CH-C$(CH_3)_2$- | |
| 171 | $CH_3$ | $CH_3$ | 6-$CH_3$ | $C_3H_7$- | H | H | $(CH_3)_3C$-$CH_2$- | |
| 172 | $CH_3$ | $CH_3$ | 6-$CH_3$ | $C_3H_7$- | H | H | $CH_3O$-$CH_2$-C$(CH_3)_2$- | |
| 173 | $CH_3$ | $CH_3$ | 6-$CH_3$ | $C_3H_7$- | H | H | $ClCH_2$-C$(CH_3)_2$- | |
| 174 | $CH_3$ | $CH_3$ | 6-$CH_3$ | $C_3H_7$- | H | H | 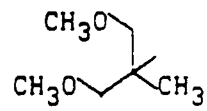 | |
| 175 | $CH_3$ | $CH_3$ | 6-$CH_3$ | $C_3H_7$- | H | H | $(CH_3)_2C$=CH- | |
| 176 | $CH_3$ | $CH_3$ | 6-$CH_3$ | $C_3H_7$- | H | H | 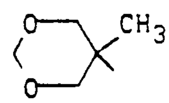 | |
| 177 | $CH_3$ | $CH_3$ | 6-$CH_3$ | $C_3H_7$- | $CH_3$- | H | $CH_3$- | |
| 178 | $CH_3$ | $CH_3$ | 6-$CH_3$ | $C_3H_7$- | $CH_3$- | H | $(CH_3)_2CH$- | |
| 179 | $CH_3$ | $CH_3$ | 6-$CH_3$ | $C_3H_7$- | $CH_3$- | H | $(CH_3)_3C$- | |
| 180 | $CH_3$ | $CH_3$ | 6-$CH_3$ | $C_3H_7$- | $CH_3$- | H | $(CH_3)_2$-CH-C$(CH_3)_2$- | |
| 181 | $CH_3$ | $CH_3$ | 6-$CH_3$ | $C_3H_7$- | $CH_3$- | H | $(CH_3)_3C$-$CH_2$- | |
| 182 | $CH_3$ | $CH_3$ | 6-$CH_3$ | $C_3H_7$- | $CH_3$- | H | $CH_3O$-$CH_2$-C$(CH_3)_2$- | |
| 183 | $CH_3$ | $CH_3$ | 6-$CH_3$ | $C_3H_7$- | $CH_3$- | H | $ClCH_2$-C$(CH_3)_2$- | |
| 184 | $CH_3$ | $CH_3$ | 6-$CH_3$ | $C_3H_7$- | $CH_3$- | H | 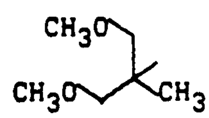 | |

Tabelle 2 (Fortsetzung)

| Bsp. Nr. | X | Y | $Z_n$ | A | B | C* | $R^1$ | Fp °C |
|---|---|---|---|---|---|---|---|---|
| 185 | $CH_3$ | $CH_3$ | $6-CH_3$ | $C_3H_7-$ | $CH_3-$ | H | $(CH_3)_2C=CH-$ | |
| 186 | $CH_3$ | $CH_3$ | $6-CH_3$ | $C_3H_7-$ | $CH_3-$ | H | 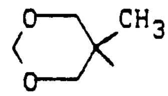 | |
| 187 | $CH_3$ | $CH_3$ | $6-CH_3$ | $C_3H_7-$ | $C_2H_5-$ | H | $CH_3-$ | |
| 188 | $CH_3$ | $CH_3$ | $6-CH_3$ | $C_3H_7-$ | $C_2H_5-$ | H | $(CH_3)_2CH-$ | |
| 189 | $CH_3$ | $CH_3$ | $6-CH_3$ | $C_3H_7-$ | $C_2H_5-$ | H | $(CH_3)_3C-$ | |
| 190 | $CH_3$ | $CH_3$ | $6-CH_3$ | $C_3H_7-$ | $C_2H_5-$ | H | $(CH_3)_2-CH-C(CH_3)_2-$ | |
| 191 | $CH_3$ | $CH_3$ | $6-CH_3$ | $C_3H_7-$ | $C_2H_5-$ | H | $(CH_3)_3C-CH_2-$ | |
| 192 | $CH_3$ | $CH_3$ | $6-CH_3$ | $C_3H_7-$ | $C_2H_5-$ | H | $CH_3O-CH_2-C(CH_3)_2-$ | |
| 193 | $CH_3$ | $CH_3$ | $6-CH_3$ | $C_3H_7-$ | $C_2H_5-$ | H | $ClCH_2-C(CH_3)_2-$ | |
| 194 | $CH_3$ | $CH_3$ | $6-CH_3$ | $C_3H_7-$ | $C_2H_5-$ | H | 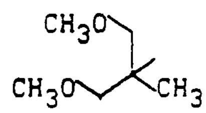 | |
| 195 | $CH_3$ | $CH_3$ | $6-CH_3$ | $C_3H_7-$ | $C_2H_5-$ | H | $(CH_3)_2C=CH-$ | |
| 196 | $CH_3$ | $CH_3$ | $6-CH_3$ | $C_3H_7-$ | $C_2H_5-$ | H | 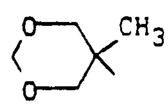 | |
| 197 | $CH_3$ | $CH_3$ | $6-CH_3$ | $C_3H_7-$ | $C_3H_7-$ | H | $CH_3-$ | |
| 198 | $CH_3$ | $CH_3$ | $6-CH_3$ | $C_3H_7-$ | $C_3H_7-$ | H | $(CH_3)_2CH-$ | |
| 199 | $CH_3$ | $CH_3$ | $6-CH_3$ | $C_3H_7-$ | $C_3H_7-$ | H | $(CH_3)_3C-$ | |
| 200 | $CH_3$ | $CH_3$ | $6-CH_3$ | $C_3H_7-$ | $C_3H_7--$ | H | $(CH_3)_2CH-C(CH_3)_2-$ | |
| 201 | $CH_3$ | $CH_3$ | $6-CH_3$ | $C_3H_7-$ | $(CH_3)_2CH-$ | H | $CH_3-$ | |
| 202 | $CH_3$ | $CH_3$ | $6-CH_3$ | $C_3H_7-$ | $(CH_3)_2CH-$ | H | $(CH_3)_2CH-$ | |

Tabelle 2 (Fortsetzung)

| Bsp. Nr. | X | Y | $Z_n$ | A | B | C* | $R^1$ | Fp °C |
|---|---|---|---|---|---|---|---|---|
| 203 | $CH_3$ | $CH_3$ | 6-$CH_3$ | $C_3H_7$- | $(CH_3)_2CH$- | H | $(CH_3)_3C$- | |
| 204 | $CH_3$ | $CH_3$ | 6-$CH_3$ | $C_3H_7$- | $(CH_3)_2CH$- | H | $(CH_3)_2CH-C(CH_3)_2$- | |
| 205 | $CH_3$ | $CH_3$ | 6-$CH_3$ | $C_3H_7$- | $CH_3$- | $CH_3$- | $CH_3$- | |
| 206 | $CH_3$ | $CH_3$ | 6-$CH_3$ | $C_3H_7$- | $CH_3$- | $CH_3$- | $(CH_3)_2CH$- | |
| 207 | $CH_3$ | $CH_3$ | 6-$CH_3$ | $C_3H_7$- | $CH_3$- | $CH_3$- | $(CH_3)_3C$- | |
| 208 | $CH_3$ | $CH_3$ | 6-$CH_3$ | $C_3H_7$- | $CH_3$- | $CH_3$- | $(CH_3)_2CH-C(CH_3)_2$- | |
| 209 | $CH_3$ | $CH_3$ | 6-$CH_3$ | $(CH_3)_2CH$- | H | H | $CH_3$- | 75 |
| 210 | $CH_3$ | $CH_3$ | 6-$CH_3$ | $(CH_3)_2CH$- | H | H | $(CH_3)_2CH$- | 80 |
| 211 | $CH_3$ | $CH_3$ | 6-$CH_3$ | $(CH_3)_2CH$- | H | H | $(CH_3)_3C$- | 86 |
| 212 | $CH_3$ | $CH_3$ | 6-$CH_3$ | $(CH_3)_2CH$- | H | H | $(CH_3)_2CH-C(CH_3)_2$- | 108 |
| 213 | $CH_3$ | $CH_3$ | 6-$CH_3$ | $(CH_3)_2CH$- | H | H | $(CH_3)_3C-CH_2$- | 74 |
| 214 | $CH_3$ | $CH_3$ | 6-$CH_3$ | $(CH_3)_2CH$- | H | H | $CH_3O-CH_2-C(CH_3)_2$- | 68 |
| 215 | $CH_3$ | $CH_3$ | 6-$CH_3$ | $(CH_3)_2CH$- | H | H | $ClCH_2-C(CH_3)_2$- | 153 |
| 216 | $CH_3$ | $CH_3$ | 6-$CH_3$ | $(CH_3)_2CH$- | H | H | 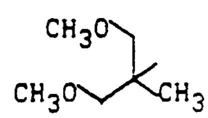 | |
| 217 | $CH_3$ | $CH_3$ | 6-$CH_3$ | $(CH_3)_2CH$- | H | H | $(CH_3)_2C=CH$- | 67 |
| 218 | $CH_3$ | $CH_3$ | 6-$CH_3$ | $(CH_3)_2CH$- | H | H | 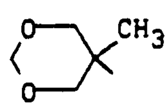 | |
| 219 | $CH_3$ | $CH_3$ | 6-$CH_3$ | $(CH_3)_2CH$- | H | H | 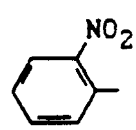 | |

Tabelle 2 (Fortsetzung)

| Bsp. Nr. | X | Y | $Z_n$ | A | B | C* | $R^1$ | Fp °C |
|---|---|---|---|---|---|---|---|---|
| 220 | $CH_3$ | $CH_3$ | 6-$CH_3$ | $(CH_3)_2CH-$ | H | H | 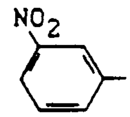 | |
| 221 | $CH_3$ | $CH_3$ | 6-$CH_3$ | $(CH_3)_2CH-$ | H | H | 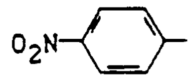 | |
| 222 | $CH_3$ | $CH_3$ | 6-$CH_3$ | $(CH_3)_2CH-$ | H | H | 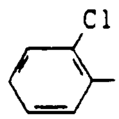 | |
| 223 | $CH_3$ | $CH_3$ | 6-$CH_3$ | $(CH_3)_2CH-$ | H | H | 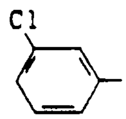 | |
| 224 | $CH_3$ | $CH_3$ | 6-$CH_3$ | $(CH_3)_2CH-$ | H | H | 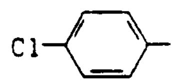 | |
| 225 | $CH_3$ | $CH_3$ | 6-$CH_3$ | $(CH_3)_2CH-$ | H | H | 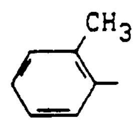 | |
| 226 | $CH_3$ | $CH_3$ | 6-$CH_3$ | $(CH_3)_2CH-$ | H | H | 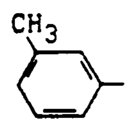 | |
| 227 | $CH_3$ | $CH_3$ | 6-$CH_3$ | $(CH_3)_2CH-$ | H | H | 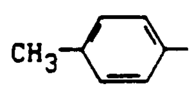 | |
| 228 | $CH_3$ | $CH_3$ | 6-$CH_3$ | $(CH_3)_2CH-$ | H | H | 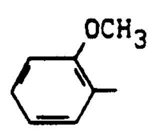 | |

Tabelle 2 (Fortsetzung)

| Bsp. Nr. | X | Y | $Z_n$ | A | B | C* | $R^1$ | Fp °C |
|---|---|---|---|---|---|---|---|---|
| 229 | $CH_3$ | $CH_3$ | 6-$CH_3$ | $(CH_3)_2CH$- | H | H | 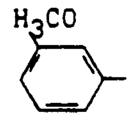 | |
| 230 | $CH_3$ | $CH_3$ | 6-$CH_3$ | $(CH_3)_2CH$- | H | H | 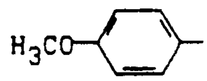 | |
| 231 | $CH_3$ | $CH_3$ | 6-$CH_3$ | $(CH_3)_2CH$- | $CH_3$ | H | $CH_3$- | Öl |
| 232 | $CH_3$ | $CH_3$ | 6-$CH_3$ | $(CH_3)_2CH$- | $CH_3$ | H | $(CH_3)_2CH$- | |
| 233 | $CH_3$ | $CH_3$ | 6-$CH_3$ | $(CH_3)_2CH$- | $CH_3$ | H | $(CH_3)_3C$- | 94 |
| 234 | $CH_3$ | $CH_3$ | 6-$CH_3$ | $(CH_3)_2CH$- | $CH_3$ | H | $(CH_3)_2$-CH-$C(CH_3)_2$- | |
| 235 | $CH_3$ | $CH_3$ | 6-$CH_3$ | $(CH_3)_2CH$- | $CH_3$ | H | $(CH_3)_3C$-$CH_2$- | Öl |
| 236 | $CH_3$ | $CH_3$ | 6-$CH_3$ | $(CH_3)_2CH$- | $CH_3$ | H | $CH_3O$-$CH_2$-$C(CH_3)_2$- | |
| 237 | $CH_3$ | $CH_3$ | 6-$CH_3$ | $(CH_3)_2CH$- | $CH_3$ | H | $ClCH_2$-$C(CH_3)_2$- | 112 |
| 238 | $CH_3$ | $CH_3$ | 6-$CH_3$ | $(CH_3)_2CH$- | $CH_3$ | H | 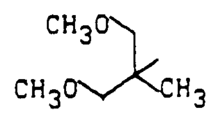 | |
| 239 | $CH_3$ | $CH_3$ | 6-$CH_3$ | $(CH_3)_2CH$- | $CH_3$ | H | $(CH_3)_2C$=CH- | |
| 240 | $CH_3$ | $CH_3$ | 6-$CH_3$ | $(CH_3)_2CH$- | $CH_3$ | H | 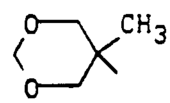 | |
| 241 | $CH_3$ | $CH_3$ | 6-$CH_3$ | $(CH_3)_2CH$- | $C_2H_5$- | H | $CH_3$- | |
| 242 | $CH_3$ | $CH_3$ | 6-$CH_3$ | $(CH_3)_2CH$- | $C_2H_5$- | H | $(CH_3)_2CH$- | |
| 243 | $CH_3$ | $CH_3$ | 6-$CH_3$ | $(CH_3)_2CH$- | $C_2H_5$- | H | $(CH_3)_3C$- | |
| 244 | $CH_3$ | $CH_3$ | 6-$CH_3$ | $(CH_3)_2CH$- | $C_2H_5$- | H | $(CH_3)_2$-CH-$C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 245 | $CH_3$ | $CH_3$ | $6\text{-}CH_3$ | $(CH_3)_2CH\text{-}$ | $C_2H_5\text{-}$ | H | $(CH_3)_3C\text{-}CH_2\text{-}$ | |
| 246 | $CH_3$ | $CH_3$ | $6\text{-}CH_3$ | $(CH_3)_2CH\text{-}$ | $C_2H_5\text{-}$ | H | $CH_3O\text{-}CH_2\text{-}C(CH_3)_2\text{-}$ | |
| 247 | $CH_3$ | $CH_3$ | $6\text{-}CH_3$ | $(CH_3)_2CH\text{-}$ | $C_2H_5\text{-}$ | H | $ClCH_2\text{-}C(CH_3)_2\text{-}$ | |
| 248 | $CH_3$ | $CH_3$ | $6\text{-}CH_3$ | $(CH_3)_2CH\text{-}$ | $C_2H_5\text{-}$ | H | 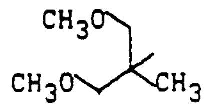 | |
| 249 | $CH_3$ | $CH_3$ | $6\text{-}CH_3$ | $(CH_3)_2CH\text{-}$ | $C_2H_5\text{-}$ | H | $(CH_3)_2C{=}CH\text{-}$ | |
| 250 | $CH_3$ | $CH_3$ | $6\text{-}CH_3$ | $(CH_3)_2CH\text{-}$ | $C_2H_5\text{-}$ | H | 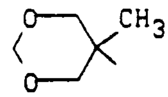 | |
| 251 | $CH_3$ | $CH_3$ | $6\text{-}CH_3$ | $(CH_3)_2CH\text{-}$ | $C_3H_7\text{-}$ | H | $CH_3\text{-}$ | |
| 252 | $CH_3$ | $CH_3$ | $6\text{-}CH_3$ | $(CH_3)_2CH\text{-}$ | $C_3H_7\text{-}$ | H | $(CH_3)_2CH\text{-}$ | |
| 253 | $CH_3$ | $CH_3$ | $6\text{-}CH_3$ | $(CH_3)_2CH\text{-}$ | $C_3H_7\text{-}$ | H | $(CH_3)_3C\text{-}$ | |
| 254 | $CH_3$ | $CH_3$ | $6\text{-}CH_3$ | $(CH_3)_2CH\text{-}$ | $C_3H_7\text{-}$ | H | $(CH_3)_2CH\text{-}C(CH_3)_2\text{-}$ | |
| 255 | $CH_3$ | $CH_3$ | $6\text{-}CH_3$ | $(CH_3)_2CH\text{-}$ | $(CH_3)_2CH\text{-}$ | H | $CH_3\text{-}$ | |
| 256 | $CH_3$ | $CH_3$ | $6\text{-}CH_3$ | $(CH_3)_2CH\text{-}$ | $(CH_3)_2CH\text{-}$ | H | $(CH_3)_2CH\text{-}$ | |
| 257 | $CH_3$ | $CH_3$ | $6\text{-}CH_3$ | $(CH_3)_2CH\text{-}$ | $(CH_3)_2CH\text{-}$ | H | $(CH_3)_3C\text{-}$ | |
| 258 | $CH_3$ | $CH_3$ | $6\text{-}CH_3$ | $(CH_3)_2CH\text{-}$ | $(CH_3)_2CH\text{-}$ | H | $(CH_3)_2CH\text{-}C(CH_3)_2\text{-}$ | |
| 259 | $CH_3$ | $CH_3$ | $6\text{-}CH_3$ | $(CH_3)_2CH\text{-}$ | $CH_3\text{-}$ | $CH_3\text{-}$ | $CH_3\text{-}$ | |
| 260 | $CH_3$ | $CH_3$ | $6\text{-}CH_3$ | $(CH_3)_2CH\text{-}$ | $CH_3\text{-}$ | $CH_3\text{-}$ | $(CH_3)_2CH\text{-}$ | |
| 261 | $CH_3$ | $CH_3$ | $6\text{-}CH_3$ | $(CH_3)_2CH\text{-}$ | $CH_3\text{-}$ | $CH_3\text{-}$ | $(CH_3)_3C\text{-}$ | |
| 262 | $CH_3$ | $CH_3$ | $6\text{-}CH_3$ | $(CH_3)_2CH\text{-}$ | $CH_3\text{-}$ | $CH_3\text{-}$ | $(CH_3)_2CH\text{-}C(CH_3)_2\text{-}$ | |

Tabelle 2 (Fortsetzung)

| Bsp. Nr. | X | Y | $Z_n$ | A | B | C* | $R^1$ | Fp °C |
|---|---|---|---|---|---|---|---|---|
| 263 | $CH_3$ | $CH_3$ | 6-$CH_3$ | $C_4H_9$- | H | H | $CH_3$- | |
| 264 | $CH_3$ | $CH_3$ | 6-$CH_3$ | $C_4H_9$- | H | H | $(CH_3)_2CH$- | |
| 265 | $CH_3$ | $CH_3$ | 6-$CH_3$ | $C_4H_9$- | H | H | $(CH_3)_3C$- | |
| 266 | $CH_3$ | $CH_3$ | 6-$CH_3$ | $C_4H_9$- | H | H | $(CH_3)_2CH\text{-}C(CH_3)_2$- | |
| 267 | $CH_3$ | $CH_3$ | 6-$CH_3$ | $C_4H_9$- | $CH_3$- | H | $CH_3$- | |
| 268 | $CH_3$ | $CH_3$ | 6-$CH_3$ | $C_4H_9$- | $CH_3$- | H | $(CH_3)_2CH$- | |
| 269 | $CH_3$ | $CH_3$ | 6-$CH_3$ | $C_4H_9$- | $CH_3$- | H | $(CH_3)_3C$- | |
| 270 | $CH_3$ | $CH_3$ | 6-$CH_3$ | $C_4H_9$- | $CH_3$- | H | $(CH_3)_2CH\text{-}C(CH_3)_2$- | |
| 271 | $CH_3$ | $CH_3$ | 6-$CH_3$ | $(CH_3)_2CH\text{-}CH_2$- | H | H | $CH_3$- | Öl |
| 272 | $CH_3$ | $CH_3$ | 6-$CH_3$ | $(CH_3)_2CH\text{-}CH_2$- | H | H | $(CH_3)_2CH$- | Öl |
| 273 | $CH_3$ | $CH_3$ | 6-$CH_3$ | $(CH_3)_2CH\text{-}CH_2$- | H | H | $(CH_3)_3C$- | Öl |
| 274 | $CH_3$ | $CH_3$ | 6-$CH_3$ | $(CH_3)_2CH\text{-}CH_2$- | H | H | $(CH_3)_2CH\text{-}C(CH_3)_2$- | Öl |
| 275 | $CH_3$ | $CH_3$ | 6-$CH_3$ | $(CH_3)_2CH\text{-}CH_2$- | $CH_3$- | H | $CH_3$- | 73 |
| 276 | $CH_3$ | $CH_3$ | 6-$CH_3$ | $(CH_3)_2CH\text{-}CH_2$- | $CH_3$- | H | $(CH_3)_3C$- | Öl |
| 277 | $CH_3$ | $CH_3$ | 6-$CH_3$ | $(CH_3)_2CH\text{-}CH_2$- | $CH_3$- | H | $(CH_3)_3C\text{-}CH_2$- | Öl |
| 278 | $CH_3$ | $CH_3$ | 6-$CH_3$ | $(CH_3)_2CH\text{-}CH_2$- | $CH_3$- | H | $(CH_3)_2CH\text{-}C(CH_3)_2$- | |
| 279 | $CH_3$ | $CH_3$ | 6-$CH_3$ | 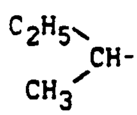 | H | H | $CH_3$- | Öl |
| 280 | $CH_3$ | $CH_3$ | 6-$CH_3$ | 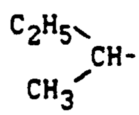 | H | H | $(CH_3)_2CH$- | 66 |

Tabelle 2 (Fortsetzung)

| Bsp. Nr. | X | Y | $Z_n$ | A | B | C* | $R^1$ | Fp °C |
|---|---|---|---|---|---|---|---|---|
| 281 | $CH_3$ | $CH_3$ | 6-$CH_3$ | 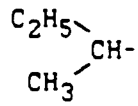 | H | H | $(CH_3)_3C$- | 99 |
| 282 | $CH_3$ | $CH_3$ | 6-$CH_3$ | 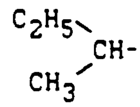 | H | H | $(CH_3)_2CH-C(CH_3)_2$- | 66 |
| 283 | $CH_3$ | $CH_3$ | 6-$CH_3$ | 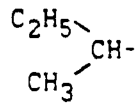 | $CH_3$- | H | $CH_3$- | Öl |
| 284 | $CH_3$ | $CH_3$ | 6-$CH_3$ | 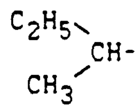 | $CH_3$- | H | $(CH_3)_2CH$- | |
| 285 | $CH_3$ | $CH_3$ | 6-$CH_3$ | 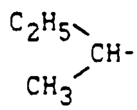 | $CH_3$- | H | $(CH_3)_3C$- | 100 |
| 286 | $CH_3$ | $CH_3$ | 6-$CH_3$ | 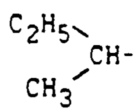 | $CH_3$- | H | $(CH_3)_2CH-C(CH_3)_2$- | |
| 287 | $CH_3$ | $CH_3$ | 6-$CH_3$ | $(CH_3)_3C$- | H | H | $(CH_3)_2CH$- | Öl |
| 288 | $CH_3$ | $CH_3$ | 6-$CH_3$ | $(CH_3)_3C$- | H | H | $(CH_3)_3C$- | 85 |
| 289 | $CH_3$ | $CH_3$ | 6-$CH_3$ | $(CH_3)_3C$- | H | H | $(CH_3)_2CH-C(CH_3)_2$- | 107 |
| 290 | $CH_3$ | $CH_3$ | 6-$CH_3$ | $(CH_3)_3C-CH_2$- | H | H | $CH_3$- | Öl |
| 291 | $CH_3$ | $CH_3$ | 6-$CH_3$ | $(CH_3)_3C-CH_2$- | H | H | $(CH_3)_2CH$- | |
| 292 | $CH_3$ | $CH_3$ | 6-$CH_3$ | $(CH_3)_3C-CH_2$- | H | H | $(CH_3)_3C$- | 83 |
| 293 | $CH_3$ | $CH_3$ | 6-$CH_3$ | $(CH_3)_3C-CH_2$- | H | H | $(CH_3)_2CH-C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 294 | $CH_3$ | $CH_3$ | 6-$CH_3$ | $(CH_3)_2CH-CH(CH_3)-$ | H | H | $CH_3-$ | |
| 295 | $CH_3$ | $CH_3$ | 6-$CH_3$ | $(CH_3)_2CH-CH(CH_3)-$ | H | H | $(CH_3)_2CH-$ | |
| 296 | $CH_3$ | $CH_3$ | 6-$CH_3$ | $(CH_3)_2CH-CH(CH_3)-$ | H | H | $(CH_3)_3C-$ | |
| 297 | $CH_3$ | $CH_3$ | 6-$CH_3$ | $(CH_3)_2CH-CH(CH_3)-$ | H | H | $(CH_3)_2CH-C(CH_3)_2-$ | |
| 298 | $CH_3$ | $CH_3$ | 6-$CH_3$ | $(CH_3)_3C-CH(CH_3)-$ | H | H | $CH_3-$ | Öl |
| 299 | $CH_3$ | $CH_3$ | 6-$CH_3$ | $(CH_3)_3C-CH(CH_3)-$ | H | H | $(CH_3)_2CH-$ | |
| 300 | $CH_3$ | $CH_3$ | 6-$CH_3$ | $(CH_3)_3C-CH(CH_3)-$ | H | H | $(CH_3)_3C-$ | 92 |
| 301 | $CH_3$ | $CH_3$ | 6-$CH_3$ | $(CH_3)_3C-CH(CH_3)-$ | H | H | $(CH_3)_2CH-C(CH_3)_2-$ | |
| 302 | $CH_3$ | $CH_3$ | 6-$CH_3$ | $CH_2=CH-CH_2-$ | H | H | $CH_3-$ | Öl |
| 303 | $CH_3$ | $CH_3$ | 6-$CH_3$ | $CH_2=CH-CH_2-$ | H | H | $(CH_3)_2CH-$ | |
| 304 | $CH_3$ | $CH_3$ | 6-$CH_3$ | $CH_2=CH-CH_2-$ | H | H | $(CH_3)_3C-$ | Öl |
| 305 | $CH_3$ | $CH_3$ | 6-$CH_3$ | $CH_2=CH-CH_2-$ | H | H | $(CH_3)_2CH-C(CH_3)_2-$ | |
| 306 | $CH_3$ | $CH_3$ | 6-$CH_3$ | $CH_2=CH-CH_2-$ | $CH_3-$ | H | $CH_3-$ | |
| 307 | $CH_3$ | $CH_3$ | 6-$CH_3$ | $CH_2=CH-CH_2-$ | $CH_3-$ | H | $(CH_3)_2CH-$ | |
| 308 | $CH_3$ | $CH_3$ | 6-$CH_3$ | $CH_2=CH-CH_2-$ | $CH_3-$ | H | $(CH_3)_3C-$ | |
| 309 | $CH_3$ | $CH_3$ | 6-$CH_3$ | $CH_2=CH-CH_2-$ | $CH_3-$ | H | $(CH_3)_2CH-C(CH_3)_2-$ | |
| 310 | $CH_3$ | $CH_3$ | 6-$CH_3$ | 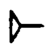 | H | H | $CH_3-$ | Öl |
| 311 | $CH_3$ | $CH_3$ | 6-$CH_3$ | 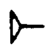 | H | H | $(CH_3)_2CH-$ | 35 |
| 312 | $CH_3$ | $CH_3$ | 6-$CH_3$ | 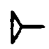 | H | H | $(CH_3)_3C-$ | 75 |
| 313 | $CH_3$ | $CH_3$ | 6-$CH_3$ | 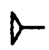 | H | H | $(CH_3)_2CH-C(CH_3)_2-$ | |

Tabelle 2 (Fortsetzung)

| Bsp. Nr. | X | Y | $Z_n$ | A | B | C* | $R^1$ | Fp °C |
|---|---|---|---|---|---|---|---|---|
| 314 | $CH_3$ | $CH_3$ | 6-$CH_3$ | 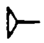 | H | H | $(CH_3)_3C-CH_2-$ | |
| 315 | $CH_3$ | $CH_3$ | 6-$CH_3$ | 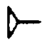 | H | H | $CH_3O-CH_2-C(CH_3)_2-$ | |
| 316 | $CH_3$ | $CH_3$ | 6-$CH_3$ | 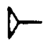 | H | H | $ClCH_2-C(CH_3)_2-$ | |
| 317 | $CH_3$ | $CH_3$ | 6-$CH_3$ | 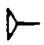 | H | H | 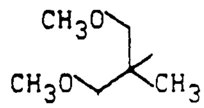 | |
| 318 | $CH_3$ | $CH_3$ | 6-$CH_3$ | 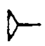 | H | H | $(CH_3)_2C=CH-$ | |
| 319 | $CH_3$ | $CH_3$ | 6-$CH_3$ | 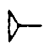 | H | H | 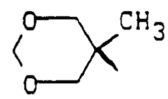 | |
| 320 | $CH_3$ | $CH_3$ | 6-$CH_3$ | 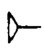 | $CH_3$ | H | $CH_3-$ | 83 |
| 321 | $CH_3$ | $CH_3$ | 6-$CH_3$ | 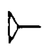 | $CH_3$ | H | $(CH_3)_2CH-$ | |
| 322 | $CH_3$ | $CH_3$ | 6-$CH_3$ | 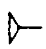 | $CH_3$ | H | $(CH_3)_3C-$ | Öl |
| 323 | $CH_3$ | $CH_3$ | 6-$CH_3$ | 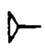 | $CH_3$ | H | $(CH_3)_2-CH-C(CH_3)_2-$ | |
| 324 | $CH_3$ | $CH_3$ | 6-$CH_3$ | 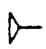 | $CH_3$ | H | $(CH_3)_3C-CH_2-$ | Öl |
| 325 | $CH_3$ | $CH_3$ | 6-$CH_3$ | 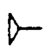 | $CH_3$ | H | $CH_3O-CH_2-C(CH_3)_2-$ | |
| 326 | $CH_3$ | $CH_3$ | 6-$CH_3$ | 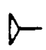 | $CH_3$ | H | $ClCH_2-C(CH_3)_2-$ | |
| 327 | $CH_3$ | $CH_3$ | 6-$CH_3$ | 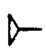 | $CH_3$ | H | 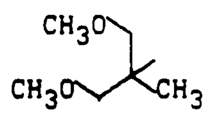 | |
| 328 | $CH_3$ | $CH_3$ | 6-$CH_3$ | 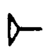 | $CH_3$ | H | $(CH_3)_2C=CH-$ | |
| 329 | $CH_3$ | $CH_3$ | 6-$CH_3$ | 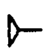 | $CH_3$ | H | 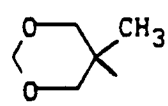 | |

Tabelle 2 (Fortsetzung)

| Bsp. Nr. | X | Y | $Z_n$ | A | B | C* | $R^1$ | Fp °C |
|---|---|---|---|---|---|---|---|---|
| 330 | $CH_3$ | $CH_3$ | 6-$CH_3$ | 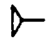 | $C_2H_5$- | H | $CH_3$- | |
| 331 | $CH_3$ | $CH_3$ | 6-$CH_3$ | 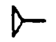 | $C_2H_5$- | H | $(CH_3)_2CH$- | |
| 332 | $CH_3$ | $CH_3$ | 6-$CH_3$ | 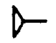 | $C_2H_5$- | H | $(CH_3)_3C$- | |
| 333 | $CH_3$ | $CH_3$ | 6-$CH_3$ | 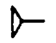 | $C_2H_5$- | H | $(CH_3)_2$-CH-C$(CH_3)_2$- | |
| 334 | $CH_3$ | $CH_3$ | 6-$CH_3$ | 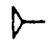 | $C_2H_5$- | H | $(CH_3)_3C$-$CH_2$- | |
| 365 | $CH_3$ | $CH_3$ | 6-$CH_3$ | 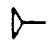 | $C_2H_5$- | H | $CH_3O$-$CH_2$-C$(CH_3)_2$- | |
| 336 | $CH_3$ | $CH_3$ | 6-$CH_3$ | 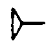 | $C_2H_5$- | H | Cl$CH_2$-C$(CH_3)_2$- | |
| 337 | $CH_3$ | $CH_3$ | 6-$CH_3$ | 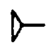 | $C_2H_5$- | H | 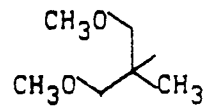 | |
| 338 | $CH_3$ | $CH_3$ | 6-$CH_3$ | 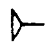 | $C_2H_5$- | H | $(CH_3)_2C$=CH- | |
| 339 | $CH_3$ | $CH_3$ | 6-$CH_3$ | 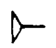 | $C_2H_5$- | H | 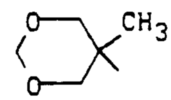 | |
| 340 | $CH_3$ | $CH_3$ | 6-$CH_3$ | 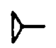 | $C_3H_7$- | H | $CH_3$- | |
| 341 | $CH_3$ | $CH_3$ | 6-$CH_3$ | 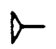 | $C_3H_7$- | H | $(CH_3)_2CH$- | |
| 342 | $CH_3$ | $CH_3$ | 6-$CH_3$ | 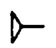 | $C_3H_7$- | H | $(CH_3)_3C$- | |
| 343 | $CH_3$ | $CH_3$ | 6-$CH_3$ | 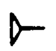 | $C_3H_7$- | H | $(CH_3)_2CH$-C$(CH_3)_2$- | |
| 344 | $CH_3$ | $CH_3$ | 6-$CH_3$ | 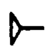 | $(CH_3)_2CH$- | H | $CH_3$- | |
| 345 | $CH_3$ | $CH_3$ | 6-$CH_3$ | 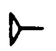 | $(CH_3)_2CH$- | H | $(CH_3)_2CH$- | |
| 346 | $CH_3$ | $CH_3$ | 6-$CH_3$ | 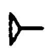 | $(CH_3)_2CH$- | H | $(CH_3)_3C$- | |
| 347 | $CH_3$ | $CH_3$ | 6-$CH_3$ | 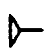 | $(CH_3)_2CH$- | H | $(CH_3)_2CH$-C$(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 348 | $CH_3$ | $CH_3$ | 6-$CH_3$ | 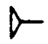 | $CH_3$- | $CH_3$- | $CH_3$- | |
| 349 | $CH_3$ | $CH_3$ | 6-$CH_3$ | 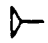 | $CH_3$- | $CH_3$- | $(CH_3)_2CH$- | |
| 350 | $CH_3$ | $CH_3$ | 6-$CH_3$ | 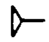 | $CH_3$- | $CH_3$- | $(CH_3)_3C$- | |
| 351 | $CH_3$ | $CH_3$ | 6-$CH_3$ | 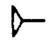 | $CH_3$- | $CH_3$- | $(CH_3)_2CH-C(CH_3)_2$- | |
| 352 | $CH_3$ | $CH_3$ | 6-$CH_3$ | 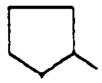 | H | H | $CH_3$- | 60 |
| 353 | $CH_3$ | $CH_3$ | 6-$CH_3$ | 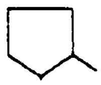 | H | H | $(CH_3)_2CH$- | Öl |
| 354 | $CH_3$ | $CH_3$ | 6-$CH_3$ | 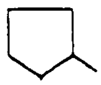 | H | H | $(CH_3)_3C$- | 80 |
| 355 | $CH_3$ | $CH_3$ | 6-$CH_3$ | 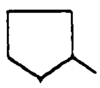 | H | H | $(CH_3)_2$-$CH$-$C(CH_3)_2$- | 85 |
| 356 | $CH_3$ | $CH_3$ | 6-$CH_3$ | 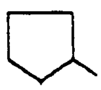 | H | H | $(CH_3)_3C$-$CH_2$- | 74 |
| 357 | $CH_3$ | $CH_3$ | 6-$CH_3$ | 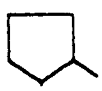 | H | H | $CH_3O$-$CH_2$-$C(CH_3)_2$- | |
| 358 | $CH_3$ | $CH_3$ | 6-$CH_3$ | 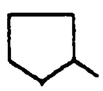 | H | H | $ClCH_2$-$C(CH_3)_2$- | 94 |
| 359 | $CH_3$ | $CH_3$ | 6-$CH_3$ | 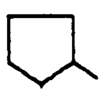 | H | H | 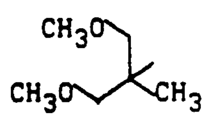 | 39 |

Tabelle 2 (Fortsetzung)

| Bsp. Nr. | X | Y | $Z_n$ | A | B | C* | $R^1$ | Fp °C |
|---|---|---|---|---|---|---|---|---|
| 360 | $CH_3$ | $CH_3$ | 6-$CH_3$ | 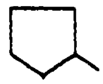 | H | H | $(CH_3)_2C{=}CH-$ | Öl |
| 361 | $CH_3$ | $CH_3$ | 6-$CH_3$ | 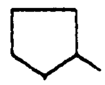 | H | H | 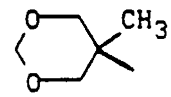 | |
| 362 | $CH_3$ | $CH_3$ | 6-$CH_3$ | 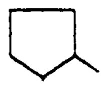 | $CH_3$- | H | $CH_3$- | 96 |
| 363 | $CH_3$ | $CH_3$ | 6-$CH_3$ | 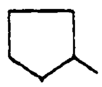 | $CH_3$- | H | $(CH_3)_2CH-$ | Öl |
| 364 | $CH_3$ | $CH_3$ | 6-$CH_3$ | 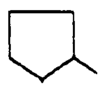 | $CH_3$- | H | $(CH_3)_3C-$ | 63 |
| 365 | $CH_3$ | $CH_3$ | 6-$CH_3$ | 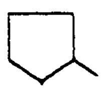 | $CH_3$- | H | $(CH_3)_2CH-C(CH_3)_2-$ | Öl |
| 366 | $CH_3$ | $CH_3$ | 6-$CH_3$ | 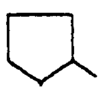 | $CH_3$- | H | $(CH_3)_3C-CH_2-$ | |
| 367 | $CH_3$ | $CH_3$ | 6-$CH_3$ | 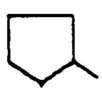 | $CH_3$- | H | $CH_3O-CH_2-C(CH_3)_2-$ | |
| 368 | $CH_3$ | $CH_3$ | 6-$CH_3$ | 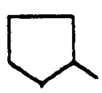 | $CH_3$- | H | $ClCH_2-C(CH_3)_2-$ | |
| 369 | $CH_3$ | $CH_3$ | 6-$CH_3$ | 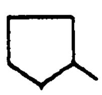 | $CH_3$- | H | 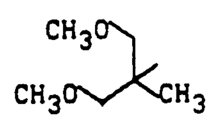 | |

Tabelle 2 (Fortsetzung)

| Bsp. Nr. | X | Y | $Z_n$ | A | B | C* | $R^1$ | Fp °C |
|---|---|---|---|---|---|---|---|---|
| 370 | $CH_3$ | $CH_3$ | 6-$CH_3$ | 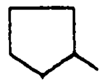 | $CH_3$- | H | $(CH_3)_2C$=CH- | |
| 371 | $CH_3$ | $CH_3$ | 6-$CH_3$ | 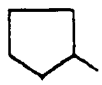 | $CH_3$- | H | 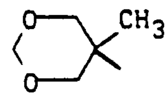 | |
| 372 | $CH_3$ | $CH_3$ | 6-$CH_3$ | 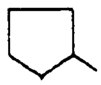 | $C_2H_5$- | H | $CH_3$- | |
| 373 | $CH_3$ | $CH_3$ | 6-$CH_3$ | 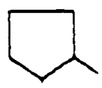 | $C_2H_5$- | H | $(CH_3)_2CH$- | |
| 374 | $CH_3$ | $CH_3$ | 6-$CH_3$ | 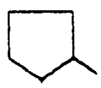 | $C_2H_5$- | H | $(CH_3)_3C$- | |
| 375 | $CH_3$ | $CH_3$ | 6-$CH_3$ | 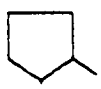 | $C_2H_5$- | H | $(CH_3)_2$-CH-C$(CH_3)_2$- | |
| 376 | $CH_3$ | $CH_3$ | 6-$CH_3$ | 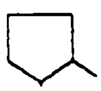 | $C_2H_5$- | H | $(CH_3)_3C$-$CH_2$- | |
| 377 | $CH_3$ | $CH_3$ | 6-$CH_3$ | 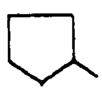 | $C_2H_5$- | H | $CH_3O$-$CH_2$-C$(CH_3)_2$- | |
| 378 | $CH_3$ | $CH_3$ | 6-$CH_3$ | 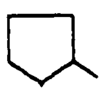 | $C_2H_5$- | H | $ClCH_2$-C$(CH_3)_2$- | |
| 379 | $CH_3$ | $CH_3$ | 6-$CH_3$ | 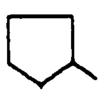 | $C_2H_5$- | H | 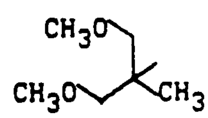 | |

Tabelle 2 (Fortsetzung)

| Bsp. Nr. | X | Y | $Z_n$ | A | B | C* | $R^1$ | Fp °C |
|---|---|---|---|---|---|---|---|---|
| 380 | $CH_3$ | $CH_3$ | 6-$CH_3$ | 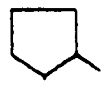 | $C_2H_5$- | H | $(CH_3)_2C$=CH- | |
| 381 | $CH_3$ | $CH_3$ | 6-$CH_3$ | 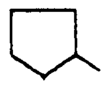 | $C_2H_5$- | H | 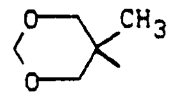 | |
| 382 | $CH_3$ | $CH_3$ | 6-$CH_3$ | 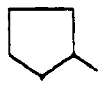 | $C_3H_7$- | H | $CH_3$- | |
| 383 | $CH_3$ | $CH_3$ | 6-$CH_3$ | 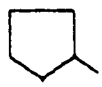 | $C_3H_7$- | H | $(CH_3)_2CH$- | |
| 384 | $CH_3$ | $CH_3$ | 6-$CH_3$ | 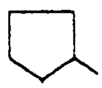 | $C_3H_7$- | H | $(CH_3)_3C$- | |
| 385 | $CH_3$ | $CH_3$ | 6-$CH_3$ | 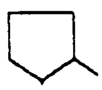 | $C_3H_7$- | H | $(CH_3)_2CH$-$C(CH_3)_2$- | |
| 386 | $CH_3$ | $CH_3$ | 6-$CH_3$ | 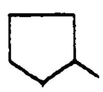 | $(CH_3)_2CH$- | H | $CH_3$- | |
| 387 | $CH_3$ | $CH_3$ | 6-$CH_3$ | 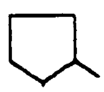 | $(CH_3)_2CH$- | H | $(CH_3)_2CH$- | |
| 388 | $CH_3$ | $CH_3$ | 6-$CH_3$ | 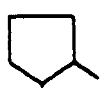 | $(CH_3)_2CH$- | H | $(CH_3)_3C$- | |
| 389 | $CH_3$ | $CH_3$ | 6-$CH_3$ | 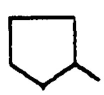 | $(CH_3)_2CH$- | H | $(CH_3)_2CH$-$C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 390 | $CH_3$ | $CH_3$ | 6-$CH_3$ | 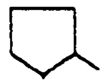 | $CH_3$- | $CH_3$- | $CH_3$- | |
| 391 | $CH_3$ | $CH_3$ | 6-$CH_3$ | 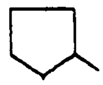 | $CH_3$- | $CH_3$- | $(CH_3)_2CH$- | |
| 392 | $CH_3$ | $CH_3$ | 6-$CH_3$ | 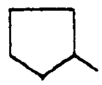 | $CH_3$- | $CH_3$- | $(CH_3)_3C$- | |
| 393 | $CH_3$ | $CH_3$ | 6-$CH_3$ | 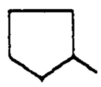 | $CH_3$- | $CH_3$- | $(CH_3)_2CH-C(CH_3)_2$- | |
| 394 | $CH_3$ | $CH_3$ | 6-$CH_3$ | 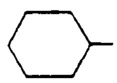 | H | H | $CH_3$- | 78 |
| 395 | $CH_3$ | $CH_3$ | 6-$CH_3$ | 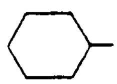 | H | H | $(CH_3)_2CH$- | Öl |
| 396 | $CH_3$ | $CH_3$ | 6-$CH_3$ | 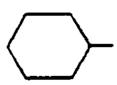 | H | H | $(CH_3)_3C$- | 97 |
| 397 | $CH_3$ | $CH_3$ | 6-$CH_3$ | 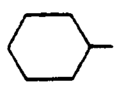 | H | H | $(CH_3)_2CH-C(CH_3)_2$- | 122 |
| 398 | $CH_3$ | $CH_3$ | 6-$CH_3$ | 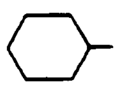 | H | H | $(CH_3)_3C-CH_2$- | |
| 399 | $CH_3$ | $CH_3$ | 6-$CH_3$ | 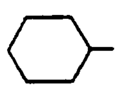 | H | H | $CH_3O-CH_2-C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 400 | $CH_3$ | $CH_3$ | 6-$CH_3$ | 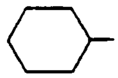 | H | H | $ClCH_2$-$C(CH_3)_2$- | |
| 401 | $CH_3$ | $CH_3$ | 6-$CH_3$ | 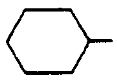 | H | H | 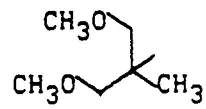 | |
| 402 | $CH_3$ | $CH_3$ | 6-$CH_3$ | 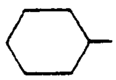 | H | H | $(CH_3)_2C$=CH- | |
| 403 | $CH_3$ | $CH_3$ | 6-$CH_3$ | 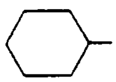 | H | H | 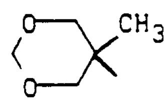 | |
| 404 | $CH_3$ | $CH_3$ | 6-$CH_3$ | 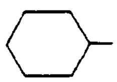 | $CH_3$- | H | $CH_3$- | 84 |
| 405 | $CH_3$ | $CH_3$ | 6-$CH_3$ | 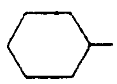 | $CH_3$- | H | $(CH_3)_2CH$- | Öl |
| 406 | $CH_3$ | $CH_3$ | 6-$CH_3$ | 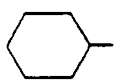 | $CH_3$- | H | $(CH_3)_3C$- | 72 |
| 407 | $CH_3$ | $CH_3$ | 6-$CH_3$ | 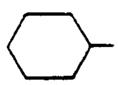 | $CH_3$- | H | $(CH_3)_2$-CH-$C(CH_3)_2$- | |
| 408 | $CH_3$ | $CH_3$ | 6-$CH_3$ | 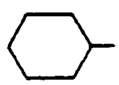 | $CH_3$- | H | $(CH_3)_3C$-$CH_2$- | 118 |
| 409 | $CH_3$ | $CH_3$ | 6-$CH_3$ | 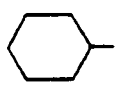 | $CH_3$- | H | $CH_3O$-$CH_2$-$C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 410 | $CH_3$ | $CH_3$ | 6-$CH_3$ | 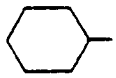 | $CH_3$- | H | $ClCH_2$-$C(CH_3)_2$- | 115 |
| 411 | $CH_3$ | $CH_3$ | 6-$CH_3$ | 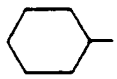 | $CH_3$- | H | 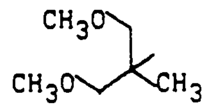 | |
| 412 | $CH_3$ | $CH_3$ | 6-$CH_3$ | 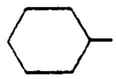 | $CH_3$- | H | $(CH_3)_2C$=CH- | |
| 413 | $CH_3$ | $CH_3$ | 6-$CH_3$ | 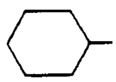 | $CH_3$- | H | 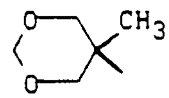 | |
| 414 | $CH_3$ | $CH_3$ | 6-$CH_3$ | 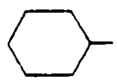 | $C_2H_5$- | H | $CH_3$- | |
| 415 | $CH_3$ | $CH_3$ | 6-$CH_3$ | 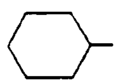 | $C_2H_5$- | H | $(CH_3)_2CH$- | |
| 416 | $CH_3$ | $CH_3$ | 6-$CH_3$ | 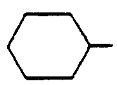 | $C_2H_5$ | H | $(CH_3)_3C$- | |
| 417 | $CH_3$ | $CH_3$ | 6-$CH_3$ | 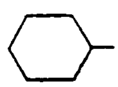 | $C_2H_5$- | H | $(CH_3)_2CH$-$C(CH_3)_2$- | |
| 418 | $CH_3$ | $CH_3$ | 6-$CH_3$ | 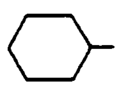 | $C_2H_5$- | H | $(CH_3)_3C$-$CH_2$- | |
| 419 | $CH_3$ | $CH_3$ | 6-$CH_3$ | 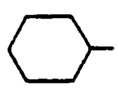 | $C_2H_5$- | H | $CH_3O$-$CH_2$-$C(CH_3)_2$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 420 | $CH_3$ | $CH_3$ | 6-$CH_3$ | 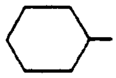 | $C_2H_5$- | H | $ClCH_2$-$C(CH_3)_2$- | |
| 421 | $CH_3$ | $CH_3$ | 6-$CH_3$ | 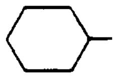 | $C_2H_5$- | H | 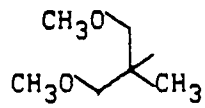 | |
| 422 | $CH_3$ | $CH_3$ | 6-$CH_3$ | 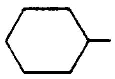 | $C_2H_5$- | H | $(CH_3)_2C$=CH- | |
| 423 | $CH_3$ | $CH_3$ | 6-$CH_3$ | 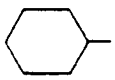 | $C_2H_5$- | H | 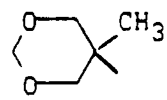 | |
| 424 | $CH_3$ | $CH_3$ | 6-$CH_3$ | 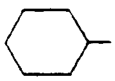 | $C_3H_7$- | H | $CH_3$- | |
| 425 | $CH_3$ | $CH_3$ | 6-$CH_3$ | 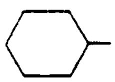 | $C_3H_7$- | H | $(CH_3)_2CH$- | |
| 426 | $CH_3$ | $CH_3$ | 6-$CH_3$ | 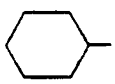 | $C_3H_7$- | H | $(CH_3)_3C$- | |
| 427 | $CH_3$ | $CH_3$ | 6-$CH_3$ | 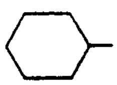 | $C_3H_7$- | H | $(CH_3)_2CH$-$C(CH_3)_2$- | |
| 428 | $CH_3$ | $CH_3$ | 6-$CH_3$ | 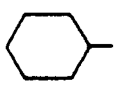 | $(CH_3)_2CH$- | H | $CH_3$- | |
| 429 | $CH_3$ | $CH_3$ | 6-$CH_3$ | 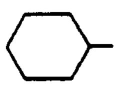 | $(CH_3)_2CH$- | H | $(CH_3)_2CH$- | |

Tabelle 2 (Fortsetzung)

| Bsp. Nr. | X | Y | $Z_n$ | A | B | C* | $R^1$ | Fp °C |
|---|---|---|---|---|---|---|---|---|
| 430 | $CH_3$ | $CH_3$ | $6-CH_3$ | 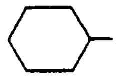 | $(CH_3)_2CH-$ | H | $(CH_3)_3C-$ | |
| 431 | $CH_3$ | $CH_3$ | $6-CH_3$ | 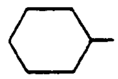 | $(CH_3)_2CH-$ | H | $(CH_3)_2CH-C(CH_3)_2-$ | |
| 432 | $CH_3$ | $CH_3$ | $6-CH_3$ | 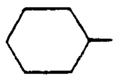 | $CH_3-$ | $CH_3-$ | $CH_3-$ | |
| 433 | $CH_3$ | $CH_3$ | $6-CH_3$ | 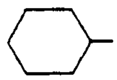 | $CH_3-$ | $CH_3-$ | $(CH_3)_2CH-$ | |
| 434 | $CH_3$ | $CH_3$ | $6-CH_3$ | 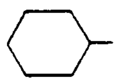 | $CH_3-$ | $CH_3-$ | $(CH_3)_3C-$ | |
| 435 | $CH_3$ | $CH_3$ | $6-CH_3$ | 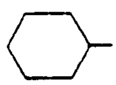 | $CH_3-$ | $CH_3-$ | $(CH_3)_2CH-C(CH_3)_2-$ | |
| 436 | $CH_3$ | $CH_3$ | $6-CH_3$ | 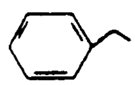 | H | H | $CH_3-$ | Öl |
| 437 | $CH_3$ | $CH_3$ | $6-CH_3$ | 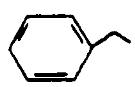 | H | H | $(CH_3)_2CH-$ | |
| 438 | $CH_3$ | $CH_3$ | $6-CH_3$ | 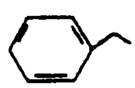 | H | H | $(CH_3)_3C-$ | 104 |
| 439 | $CH_3$ | $CH_3$ | $6-CH_3$ | 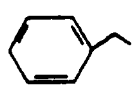 | H | H | $(CH_3)_2-CH-C(CH_3)_2-$ | |

Tabelle 2 (Fortsetzung)

| Bsp. Nr. | X | Y | $Z_n$ | A | B | C* | $R^1$ | Fp °C |
|---|---|---|---|---|---|---|---|---|
| 440 | $CH_3$ | $CH_3$ | 6-$CH_3$ | $CH_3$-O-$CH_2$-CH($CH_3$)- | H | H | $CH_3$- | 76 |
| 441 | $CH_3$ | $CH_3$ | 6-$CH_3$ | $CH_3$-O-$CH_2$-CH($CH_3$)- | H | H | $(CH_3)_2CH$- | |
| 442 | $CH_3$ | $CH_3$ | 6-$CH_3$ | $CH_3$-O-$CH_2$-CH($CH_3$)- | H | H | $(CH_3)_3C$- | Öl |
| 443 | $CH_3$ | $CH_3$ | 6-$CH_3$ | $CH_3$-O-$CH_2$-CH($CH_3$)- | H | H | $(CH_3)_2$ CH-C$(CH_3)_2$- | |
| 444 | $CH_3$ | $CH_3$ | 6-$CH_3$ | $CH_3O$-$(CH_2)_2$- | H | H | $CH_3$- | Öl |
| 445 | $CH_3$ | $CH_3$ | 6-$CH_3$ | $CH_3O$-$(CH_2)_2$- | H | H | $(CH_3)_2CH$- | Öl |
| 446 | $CH_3$ | $CH_3$ | 6-$CH_3$ | $CH_3O$-$(CH_2)_2$- | H | H | $(CH_3)_3C$- | Öl |
| 447 | $CH_3$ | $CH_3$ | 6-$CH_3$ | $CH_3O$-$(CH_2)_2$- | H | H | $(CH_3)_2$CH-C$(CH_3)_2$- | Öl |
| 448 | $CH_3$ | $CH_3$ | 6-$CH_3$ | $CH_3O$-$(CH_2)_2$- | $CH_3$- | H | $CH_3$- | Öl |
| 449 | $CH_3$ | $CH_3$ | 6-$CH_3$ | $CH_3O$-$(CH_2)_2$- | $CH_3$- | H | $(CH_3)_2CH$- | Öl |
| 450 | $CH_3$ | $CH_3$ | 6-$CH_3$ | $CH_3O$-$(CH_2)_2$- | $CH_3$- | H | $(CH_3)_3C$- | Öl |
| 451 | $CH_3$ | $CH_3$ | 6-$CH_3$ | $CH_3O$-$(CH_2)_2$- | $CH_3$- | H | $(CH_3)_2$CH-C$(CH_3)_2$- | Öl |
| 452 | $CH_3$ | $CH_3$ | 6-$CH_3$ | $CH_3O$-$(CH_2)_3$- | H | H | $CH_3$- | Öl |
| 453 | $CH_3$ | $CH_3$ | 6-$CH_3$ | $CH_3O$-$(CH_2)_3$- | H | H | $(CH_3)_2CH$- | Öl |
| 454 | $CH_3$ | $CH_3$ | 6-$CH_3$ | $CH_3O$-$(CH_2)_3$- | H | H | $(CH_3)_3C$- | Öl |
| 455 | $CH_3$ | $CH_3$ | 6-$CH_3$ | $CH_3O$-$(CH_2)_3$- | H | H | $(CH_3)_2$CH-C$(CH_3)_2$- | Öl |
| 456 | $CH_3$ | $CH_3$ | 6-$CH_3$ | $CH_3O$-$(CH_2)_3$- | $CH_3$- | H | CH3- | Öl |
| 457 | $CH_3$ | $CH_3$ | 6-$CH_3$ | $CH_3O$-$(CH_2)_3$- | $CH_3$- | H | $(CH_3)_2CH$- | Öl |
| 458 | $CH_3$ | $CH_3$ | 6-$CH_3$ | $CH_3O$-$(CH_2)_3$- | $CH_3$- | H | $(CH_3)_3C$- | Öl |
| 459 | $CH_3$ | $CH_3$ | 6-$CH_3$ | $CH_3O$-$(CH_2)_3$- | $CH_3$- | H | $(CH_3)_2$ CH-C$(CH_3)_2$- | Öl |

Tabelle 2 (Fortsetzung)

| Bsp. Nr. | X | Y | $Z_n$ | A | B | C* | $R^1$ | Fp °C |
|---|---|---|---|---|---|---|---|---|
| 460 | $CH_3$ | $CH_3$ | 6-$CH_3$ | $C_2H_5O-(CH_2)_2-CH(CH_3)-$ | H | H | $CH_3-$ | Öl |
| 461 | $CH_3$ | $CH_3$ | 6-$CH_3$ | $C_2H_5O-(CH_2)_2-CH(CH_3)-$ | H | H | $(CH_3)_2CH-$ | Öl |
| 462 | $CH_3$ | $CH_3$ | 6-$CH_3$ | $C_2H_5O-(CH_2)_2-CH(CH_3)-$ | H | H | $(CH_3)_3C-$ | Öl |
| 463 | $CH_3$ | $CH_3$ | 6-$CH_3$ | $C_2H_5O-(CH_2)_2-CH(CH_3)-$ | H | H | $(CH_3)_2CH-C(CH_3)_2-$ | Öl |
| 464 | $CH_3$ | $CH_3$ | 6-$CH_3$ | $CH_3O-CH_2-CH(CH_3)-CH-$ | H | H | $CH_3-$ | Öl |
| 465 | $CH_3$ | $CH_3$ | 6-$CH_3$ | $CH_3O-CH_2-CH(CH_3)-CH-$ | H | H | $(CH_3)_2CH-$ | Öl |
| 466 | $CH_3$ | $CH_3$ | 6-$CH_3$ | $CH_3O-CH_2-CH(CH_3)-CH-$ | H | H | $(CH_3)_3C-$ | Öl |
| 467 | $CH_3$ | $CH_3$ | 6-$CH_3$ | $CH_3O-CH_2-CH(CH_3)-CH-$ | H | H | $(CH_3)_2CH-C(CH_3)_2-$ | Öl |
| 468 | $CH_3$ | $CH_3$ | 6-$CH_3$ | $C_2H_5-S-(CH_2)_2-$ | H | H | $CH_3-$ | Öl |
| 469 | $CH_3$ | $CH_3$ | 6-$CH_3$ | $C_2H_5-S-(CH_2)_2-$ | H | H | $(CH_3)_2CH-$ | Öl |
| 470 | $CH_3$ | $CH_3$ | 6-$CH_3$ | $C_2H_5-S-(CH_2)_2-$ | H | H | $(CH_3)_3C-$ | Öl |
| 471 | $CH_3$ | $CH_3$ | 6-$CH_3$ | $C_2H_5-S-(CH_2)_2-$ | H | H | $(CH_3)_2CH-C(CH_3)_2-$ | Öl |

Tabelle 3

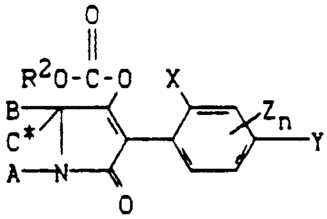 (Ic)

| Bsp. Nr. | X | Y | $Z_n$ | A | B | C* | $R^1$ | Fp °C |
|---|---|---|---|---|---|---|---|---|
| 472 | $CH_3$ | $CH_3$ | 6-$CH_3$ | $CH_3$- | H | H | $CH_3$- | |
| 473 | $CH_3$ | $CH_3$ | 6-$CH_3$ | $CH_3$- | H | H | $C_2H_5$- | |
| 474 | $CH_3$ | $CH_3$ | 6-$CH_3$ | $CH_3$- | H | H | $(CH_3)_2CH$- | |
| 475 | $CH_3$ | $CH_3$ | 6-$CH_3$ | $CH_3$- | H | H | $(CH_3)_2CH$-$CH_2$- | |
| 476 | $CH_3$ | $CH_3$ | 6-$CH_3$ | $CH_3$- | H | H | 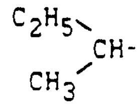 | |
| 477 | $CH_3$ | $CH_3$ | 6-$CH_3$ | $CH_3$- | H | H | $(CH_3)_3C$-$CH_2$- | |
| 478 | $CH_3$ | $CH_3$ | 6-$CH_3$ | $CH_3$- | H | H | 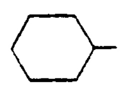 | |
| 479 | $CH_3$ | $CH_3$ | 6-$CH_3$ | $CH_3$- | H | H | 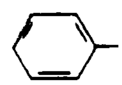 | |
| 480 | $CH_3$ | $CH_3$ | 6-$CH_3$ | $CH_3$- | $CH_3$- | H | $CH_3$- | |
| 481 | $CH_3$ | $CH_3$ | 6-$CH_3$ | $CH_3$- | $CH_3$- | H | $C_2H_5$- | Öl |
| 482 | $CH_3$ | $CH_3$ | 6-$CH_3$ | $CH_3$- | $CH_3$- | H | $(CH_3)_2CH$- | Öl |
| 483 | $CH_3$ | $CH_3$ | 6-$CH_3$ | $CH_3$- | $CH_3$- | H | $(CH_3)_2$ CH-$CH_2$- | Öl |

Tabelle 3 (Fortsetzung)

| Bsp. Nr. | X | Y | $Z_n$ | A | B | C* | $R^1$ | Fp °C |
|---|---|---|---|---|---|---|---|---|
| 484 | $CH_3$ | $CH_3$ | 6-$CH_3$ | $CH_3$- | $CH_3$- | H | $C_2H_5$<br>CH-<br>$CH_3$ | Öl |
| 485 | $CH_3$ | $CH_3$ | 6-$CH_3$ | $CH_3$- | $CH_3$- | H | $(CH_3)_3C-CH_2$- | 146 |
| 486 | $CH_3$ | $CH_3$ | 6-$CH_3$ | $CH_3$- | $CH_3$- | H | 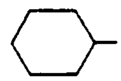 | |
| 487 | $CH_3$ | $CH_3$ | 6-$CH_3$ | $CH_3$- | $CH_3$- | H | 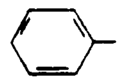 | |
| 488 | $CH_3$ | $CH_3$ | 6-$CH_3$ | $CH_3$- | $C_2H_5$- | H | $CH_3$- | |
| 489 | $CH_3$ | $CH_3$ | 6-$CH_3$ | $CH_3$- | $C_2H_5$- | H | $C_2H_5$- | |
| 490 | $CH_3$ | $CH_3$ | 6-$CH_3$ | $CH_3$- | $C_2H_5$- | H | $(CH_3)_2CH$- | |
| 491 | $CH_3$ | $CH_3$ | 6-$CH_3$ | $CH_3$- | $C_2H_5$- | H | $(CH_3)_2CH-CH_2$- | |
| 492 | $CH_3$ | $CH_3$ | 6-$CH_3$ | $CH_3$- | $C_2H_5$- | H | $C_2H_5$<br>CH-<br>$CH_3$ | |
| 493 | $CH_3$ | $CH_3$ | 6-$CH_3$ | $CH_3$- | $C_2H_5$- | H | $(CH_3)_3C-CH_2$- | |
| 494 | $CH_3$ | $CH_3$ | 6-$CH_3$ | $CH_3$- | $C_2H_5$- | H | 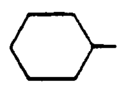 | |
| 495 | $CH_3$ | $CH_3$ | 6-$CH_3$ | $CH_3$- | $C_2H_5$- | H | 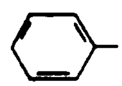 | |
| 496 | $CH_3$ | $CH_3$ | 6-$CH_3$ | $CH_3$- | $C_3H_7$- | H | $C_2H_5$- | |
| 497 | $CH_3$ | $CH_3$ | 6-$CH_3$ | $CH_3$- | $C_3H_7$- | H | $(CH_3)_2CH$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 498 | $CH_3$ | $CH_3$ | 6-$CH_3$ | $CH_3$- | $C_3H_7$- | H | $(CH_3)_2CH-CH_2$- | |
| 499 | $CH_3$ | $CH_3$ | 6-$CH_3$ | $CH_3$- | $C_3H_7$- | H | $CH_3$\ <br> CH- <br> $C_2H_5$/ | |
| 500 | $CH_3$ | $CH_3$ | 6-$CH_3$ | $CH_3$- | $(CH_3)_2CH$- | H | $(CH_3)_3C-CH_2$- | |
| 501 | $CH_3$ | $CH_3$ | 6-$CH_3$ | $CH_3$- | $(CH_3)_2CH$- | H | $C_2H_5$- | |
| 502 | $CH_3$ | $CH_3$ | 6-$CH_3$ | $CH_3$- | $(CH_3)_2CH$- | H | $(CH_3)_2CH$- | |
| 503 | $CH_3$ | $CH_3$ | 6-$CH_3$ | $CH_3$- | $(CH_3)_2CH$- | H | $(CH_3)_2CH-CH_2$- | |
| 504 | $CH_3$ | $CH_3$ | 6-$CH_3$ | $CH_3$- | $(CH_3)_2CH$- | H | $CH_3$\ <br> CH- <br> $C_2H_5$/ | |
| 505 | $CH_3$ | $CH_3$ | 6-$CH_3$ | $CH_3$- | $(CH_3)_2CH$- | H | $(CH_3)_3C-CH_2$- | |
| 506 | $CH_3$ | $CH_3$ | 6-$CH_3$ | $C_2H_5$- | H | H | $CH_3$- | |
| 507 | $CH_3$ | $CH_3$ | 6-$CH_3$ | $C_2H_5$- | H | H | $C_2H_5$- | |
| 508 | $CH_3$ | $CH_3$ | 6-$CH_3$ | $C_2H_5$- | H | H | $(CH_3)_2CH$- | Öl |
| 509 | $CH_3$ | $CH_3$ | 6-$CH_3$ | $C_2H_5$- | H | H | $(CH_3)_2CH-CH_2$- | |
| 510 | $CH_3$ | $CH_3$ | 6-$CH_3$ | $C_2H_5$- | H | H | $C_2H_5$\ <br> CH- <br> $CH_3$/ | |
| 511 | $CH_3$ | $CH_3$ | 6-$CH_3$ | $C_2H_5$- | H | H | $(CH_3)_3C-CH_2$- | |
| 512 | $CH_3$ | $CH_3$ | 6-$CH_3$ | $C_2H_5$- | H | H | 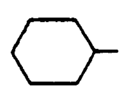 | |
| 513 | $CH_3$ | $CH_3$ | 6-$CH_3$ | $C_2H_5$- | H | H | 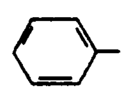 | |

Tabelle 3 (Fortsetzung)

| Bsp. Nr. | X | Y | $Z_n$ | A | B | C* | $R^1$ | Fp °C |
|---|---|---|---|---|---|---|---|---|
| 514 | $CH_3$ | $CH_3$ | 6-$CH_3$ | $C_2H_5$- | $CH_3$- | H | $CH_3$- | |
| 515 | $CH_3$ | $CH_3$ | 6-$CH_3$ | $C_2H_5$- | $CH_3$- | H | $C_2H_5$- | Öl |
| 516 | $CH_3$ | $CH_3$ | 6-$CH_3$ | $C_2H_5$- | $CH_3$- | H | $(CH_3)_2CH$- | |
| 517 | $CH_3$ | $CH_3$ | 6-$CH_3$ | $C_2H_5$- | $CH_3$- | H | $(CH_3)_2CH$-$CH_2$- | |
| 518 | $CH_3$ | $CH_3$ | 6-$CH_3$ | $C_2H_5$- | $CH_3$- | H | $C_2H_5$(CH$_3$)CH- | |
| 519 | $CH_3$ | $CH_3$ | 6-$CH_3$ | $C_2H_5$- | $CH_3$- | H | $(CH_3)_3C$-$CH_2$- | |
| 520 | $CH_3$ | $CH_3$ | 6-$CH_3$ | $C_2H_5$- | $CH_3$- | H | 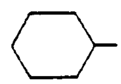 | |
| 521 | $CH_3$ | $CH_3$ | 6-$CH_3$ | $C_2H_5$- | $CH_3$- | H | 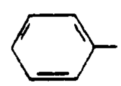 | |
| 522 | $CH_3$ | $CH_3$ | 6-$CH_3$ | $C_2H_5$- | $C_2H_5$- | H | $CH_3$- | |
| 523 | $CH_3$ | $CH_3$ | 6-$CH_3$ | $C_2H_5$- | $C_2H_5$- | H | $C_2H_5$- | |
| 524 | $CH_3$ | $CH_3$ | 6-$CH_3$ | $C_2H_5$- | $C_2H_5$- | H | $(CH_3)_2CH$- | |
| 525 | $CH_3$ | $CH_3$ | 6-$CH_3$ | $C_2H_5$- | $C_2H_5$- | H | $(CH_3)_2CH$-$CH_2$- | |
| 526 | $CH_3$ | $CH_3$ | 6-$CH_3$ | $C_2H_5$- | $C_2H_5$- | H | $C_2H_5$(CH$_3$)CH- | |
| 527 | $CH_3$ | $CH_3$ | 6-$CH_3$ | $C_2H_5$- | $C_2H_5$- | H | $(CH_3)_3C$-$CH_2$- | |
| 528 | $CH_3$ | $CH_3$ | 6-$CH_3$ | $C_2H_5$- | $C_2H_5$- | H | 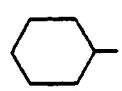 | |

Tabelle 3 (Fortsetzung)

| Bsp. Nr. | X | Y | $Z_n$ | A | B | C* | $R^1$ | Fp °C |
|---|---|---|---|---|---|---|---|---|
| 529 | $CH_3$ | $CH_3$ | $6-CH_3$ | $C_2H_5-$ | $C_2H_5-$ | H | 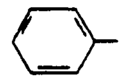 | |
| 530 | $CH_3$ | $CH_3$ | $6-CH_3$ | $C_2H_5-$ | $C_3H_7-$ | H | $C_2H_5-$ | |
| 531 | $CH_3$ | $CH_3$ | $6-CH_3$ | $C_2H_5-$ | $C_3H_7-$ | H | $(CH_3)_2CH-$ | |
| 532 | $CH_3$ | $CH_3$ | $6-CH_3$ | $C_2H_5-$ | $C_3H_7-$ | H | $(CH_3)_2CH-CH_2-$ | |
| 533 | $CH_3$ | $CH_3$ | $6-CH_3$ | $C_2H_5-$ | $C_3H_7-$ | H | $CH_3$<br>$CH-$<br>$C_2H_5$ | |
| 534 | $CH_3$ | $CH_3$ | $6-CH_3$ | $C_2H_5-$ | $(CH_3)_2CH-$ | H | $(CH_3)_3C-CH_2-$ | |
| 535 | $CH_3$ | $CH_3$ | $6-CH_3$ | $C_2H_5-$ | $(CH_3)_2CH-$ | H | $C_2H_5-$ | |
| 536 | $CH_3$ | $CH_3$ | $6-CH_3$ | $C_2H_5-$ | $(CH_3)_2CH-$ | H | $(CH_3)_2CH-$ | |
| 537 | $CH_3$ | $CH_3$ | $6-CH_3$ | $C_2H_5-$ | $(CH_3)_2CH-$ | H | $(CH_3)_2CH-CH_2-$ | |
| 538 | $CH_3$ | $CH_3$ | $6-CH_3$ | $C_2H_5-$ | $(CH_3)_2CH-$ | H | $CH_3$<br>$CH-$<br>$C_2H_5$ | |
| 539 | $CH_3$ | $CH_3$ | $6-CH_3$ | $C_2H_5-$ | $(CH_3)_2CH-$ | H | $(CH_3)_3C-CH_2-$ | |
| 540 | $CH_3$ | $CH_3$ | $6-CH_3$ | $C_3H_7-$ | H | H | $CH_3-$ | |
| 541 | $CH_3$ | $CH_3$ | $6-CH_3$ | $C_3H_7-$ | H | H | $C_2H_5-$ | |
| 542 | $CH_3$ | $CH_3$ | $6-CH_3$ | $C_3H_7-$ | H | H | $(CH_3)_2CH-$ | |
| 543 | $CH_3$ | $CH_3$ | $6-CH_3$ | $C_3H_7-$ | H | H | $(CH_3)_2\ CH-CH_2-$ | |
| 544 | $CH_3$ | $CH_3$ | $6-CH_3$ | $C_3H_7-$ | H | H | $C_2H_5$<br>$CH-$<br>$CH_3$ | |

Tabelle 3 (Fortsetzung)

| Bsp. Nr. | X | Y | $Z_n$ | A | B | C* | $R^1$ | Fp °C |
|---|---|---|---|---|---|---|---|---|
| 545 | $CH_3$ | $CH_3$ | 6-$CH_3$ | $C_3H_7$- | H | H | $(CH_3)_3C-CH_2$- | |
| 546 | $CH_3$ | $CH_3$ | 6-$CH_3$ | $C_3H_7$- | H | H | 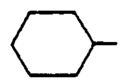 | |
| 547 | $CH_3$ | $CH_3$ | 6-$CH_3$ | $C_3H_7$- | H | H | 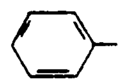 | |
| 548 | $CH_3$ | $CH_3$ | 6-$CH_3$ | $C_3H_7$- | $CH_3$- | H | $CH_3$- | |
| 549 | $CH_3$ | $CH_3$ | 6-$CH_3$ | $C_3H_7$- | $CH_3$- | H | $C_2H_5$- | |
| 550 | $CH_3$ | $CH_3$ | 6-$CH_3$ | $C_3H_7$- | $CH_3$- | H | $(CH_3)_2CH$- | |
| 551 | $CH_3$ | $CH_3$ | 6-$CH_3$ | $C_3H_7$- | $CH_3$- | H | $(CH_3)_2CH-CH_2$- | |
| 552 | $CH_3$ | $CH_3$ | 6-$CH_3$ | $C_3H_7$- | $CH_3$- | H | 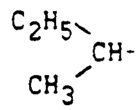 | |
| 553 | $CH_3$ | $CH_3$ | 6-$CH_3$ | $C_3H_7$- | $CH_3$- | H | $(CH_3)_3C-CH_2$- | |
| 554 | $CH_3$ | $CH_3$ | 6-$CH_3$ | $C_2H_5$- | $CH_3$- | H | 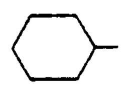 | |
| 555 | $CH_3$ | $CH_3$ | 6-$CH_3$ | $C_2H_5$- | $CH_3$- | H | 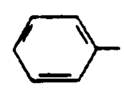 | |
| 556 | $CH_3$ | $CH_3$ | 6-$CH_3$ | $C_3H_7$- | $C_2H_5$ | H | $CH_3$- | |
| 557 | $CH_3$ | $CH_3$ | 6-$CH_3$ | $C_3H_7$- | $C_2H_5$- | H | $C_2H_5$- | |
| 558 | $CH_3$ | $CH_3$ | 6-$CH_3$ | $C_3H_7$- | $C_2H_5$- | H | $(CH_3)_2CH$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 559 | $CH_3$ | $CH_3$ | 6-$CH_3$ | $C_3H_7$- | $C_2H_5$- | H | $(CH_3)_2CH-CH_2$- | |
| 560 | $CH_3$ | $CH_3$ | 6-$CH_3$ | $C_3H_7$- | $C_2H_5$- | H | 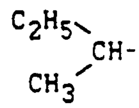 | |
| 561 | $CH_3$ | $CH_3$ | 6-$CH_3$ | $C_3H_7$- | $C_2H_5$- | H | $(CH_3)_3C-CH_2$- | |
| 562 | $CH_3$ | $CH_3$ | 6-$CH_3$ | $C_3H_7$- | $C_2H_5$- | H | 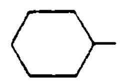 | |
| 563 | $CH_3$ | $CH_3$ | 6-$CH_3$ | $C_3H_7$- | $C_2H_5$- | H | 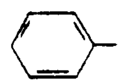 | |
| 564 | $CH_3$ | $CH_3$ | 6-$CH_3$ | $C_3H_7$- | $C_3H_7$- | H | $C_2H_5$- | |
| 565 | $CH_3$ | $CH_3$ | 6-$CH_3$ | $C_3H_7$- | $C_3H_7$- | H | $(CH_3)_2CH$- | |
| 566 | $CH_3$ | $CH_3$ | 6-$CH_3$ | $C_3H_7$- | $C_3H_7$- | H | $(CH_3)_2CH-CH_2$- | |
| 567 | $CH_3$ | $CH_3$ | 6-$CH_3$ | $C_3H_7$- | $C_3H_7$- | H | 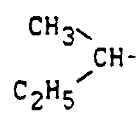 | |
| 568 | $CH_3$ | $CH_3$ | 6-$CH_3$ | $C_3H_7$- | $C_3H_7$- | H | $(CH_3)_3C-CH_2$- | |
| 569 | $CH_3$ | $CH_3$ | 6-$CH_3$ | $C_3H_7$- | $(CH_3)_2CH$- | H | $C_2H_5$- | |
| 570 | $CH_3$ | $CH_3$ | 6-$CH_3$ | $C_3H_7$- | $(CH_3)_2CH$- | H | $(CH_3)_2CH$- | |
| 571 | $CH_3$ | $CH_3$ | 6-$CH_3$ | $C_3H_7$- | $(CH_3)_2CH$- | H | $(CH_3)_2CH-CH_2$- | |
| 572 | $CH_3$ | $CH_3$ | 6-$CH_3$ | $C_3H_7$- | $(CH_3)_2CH$- | H | 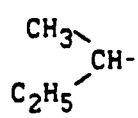 | |
| 573 | $CH_3$ | $CH_3$ | 6-$CH_3$ | $C_3H_7$- | $(CH_3)_2CH$- | H | $(CH_3)_3C-CH_2$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 574 | $CH_3$ | $CH_3$ | 6-$CH_3$ | $(CH_3)_2CH$- | H | H | $CH_3$- | 67 |
| 575 | $CH_3$ | $CH_3$ | 6-$CH_3$ | $(CH_3)_2CH$- | H | H | $C_2H_5$- | 87 |
| 576 | $CH_3$ | $CH_3$ | 6-$CH_3$ | $(CH_3)_2CH$- | H | H | $(CH_3)_2$ CH-$CH_2$ | 41 |
| 577 | $CH_3$ | $CH_3$ | 6-$CH_3$ | $(CH_3)_2CH$- | H | H | $C_2H_5$<br>CH-<br>$CH_3$ | 83 |
| 578 | $CH_3$ | $CH_3$ | 6-$CH_3$ | $(CH_3)_2CH$- | H | H | $(CH_3)_3C$-$CH_2$- | Öl |
| 579 | $CH_3$ | $CH_3$ | 6-$CH_3$ | $(CH_3)_2CH$- | H | H | 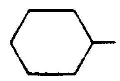 | |
| 580 | $CH_3$ | $CH_3$ | 6-$CH_3$ | $(CH_3)_2CH$- | $CH_3$- | H | $CH_3$- | |
| 581 | $CH_3$ | $CH_3$ | 6-$CH_3$ | $(CH_3)_2CH$- | $CH_3$- | H | $C_2H_5$- | |
| 582 | $CH_3$ | $CH_3$ | 6-$CH_3$ | $(CH_3)_2CH$- | $CH_3$- | H | $(CH_3)_2CH$- | |
| 583 | $CH_3$ | $CH_3$ | 6-$CH_3$ | $(CH_3)_2CH$- | $CH_3$- | H | $(CH_3)_2CH$-$CH_2$- | |
| 584 | $CH_3$ | $CH_3$ | 6-$CH_3$ | $(CH_3)_2CH$- | $CH_3$- | H | $C_2H_5$<br>CH-<br>$CH_3$ | |
| 585 | $CH_3$ | $CH_3$ | 6-$CH_3$ | $(CH_3)_2CH$- | $CH_3$- | H | $(CH_3)_3C$-$CH_2$- | 83 |
| 586 | $CH_3$ | $CH_3$ | 6-$CH_3$ | $(CH_3)_2CH$- | $CH_3$- | H | 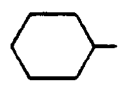 | |
| 587 | $CH_3$ | $CH_3$ | 6-$CH_3$ | $(CH_3)_2CH$- | $CH_3$- | H | 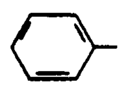 | |

Tabelle 3 (Fortsetzung)

| Bsp. Nr. | X | Y | $Z_n$ | A | B | C* | $R^1$ | Fp °C |
|---|---|---|---|---|---|---|---|---|
| 588 | $CH_3$ | $CH_3$ | 6-$CH_3$ | $(CH_3)_2CH-$ | $C_2H_5-$ | H | $CH_3-$ | |
| 589 | $CH_3$ | $CH_3$ | 6-$CH_3$ | $(CH_3)_2CH-$ | $C_2H_5-$ | H | $C_2H_5-$ | |
| 590 | $CH_3$ | $CH_3$ | 6-$CH_3$ | $(CH_3)_2CH-$ | $C_2H_5-$ | H | $(CH_3)_2CH-$ | |
| 591 | $CH_3$ | $CH_3$ | 6-$CH_3$ | $(CH_3)_2CH-$ | $C_2H_5-$ | H | $(CH_3)_2CH-CH_2-$ | |
| 592 | $CH_3$ | $CH_3$ | 6-$CH_3$ | $(CH_3)_2CH-$ | $C_2H_5-$ | H | $C_2H_5$<br>CH-<br>$CH_3$ | |
| 593 | $CH_3$ | $CH_3$ | 6-$CH_3$ | $(CH_3)_2CH-$ | $C_2H_5-$ | H | $(CH_3)_3C-CH_2-$ | |
| 594 | $CH_3$ | $CH_3$ | 6-$CH_3$ | $(CH_3)_2CH-$ | $C_2H_5-$ | H | 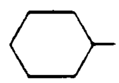 | |
| 595 | $CH_3$ | $CH_3$ | 6-$CH_3$ | $(CH_3)_2CH-$ | $C_2H_5-$ | H | 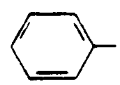 | |
| 596 | $CH_3$ | $CH_3$ | 6-$CH_3$ | $(CH_3)_2CH-$ | $C_3H_7-$ | H | $C_2H_5-$ | |
| 597 | $CH_3$ | $CH_3$ | 6-$CH_3$ | $(CH_3)_2CH-$ | $C_3H_7-$ | H | $(CH_3)_2CH-$ | |
| 598 | $CH_3$ | $CH_3$ | 6-$CH_3$ | $(CH_3)_2CH-$ | $C_3H_7-$ | H | $(CH_3)_2CH-CH_2-$ | |
| 599 | $CH_3$ | $CH_3$ | 6-$CH_3$ | $(CH_3)_2CH-$ | $C_3H_7-$ | H | $CH_3$<br>CH-<br>$C_2H_5$ | |
| 600 | $CH_3$ | $CH_3$ | 6-$CH_3$ | $(CH_3)_2CH-$ | $C_3H_7-$ | H | $(CH_3)_3C-CH_2-$ | |
| 601 | $CH_3$ | $CH_3$ | 6-$CH_3$ | $(CH_3)_2CH-$ | $(CH_3)_2CH-$ | H | $C_2H_5-$ | |
| 602 | $CH_3$ | $CH_3$ | 6-$CH_3$ | $(CH_3)_2CH-$ | $(CH_3)_2CH-$ | H | $(CH_3)_2CH-$ | |
| 603 | $CH_3$ | $CH_3$ | 6-$CH_3$ | $(CH_3)_2CH-$ | $(CH_3)_2CH-$ | H | $(CH_3)_2CH-CH_2-$ | |

Tabelle 3 (Fortsetzung)

| Bsp. Nr. | X | Y | $Z_n$ | A | B | C* | $R^1$ | Fp °C |
|---|---|---|---|---|---|---|---|---|
| 604 | $CH_3$ | $CH_3$ | 6-$CH_3$ | $(CH_3)_2CH$- | $(CH_3)_2CH$- | H | $CH_3$<br>CH-<br>$C_2H_5$ | |
| 605 | $CH_3$ | $CH_3$ | 6-$CH_3$ | $(CH_3)_2CH$- | $(CH_3)_2CH$- | H | $(CH_3)_3C-CH_2$- | |
| 606 | $CH_3$ | $CH_3$ | 6-$CH_3$ | $C_4H_9$- | H | H | $C2H_5$- | |
| 607 | $CH_3$ | $CH_3$ | 6-$CH_3$ | $C_4H_9$- | H | H | $(CH_3)_2CH$- | |
| 608 | $CH_3$ | $CH_3$ | 6-$CH_3$ | $C_4H_9$- | H | H | $(CH_3)_2CH-CH_2$- | |
| 609 | $CH_3$ | $CH_3$ | 6-$CH_3$ | $C_4H_9$- | H | H | $CH_3$<br>CH-<br>$C_2H_5$ | |
| 610 | $CH_3$ | $CH_3$ | 6-$CH_3$ | $C_4H_9$- | H | H | $(CH_3)_3C-CH_2$- | |
| 611 | $CH_3$ | $CH_3$ | 6-$CH_3$ | $C_4H_9$- | $CH_3$- | H | $C_2H_5$- | |
| 612 | $CH_3$ | $CH_3$ | 6-$CH_3$ | $C_4H_9$- | $CH_3$- | H | $(CH_3)_2CH$- | |
| 613 | $CH_3$ | $CH_3$ | 6-$CH_3$ | $C_4H_9$- | $CH_3$- | H | $(CH_3)_2CH-CH_2$- | |
| 614 | $CH_3$ | $CH_3$ | 6-$CH_3$ | $C_4H_9$- | $CH_3$- | H | $CH_3$<br>CH-<br>$C_2H_5$ | |
| 615 | $CH_3$ | $CH_3$ | 6-$CH_3$ | $C_4H_9$- | $CH_3$- | H | $(CH_3)_3C-CH_2$- | |
| 616 | $CH_3$ | $CH_3$ | 6-$CH_3$ | $(CH_3)_2CH-CH_2$- | H | H | $C_2H_5$- | |
| 617 | $CH_3$ | $CH_3$ | 6-$CH_3$ | $(CH_3)_2CH-CH_2$- | H | H | $(CH_3)_2CH$- | |
| 618 | $CH_3$ | $CH_3$ | 6-$CH_3$ | $(CH_3)_2CH-CH_2$- | H | H | $(CH_3)_2CH-CH_2$- | |
| 619 | $CH_3$ | $CH_3$ | 6-$CH_3$ | $(CH_3)_2CH-CH_2$- | H | H | $CH_3$<br>CH-<br>$C_2H_5$ | |
| 620 | $CH_3$ | $CH_3$ | 6-$CH_3$ | $(CH_3)_2CH-CH_2$- | H | H | $(CH_3)_3C-CH_2$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 621 | $CH_3$ | $CH_3$ | 6-$CH_3$ | $(CH_3)_2CH-CH_2-$ | $CH_3-$ | H | $C_2H_5-$ | |
| 622 | $CH_3$ | $CH_3$ | 6-$CH_3$ | $(CH_3)_2CH-CH_2-$ | $CH_3-$ | H | $(CH_3)_2CH-$ | |
| 623 | $CH_3$ | $CH_3$ | 6-$CH_3$ | $(CH_3)_2CH-CH_2-$ | $CH_3-$ | H | $(CH_3)_2CH-CH_2-$ | |
| 624 | $CH_3$ | $CH_3$ | 6-$CH_3$ | $(CH_3)_2CH-CH_2-$ | $CH_3-$ | H | 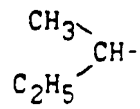 | |
| 625 | $CH_3$ | $CH_3$ | 6-$CH_3$ | $(CH_3)_2CH-CH_2-$ | $CH_3-$ | H | $(CH_3)_3C-CH_2-$ | |
| 626 | $CH_3$ | $CH_3$ | 6-$CH_3$ | 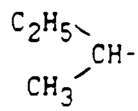 | H | H | $C_2H_5-$ | |
| 627 | $CH_3$ | $CH_3$ | 6-$CH_3$ | 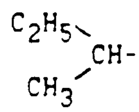 | H | H | $(CH_3)_2CH-$ | |
| 628 | $CH_3$ | $CH_3$ | 6-$CH_3$ | 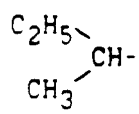 | H | H | $(CH_3)_2CH-CH_2-$ | |
| 629 | $CH_3$ | $CH_3$ | 6-$CH_3$ | 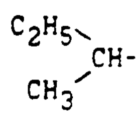 | H | H | 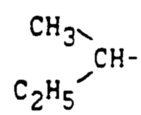 | |
| 630 | $CH_3$ | $CH_3$ | 6-$CH_3$ | 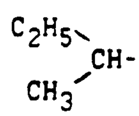 | H | H | $(CH_3)_3C-CH_2-$ | |
| 631 | $CH_3$ | $CH_3$ | 6-$CH_3$ | 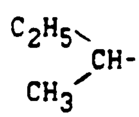 | $CH_3-$ | H | $C_2H_5-$ | |
| 632 | $CH_3$ | $CH_3$ | 6-$CH_3$ | 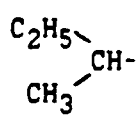 | $CH_3-$ | H | $(CH_3)_2CH-$ | |

Tabelle 3 (Fortsetzung)

| Bsp. Nr. | X | Y | $Z_n$ | A | B | C* | $R^1$ | Fp °C |
|---|---|---|---|---|---|---|---|---|
| 633 | $CH_3$ | $CH_3$ | 6-$CH_3$ | $C_2H_5$ \ CH- / $CH_3$ | $CH_3$- | H | $(CH_3)_2CH-CH_2$- | |
| 634 | $CH_3$ | $CH_3$ | 6-$CH_3$ | $C_2H_5$ \ CH- / $CH_3$ | $CH_3$- | H | $CH_3$ \ CH- / $C_2H_5$ | |
| 635 | $CH_3$ | $CH_3$ | 6-$CH_3$ | $C_2H_5$ \ CH- / $CH_3$ | $CH_3$- | H | $(CH_3)_3C-CH_2$- | |
| 636 | $CH_3$ | $CH_3$ | 6-$CH_3$ | $CH_2=CH-CH_2$- | H | H | $C_2H_5$- | |
| 637 | $CH_3$ | $CH_3$ | 6-$CH_3$ | $CH_2=CH-CH_2$- | H | H | $(CH_3)_2CH$- | Öl |
| 638 | $CH_3$ | $CH_3$ | 6-$CH_3$ | $CH_2=CH-CH_2$- | H | H | $(CH_3)_2CH-CH_2$- | |
| 639 | $CH_3$ | $CH_3$ | 6-$CH_3$ | $CH_2=CH-CH_2$- | H | H | $CH_3$ \ CH- / $C_2H_5$ | |
| 640 | $CH_3$ | $CH_3$ | 6-$CH_3$ | $CH_2=CH-CH_2$- | H | H | $(CH_3)_3C-CH_2$- | |
| 641 | $CH_3$ | $CH_3$ | 6-$CH_3$ | 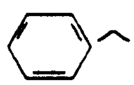 | H | H | $C_2H_5$- | |
| 642 | $CH_3$ | $CH_3$ | 6-$CH_3$ | 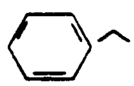 | H | H | $(CH_3)_2CH$- | Öl |
| 643 | $CH_3$ | $CH_3$ | 6-$CH_3$ | 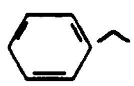 | H | H | $(CH_3)_2CH-CH_2$- | |
| 644 | $CH_3$ | $CH_3$ | 6-$CH_3$ | 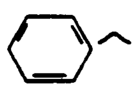 | H | H | $CH_3$ \ CH- / $C_2H_5$ | |
| 645 | $CH_3$ | $CH_3$ | 6-$CH_3$ | 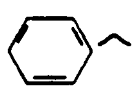 | H | H | $(CH_3)_3C-CH_2$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 646 | $CH_3$ | $CH_3$ | $6\text{-}CH_3$ | 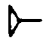 | H | H | $CH_3$- | |
| 647 | $CH_3$ | $CH_3$ | $6\text{-}CH_3$ | 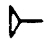 | H | H | $C_2H_5$- | |
| 648 | $CH_3$ | $CH_3$ | $6\text{-}CH_3$ | 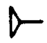 | H | H | $(CH_3)_2CH$- | |
| 649 | $CH_3$ | $CH_3$ | $6\text{-}CH_3$ | 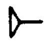 | H | H | $(CH_3)_2CH\text{-}CH_2$- | |
| 650 | $CH_3$ | $CH_3$ | $6\text{-}CH_3$ | 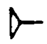 | H | H | $C_2H_5$\ CH- / $CH_3$ | Öl |
| 651 | $CH_3$ | $CH_3$ | $6\text{-}CH_3$ | 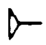 | H | H | $(CH_3)_3C\text{-}CH_2$- | |
| 652 | $CH_3$ | $CH_3$ | $6\text{-}CH_3$ | 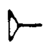 | H | H | 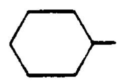 | |
| 653 | $CH_3$ | $CH_3$ | $6\text{-}CH_3$ | 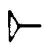 | H | H | 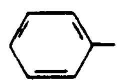 | |
| 654 | $CH_3$ | $CH_3$ | $6\text{-}CH_3$ | 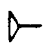 | $CH_3$- | H | $CH_3$- | |
| 655 | $CH_3$ | $CH_3$ | $6\text{-}CH_3$ | 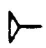 | $CH_3$- | H | $C_2H_5$- | |
| 656 | $CH_3$ | $CH_3$ | $6\text{-}CH_3$ | 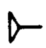 | $CH_3$- | H | $(CH_3)_2CH$- | |
| 657 | $CH_3$ | $CH_3$ | $6\text{-}CH_3$ | 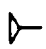 | $CH_3$- | H | $(CH_3)_2CH\text{-}CH_2$- | |
| 658 | $CH_3$ | $CH_3$ | $6\text{-}CH_3$ | 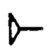 | $CH_3$- | H | $C_2H_5$\ CH- / $CH_3$ | |
| 659 | $CH_3$ | $CH_3$ | $6\text{-}CH_3$ | 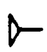 | $CH_3$- | H | $(CH_3)_3C\text{-}CH_2$- | |
| 660 | $CH_3$ | $CH_3$ | $6\text{-}CH_3$ | 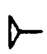 | $CH_3$- | H | 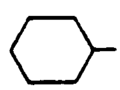 | |
| 661 | $CH_3$ | $CH_3$ | $6\text{-}CH_3$ | 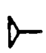 | $CH_3$- | H | 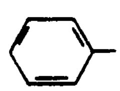 | |

Tabelle 3 (Fortsetzung)

| Bsp. Nr. | X | Y | $Z_n$ | A | B | C* | $R^1$ | Fp °C |
|---|---|---|---|---|---|---|---|---|
| 662 | $CH_3$ | $CH_3$ | 6-$CH_3$ | 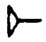 | $C_2H_5$- | H | $CH_3$- | |
| 663 | $CH_3$ | $CH_3$ | 6-$CH_3$ | 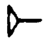 | $C_2H_5$- | H | $C_2H_5$- | |
| 664 | $CH_3$ | $CH_3$ | 6-$CH_3$ | 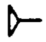 | $C_2H_5$- | H | $(CH_3)_2CH$- | |
| 665 | $CH_3$ | $CH_3$ | 6-$CH_3$ | 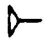 | $C_2H_5$- | H | $(CH_3)_2CH$-$CH_2$- | |
| 666 | $CH_3$ | $CH_3$ | 6-$CH_3$ | 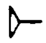 | $C_2H_5$- | H | 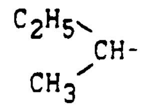 | Öl |
| 667 | $CH_3$ | $CH_3$ | 6-$CH_3$ | 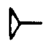 | $C_2H_5$- | H | $(CH_3)_3C$-$CH_2$- | |
| 668 | $CH_3$ | $CH_3$ | 6-$CH_3$ | 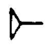 | $C_2H_5$- | H | 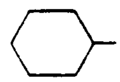 | |
| 669 | $CH_3$ | $CH_3$ | 6-$CH_3$ | 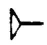 | $C_2H_5$- | H | 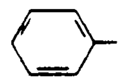 | |
| 670 | $CH_3$ | $CH_3$ | 6-$CH_3$ | 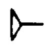 | $C_3H_7$- | H | $C_2H_5$- | |
| 671 | $CH_3$ | $CH_3$ | 6-$CH_3$ | 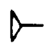 | $C_3H_7$- | H | $(CH_3)_2CH$- | |
| 672 | $CH_3$ | $CH_3$ | 6-$CH_3$ | 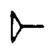 | $C_3H_7$- | H | $(CH_3)_2CH$-$CH_2$- | |
| 673 | $CH_3$ | $CH_3$ | 6-$CH_3$ | 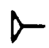 | $C_3H_7$- | H | 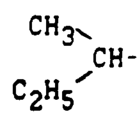 | |
| 674 | $CH_3$ | $CH_3$ | 6-$CH_3$ | 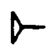 | $C_3H_7$- | H | $(CH_3)_3C$-$CH_2$- | |
| 675 | $CH_3$ | $CH_3$ | 6-$CH_3$ | 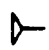 | $(CH_3)_2CH$- | H | $C_2H5$- | |
| 676 | $CH_3$ | $CH_3$ | 6-$CH_3$ | 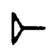 | $(CH_3)_2CH$- | H | $(CH_3)_2CH$- | |
| 677 | $CH_3$ | $CH_3$ | 6-$CH_3$ | 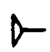 | $(CH_3)_2CH$- | H | $(CH_3)_2CH$-$CH_2$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 678 | $CH_3$ | $CH_3$ | 6-$CH_3$ | 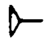 | $(CH_3)_2CH$- | H | $CH_3$<br>CH-<br>$C_2H_5$ | |
| 679 | $CH_3$ | $CH_3$ | 6-$CH_3$ | 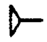 | $(CH_3)_2CH$- | H | $(CH_3)_3C$-$CH_2$- | |
| 680 | $CH_3$ | $CH_3$ | 6-$CH_3$ | 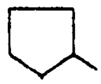 | H | H | $CH_3$- | 70 |
| 681 | $CH_3$ | $CH_3$ | 6-$CH_3$ | 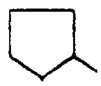 | H | H | $C_2H_5$- | 56 |
| 682 | $CH_3$ | $CH_3$ | 6-$CH_3$ | 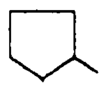 | H | H | $(CH_3)_2CH$- | 84 |
| 683 | $CH_3$ | $CH_3$ | 6-$CH_3$ | 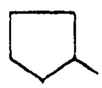 | H | H | $(CH_3)_2CH$-$CH_2$- | 69 |
| 684 | $CH_3$ | $CH_3$ | 6-$CH_3$ | 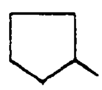 | H | H | $C_2H_5$<br>CH-<br>$CH_3$ | 64 |
| 685 | $CH_3$ | $CH_3$ | 6-$CH_3$ | 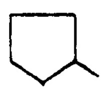 | H | H | $(CH_3)_3C$-$CH_2$- | 114 |
| 686 | $CH_3$ | $CH_3$ | 6-$CH_3$ | 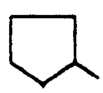 | H | H | 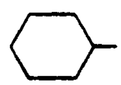 | |
| 687 | $CH_3$ | $CH_3$ | 6-$CH_3$ | 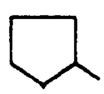 | H | H | 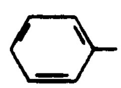 | |

Tabelle 3 (Fortsetzung)

| Bsp. Nr. | X | Y | $Z_n$ | A | B | C* | $R^1$ | Fp °C |
|---|---|---|---|---|---|---|---|---|
| 688 | $CH_3$ | $CH_3$ | 6-$CH_3$ | 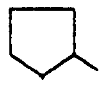 | $CH_3$- | H | $CH_3$- | |
| 689 | $CH_3$ | $CH_3$ | 6-$CH_3$ | 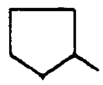 | $CH_3$- | H | $C_2H_5$- | |
| 690 | $CH_3$ | $CH_3$ | 6-$CH_3$ | 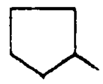 | $CH_3$- | H | $(CH_3)_2CH$- | |
| 691 | $CH_3$ | $CH_3$ | 6-$CH_3$ | 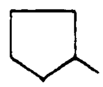 | $CH_3$- | H | $(CH_3)_2CH-CH_2$- | |
| 692 | $CH_3$ | $CH_3$ | 6-$CH_3$ | 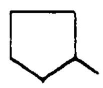 | $CH_3$- | H | $C_2H_5$ / $CH_3$ > CH- | |
| 693 | $CH_3$ | $CH_3$ | 6-$CH_3$ | 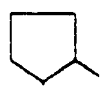 | $CH_3$- | H | $(CH_3)_3C-CH_2$- | |
| 694 | $CH_3$ | $CH_3$ | 6-$CH_3$ | 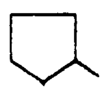 | $CH_3$- | H | 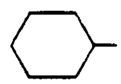 | |
| 695 | $CH_3$ | $CH_3$ | 6-$CH_3$ | 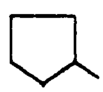 | $CH_3$- | H | 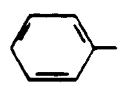 | |
| 696 | $CH_3$ | $CH_3$ | 6-$CH_3$ | 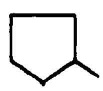 | $C_2H_5$- | H | $CH_3$- | |
| 697 | $CH_3$ | $CH_3$ | 6-$CH_3$ | 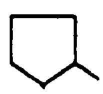 | $C_2H_5$- | H | $C_2H_5$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 698 | $CH_3$ | $CH_3$ | 6-$CH_3$ | 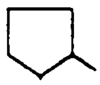 | $C_2H_5$- | H | $(CH_3)_2CH$- | |
| 699 | $CH_3$ | $CH_3$ | 6-$CH_3$ | 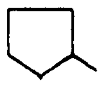 | $C_2H_5$- | H | $(CH_3)_2CH-CH_2$- | |
| 700 | $CH_3$ | $CH_3$ | 6-$CH_3$ | 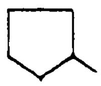 | $C_2H_5$- | H | $C_2H_5$\\<br>CH-<br>$CH_3$/ | |
| 701 | $CH_3$ | $CH_3$ | 6-$CH_3$ | 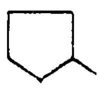 | $C_2H_5$- | H | $(CH_3)_3C-CH_2$- | |
| 702 | $CH_3$ | $CH_3$ | 6-$CH_3$ | 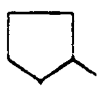 | $C_2H_5$- | H | 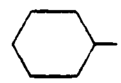 | |
| 703 | $CH_3$ | $CH_3$ | 6-$CH_3$ | 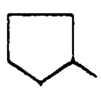 | $C_2H_5$- | H | 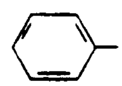 | |
| 704 | $CH_3$ | $CH_3$ | 6-$CH_3$ | 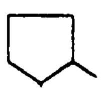 | $C_3H_7$- | H | $C_2H_5$- | |
| 705 | $CH_3$ | $CH_3$ | 6-$CH_3$ | 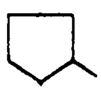 | $C_3H_7$- | H | $(CH_3)_2CH$- | |
| 706 | $CH_3$ | $CH_3$ | 6-$CH_3$ | 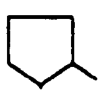 | $C_3H_7$- | H | $(CH_3)_2CH$-CH2- | |
| 707 | $CH_3$ | $CH_3$ | 6-$CH_3$ | 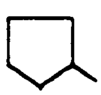 | $C_3H_7$- | H | $CH_3$\\<br>CH-<br>$C_2H_5$/ | |
| 708 | $CH_3$ | $CH_3$ | 6-$CH_3$ | 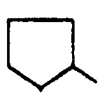 | $C_3H_7$- | H | $(CH_3)_3C$-CH2- | |

Tabelle 3 (Fortsetzung)

| Bsp. Nr. | X | Y | $Z_n$ | A | B | C* | $R^1$ | Fp °C |
|---|---|---|---|---|---|---|---|---|
| 709 | $CH_3$ | $CH_3$ | 6-$CH_3$ | 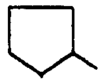 | $(CH_3)_2CH$- | H | C2$H_5$- | |
| 710 | $CH_3$ | $CH_3$ | 6-$CH_3$ | 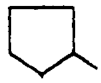 | $(CH_3)_2CH$- | H | $(CH_3)_2CH$- | |
| 711 | $CH_3$ | $CH_3$ | 6-$CH_3$ | 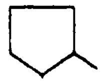 | $(CH_3)_2CH$- | H | $(CH_3)_2CH$-CH2- | |
| 712 | $CH_3$ | $CH_3$ | 6-$CH_3$ | 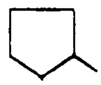 | $(CH_3)_2CH$- | H | $CH_3$<br>CH-<br>$C_2H_5$ | |
| 713 | $CH_3$ | $CH_3$ | 6-$CH_3$ | 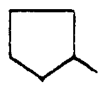 | $(CH_3)_2CH$- | H | $(CH_3)_3C$-CH2- | |
| 714 | $CH_3$ | $CH_3$ | 6-$CH_3$ | 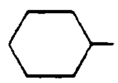 | H | H | $CH_3$- | |
| 715 | $CH_3$ | $CH_3$ | 6-$CH_3$ | 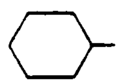 | H | H | $C_2H_5$- | |
| 716 | $CH_3$ | $CH_3$ | 6-$CH_3$ | 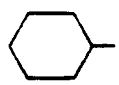 | H | H | $(CH_3)_2CH$- | |
| 717 | $CH_3$ | $CH_3$ | 6-$CH_3$ | 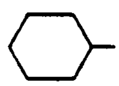 | H | H | $(CH_3)_2CH-CH_2$- | |
| 718 | $CH_3$ | $CH_3$ | 6-$CH_3$ | 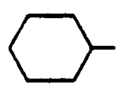 | H | H | $C_2H_5$<br>CH-<br>$CH_3$ | |

Tabelle 3 (Fortsetzung)

| Bsp. Nr. | X | Y | $Z_n$ | A | B | C* | $R^1$ | Fp °C |
|---|---|---|---|---|---|---|---|---|
| 719 | $CH_3$ | $CH_3$ | 6-$CH_3$ | 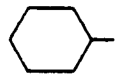 | H | H | $(CH_3)_3C-CH_2-$ | |
| 720 | $CH_3$ | $CH_3$ | 6-$CH_3$ | 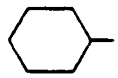 | H | H | 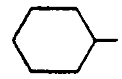 | |
| 721 | $CH_3$ | $CH_3$ | 6-$CH_3$ | 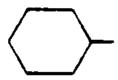 | H | H | 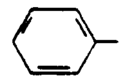 | |
| 722 | $CH_3$ | $CH_3$ | 6-$CH_3$ | 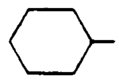 | $CH_3-$ | H | $CH_3-$ | |
| 723 | $CH_3$ | $CH_3$ | 6-$CH_3$ | 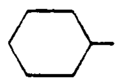 | $CH_3-$ | H | $C_2H_5-$ | |
| 724 | $CH_3$ | $CH_3$ | 6-$CH_3$ | 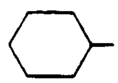 | $CH_3-$ | H | $(CH_3)_2CH-$ | |
| 725 | $CH_3$ | $CH_3$ | 6-$CH_3$ | 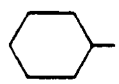 | $CH_3-$ | H | $(CH_3)_2CH-CH_2-$ | |
| 726 | $CH_3$ | $CH_3$ | 6-$CH_3$ | 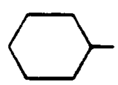 | $CH_3-$ | H | 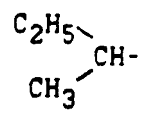 | |
| 727 | $CH_3$ | $CH_3$ | 6-$CH_3$ | 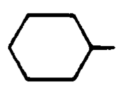 | $CH_3-$ | H | $(CH_3)_3C-CH_2-$ | |
| 728 | $CH_3$ | $CH_3$ | 6-$CH_3$ | 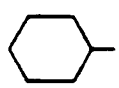 | $CH_3-$ | H | 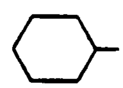 | |
| 729 | $CH_3$ | $CH_3$ | 6-$CH_3$ | 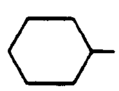 | $CH_3-$ | H | 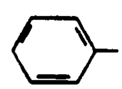 | |

Tabelle 3 (Fortsetzung)

| Bsp. Nr. | X | Y | $Z_n$ | A | B | C* | $R^1$ | Fp °C |
|---|---|---|---|---|---|---|---|---|
| 730 | $CH_3$ | $CH_3$ | 6-$CH_3$ | 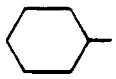 | $C_2H_5$- | H | $CH_3$- | |
| 731 | $CH_3$ | $CH_3$ | 6-$CH_3$ | 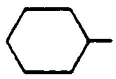 | $C_2H_5$- | H | $C_2H_5$- | |
| 732 | $CH_3$ | $CH_3$ | 6-$CH_3$ | 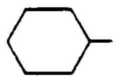 | $C_2H_5$- | H | $(CH_3)_2CH$- | |
| 733 | $CH_3$ | $CH_3$ | 6-$CH_3$ | 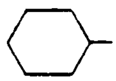 | $C_2H_5$- | H | $(CH_3)_2CH$-$CH_2$- | |
| 734 | $CH_3$ | $CH_3$ | 6-$CH_3$ | 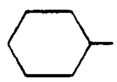 | $C_2H_5$- | H | $\begin{matrix}C_2H_5 \\ CH_3\end{matrix}\!>CH-$ | |
| 735 | $CH_3$ | $CH_3$ | 6-$CH_3$ | 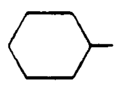 | $C_2H_5$- | H | $(CH_3)_3C$-$CH_2$- | |
| 736 | $CH_3$ | $CH_3$ | 6-$CH_3$ | 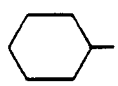 | $C_2H_5$- | H | 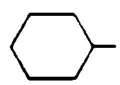 | |
| 737 | $CH_3$ | $CH_3$ | 6-$CH_3$ | 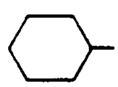 | $C_2H_5$- | H | 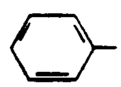 | |
| 738 | $CH_3$ | $CH_3$ | 6-$CH_3$ | 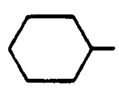 | $C_3H_7$- | H | $C_2H_5$- | |
| 739 | $CH_3$ | $CH_3$ | 6-$CH_3$ | 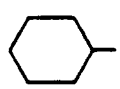 | $C_3H_7$- | H | $(CH_3)_2CH$- | |

Tabelle 3 (Fortsetzung)

| Bsp. Nr. | X | Y | $Z_n$ | A | B | C* | $R^1$ | Fp °C |
|---|---|---|---|---|---|---|---|---|
| 740 | $CH_3$ | $CH_3$ | 6-$CH_3$ | 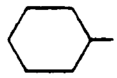 | $C_3H_7$- | H | $(CH_3)_2CH-CH_2$ | |
| 741 | $CH_3$ | $CH_3$ | 6-$CH_3$ | 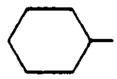 | $C_3H_7$- | H | 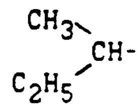 | |
| 742 | $CH_3$ | $CH_3$ | 6-$CH_3$ | 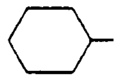 | $C_3H_7$- | H | $(CH_3)_3C-CH_2$- | |
| 743 | $CH_3$ | $CH_3$ | 6-$CH_3$ | 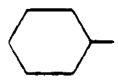 | $(CH_3)_2CH$- | H | $C_2H_5$- | |
| 744 | $CH_3$ | $CH_3$ | 6-$CH_3$ | 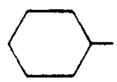 | $(CH_3)_2CH$- | H | $(CH_3)_2CH$- | |
| 745 | $CH_3$ | $CH_3$ | 6-$CH_3$ | 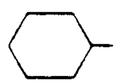 | $(CH_3)_2CH$- | H | $(CH_3)_2CH-CH_2$ | |
| 746 | $CH_3$ | $CH_3$ | 6-$CH_3$ | 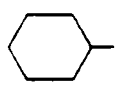 | $(CH_3)_2CH$- | H | 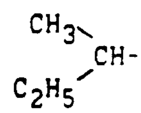 | |
| 747 | $CH_3$ | $CH_3$ | 6-$CH_3$ | 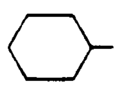 | $(CH_3)_2CH$- | H | $(CH_3)_3C-CH_2$- | |
| 748 | $CH_3$ | $CH_3$ | 6-$CH_3$ | $CH_3-O-(CH_2)_2$- | H | H | $C_2H_5$- | |
| 749 | $CH_3$ | $CH_3$ | 6-$CH_3$ | $CH_3-O-(CH_2)_2$- | H | H | $(CH_3)_2CH$- | |
| 750 | $CH_3$ | $CH_3$ | 6-$CH_3$ | $CH_3-O-(CH_2)_2$- | H | H | $(CH_3)_2CH-CH_2$ | |
| 751 | $CH_3$ | $CH_3$ | 6-$CH_3$ | $CH_3-O-(CH_2)_2$- | H | H | 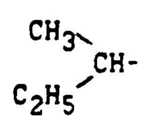 | |

Tabelle 3 (Fortsetzung)

| Bsp. Nr. | X | Y | $Z_n$ | A | B | C* | $R^1$ | Fp °C |
|---|---|---|---|---|---|---|---|---|
| 752 | $CH_3$ | $CH_3$ | 6-$CH_3$ | $CH_3$-O-$(CH_2)_2$- | H | H | $(CH_3)_3C$-$CH_2$- | |
| 753 | $CH_3$ | $CH_3$ | 6-$CH_3$ | $CH_3$-O-$(CH_2)_2$- | $CH_3$- | H | $C_2H_5$- | |
| 754 | $CH_3$ | $CH_3$ | 6-$CH_3$ | $CH_3$-O-$(CH_2)_2$- | $CH_3$- | H | $(CH_3)_2CH$- | |
| 755 | $CH_3$ | $CH_3$ | 6-$CH_3$ | $CH_3$-O-$(CH_2)_2$- | $CH_3$- | H | $(CH_3)_2CH$-$CH_2$- | 57 |
| 756 | $CH_3$ | $CH_3$ | 6-$CH_3$ | $CH_3$-O-$(CH_2)_2$- | $CH_3$- | H | $CH_3$<br>CH-<br>$C_2H_5$ | 54 |
| 757 | $CH_3$ | $CH_3$ | 6-$CH_3$ | $CH_3$-O-$(CH_2)_2$- | $CH_3$- | H | $(CH_3)_3C$-$CH_2$- | |
| 758 | $CH_3$ | $CH_3$ | 6-$CH_3$ | $CH_3$-O-$CH_2$-$CH(CH_3)$- | H | H | $C_2H_5$- | 60 |
| 759 | $CH_3$ | $CH_3$ | 6-$CH_3$ | $CH_3$-O-$CH_2$-$CH(CH_3)$- | H | H | $(CH_3)_2CH$- | |
| 760 | $CH_3$ | $CH_3$ | 6-$CH_3$ | $CH_3$-O-$CH_2$-$CH(CH_3)$- | H | H | $(CH_3)_2CH$-$CH_2$- | |
| 761 | $CH_3$ | $CH_3$ | 6-$CH_3$ | $CH_3$-O-$CH_2$-$CH(CH_3)$- | H | H | $CH_3$<br>CH-<br>$C_2H_5$ | |
| 762 | $CH_3$ | $CH_3$ | 6-$CH_3$ | $CH_3$-O-$CH_2$-$CH(CH_3)$- | H | H | $(CH_3)_3C$-$CH_2$- | |
| 763 | $CH_3$ | $CH_3$ | 6-$CH_3$ | $CH_3$-O-$CH_2$-$CH(CH_3)$- | $CH_3$- | H | $C_2H_5$- | |
| 764 | $CH_3$ | $CH_3$ | 6-$CH_3$ | $CH_3$-O-$CH_2$-$CH(CH_3)$- | $CH_3$- | H | $(CH_3)_2CH$- | |
| 765 | $CH_3$ | $CH_3$ | 6-$CH_3$ | $CH_3$-O-$CH_2$-$CH(CH_3)$- | $CH_3$- | H | $(CH_3)_2CH$-$CH_2$- | |
| 766 | $CH_3$ | $CH_3$ | 6-$CH_3$ | $CH_3$-O-$CH_2$-$CH(CH_3)$- | $CH_3$- | H | $CH_3$<br>CH-<br>$C_2H_5$ | |
| 767 | $CH_3$ | $CH_3$ | 6-$CH_3$ | $CH_3$-O-$CH_2$-$CH(CH_3)$- | $CH_3$- | H | $(CH_3)_3C$-$CH_2$- | |

Herstellung der Zwischenprodukte

Beispiel I

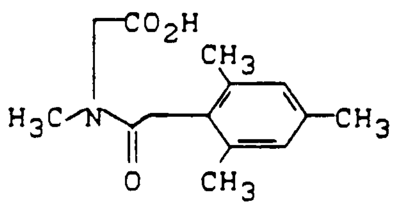

11,25 g (0,15 Mol) Sarkosin und 3 g (0,075 Mol) NaOH werden in 210 ml Wasser gelöst. Unter Wasserbadkühlung werden 9 g (0,225 Mol) NaOH, gelöst in 45 ml Wasser, und 29,6 g (0,15 Mol Mesitylenessigsäurechlorid synchron zugetropft, wobei die Temperatur auf < 40 °C gehalten wird. Nach 1 Stunde säuert man bei 0 bis 20 °C mit konz. HCl, saugt ab und trocknet im Vakuum bei 70 °C über $P_2O_5$. Es werden 37,1 g (99,3 % der Theorie N-(2.4.6-Trimethylphenyl-acetyl)-sarkosin vom Schmelzpunkt 140 °C erhalten.

Beispiel II

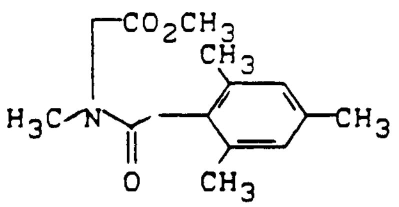

37,1 g (0,149 Mol) N-(2.4.6-Trimethylphenyl-acetyl)-sarkosin werden in 150 ml Methanol suspendiert, mit 22 ml (0,165 Mol) Dimethoxypropan versetzt und nach Zugabe von 1,43 g (7,5 mmol) p-Toluolsulfonsäuremonohydrat 3 Stunden unter Rückfluß erhitzt.

Nach Abdampfen des Lösungsmittels wird der Rückstand in $CH_2Cl_2$ aufgenommen, mit Bicarbonatlösung gewaschen, getrocknet und einrotiert. Man erhält 34 g (ca. 86,7% der Theorie) N-(2.4.6-Trimethylphenyl-acetyl)-sarkosin-methylester als hellgelbes Öl.

$^1$H-NMR(200 MHz, $CDCl_3$):

$\delta$ = 2,18, 2,2, 2,28 (s, 9H Ar-$CH_3$);
3,0, 3,2 (s, 3H, $NCH_3$),
3,64, 3,67 (s, 2H, $CH_2$-Ar),
3,66, 3,69 (s, 3H, $OCH_3$),
3,79, 4,14 (s, 2H, N-$CH_2$-CO),
6,82 (s, 2H, Ar 3-H, 5-H)

Beispiel III

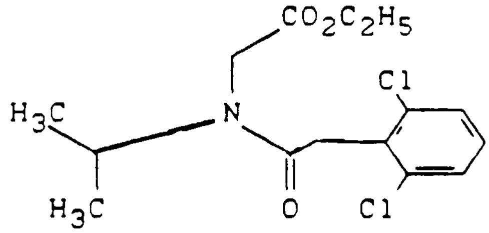

17,4 g (0,12 Mol) N-Isopropylglycin-ethylester werden in 180 ml abs. THF gelöst und mit 16,8 ml (0,12 Mol) Triethylamin versetzt. Bei 0 - 10° C werden 26,82 g (0,12 Mol) 2.6-Dichlorphenylessigsäurechlorid in 20 ml abs. THF zugetropft. Nach 1 Stunde rührt man in 1 l Eiswasser + 100 ml 1 NHCl ein, extrahiert mit $CH_2Cl_2$, trocknet und engt ein. Es werden 36,8 g (89,1 % der Theorie) eines gelben Öls erhalten.
$^1$H-NMR(200 MHz, $CDCl_3$):
δ = 1,11 - 1,32 (m, 9H $CH_2$-$CH_3$ $CH(CH_3)_2$),
7,08 - 7,15 (1H, m, Ar 4-H),
7,25 - 7,32 (m, 2H, Ar-3-H, 5-H).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter im Vor- und Nachlaufverfahren (Pre- und Postemergence) bei guter Kulturflanzenverträglichkeit aufweisen.

Die erfindungsgemäßen Wirkstoffe können z.B. bei den folgenden Pflanzen verwendet werden:
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z. B. Weisen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stofen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen,

Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Beispiel A

Nephotettix-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: (5), (54), (55), (56), (57), (58).

Die erfindungsgemäßen Verbindungen der Formel (I) weisen antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullenvorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat,Ethylacetat, Benzylalkohol, Benzylbenzoat, Propylenglykol, 1,3-Buty lenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichenund zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel B

Antimykotische in-vitro-Wirksamkeit

Versuchsbeschreibung:

Die in-vitro-Prüfungen wurden mit Keiminokula von durchschnittlich 1 x $10^4$ Keimen/ml Substrat durchgeführt. Als Nährmedium diente Yeast Nitrogen Base-Medium für Hefen und Kimmig-Medium für Schimmelpilze und Dermatophyten.

Die Bebrütungstemperatur betrug 37 °C bei Hefen und 28 °C bei Schimmelpilzen und Dermatophyten, die Bebrütungsdauer lag bei 24 bis 96 Stunden bei Hefen und 96 bis 120 Stunden bei Dermatophyten und Schimmelpilzen.

Die Beurteilung der Fungizide erfolgt durch Ausplattieren und erneutes Bebrüten voll gehemmter Ansätze, wobei fungizide Konzentrationen weniger als 100 Keime c.f.n. (colony forming unit) pro ml enthalten.

In diesem Test zeigen die erfindungsgemäßen Verbindungen der Formel (I) gemäß den Herstellungsbeispielen 45, 46, 47 eine stark ausgeprägte antimykotische Wirksamkeit.

## Patentansprüche

**1.** 3-Aryl-pyrrolidin-2,4-dion-Derivate der allgemeinen Formel (I)

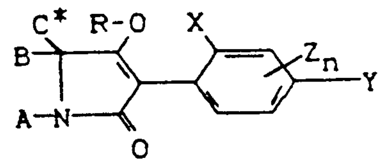

(I)

in welcher

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für 0 oder 1 steht,

R für Wasserstoff (Ia) oder für die Gruppen der Formel

$-CO-R^1$ (Ib) oder $-CO-O-R^2$ (Ic)

steht, in welchen

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,

für gegebenenfalls durch Halogen-, Nitro-, $C_1$-$C_6$-Alkyl-, $C_1$-$C_6$-Alkoxy-, $C_1$-$C_6$-Halogenalkyl-, $C_1$-$C_6$-Halogenalkoxysubstituiertes Phenyl;

für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht,

$R^2$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,

für gegebenenfalls durch Halogen-, Nitro-, $C_1$-$C_6$-Alkyl-, $C_1$-$C_6$-Alkoxy-, $C_1$-$C_6$-Halogenalkyl-substituiertes Phenyl oder Cycloalkyl mit 3-8 Ringatomen steht,

A für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann

oder gegebenenfalls durch Halogen, $C_1$-$C_6$-Alkyl-$C_1$-$C_6$-Haloalkyl-, $C_1$-$C_6$-Alkoxy-, Nitro substituiertes Aryl-$C_1$-$C_6$-alkyl steht,

B, C* unabhängig voneinander für Wasserstoff, geradkettiges oder verzweigtes $C_1$-$C_{12}$-Alkyl, $C_1$-$C_8$-Alkoxyalkyl steht,

sowie die enantiomerenreinen Formen von Verbindungen der Formel (I).

**2.** 3-Aryl-pyrrolidin-2,4-dion-Derivat der allgemeinen Formel (I) gemäß Anspruch 1, in welcher

X für $C_1$-$C_4$-Alkyl, Halogen, $C_1$-$C_4$-Alkoxy steht,

Y für Wasserstoff, $C_1$-$C_6$-Alkyl, Halogen, $C_1$-$C_4$-Alkoxy, $C_1$-$C_2$-Halogenalkyl steht,

Z für $C_1$-$C_4$-Alkyl, Halogen, $C_1$-$C_4$-Alkoxy steht,

n für 0 oder 1 steht,

R für Wasserstoff (Ia) oder für die Gruppen der Formel

-CO-$R^1$ (Ib) oder -CO-O-$R^2$ (Ic)

steht, in welchen

$R^1$ für gegebenenfalls durch Halogen substituiertes $C_1$-$C_{16}$-Alkyl, $C_2$-$C_{16}$-Alkenyl, $C_1$-$C_6$-Alkoxy-$C_2$-$C_6$-alkyl, $C_1$-$C_6$-Alkylthio-$C_2$-$C_6$-alkyl, $C_1$-$C_6$-Polyalkoxy-$C_2$-$C_6$-alkyl und Cycloalkyl mit 3-7 Ringatomen, das durch 1-2 Sauerstoff-und/oder Schwefelatome unterbrochen sein kann steht,

für gegebenenfalls durch Halogen-, Nitro-, $C_1$-$C_4$-Alkyl-, $C_1$-$C_4$-Alkoxy-, $C_1$-$C_3$-Halogenalkyl-, $C_1$-$C_3$-Halogenalkoxy-substituiertes Phenyl steht,

für gegebenenfalls durch Halogen-, $C_1$-$C_4$-Alkyl-, $C_1$-$C_4$-Alkoxy-, $C_1$-$C_3$-Halogenalkyl-, $C_1$-$C_3$-Halogenalkoxy-substituiertes Phenyl-$C_1$-$C_4$-alkyl steht,

$R^2$ für gegebenenfalls durch Halogen substituiertes $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht,

für gegebenenfalls durch Halogen, Nitro-, $C_1$-$C_4$-Alkyl, $C_1$-$C_3$-Alkoxy-, $C_1$-$C_3$-Halogenalkyl-substituiertes Phenyl oder Cycloalkyl mit 3-7 Ringatomen steht,

A für gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen, das durch 1-2 Sauerstoff- und/oder Schwefelatome unterbrochen sein kann oder geebenenfalls durch Halogen-, $C_1$-$C_4$-Alkyl-, $C_1$-$C_4$-Halogenalkyl-$C_1$-$C_4$-Alkoxy-, Nitro substituiertes Aryl-$C_1$-$C_4$-alkyl steht,

B, C* unabhängig voneinander für Wasserstoff, geradkettiges oder verzweigtes $C_1$-$C_{10}$-Alkyl, $C_1$-$C_6$-Alkoxyalkyl steht,

sowie die enantiomerenreinen Formen von Verbindungen der Formel (I).

**3.** 3-Aryl-pyrrolidin-2,4-dion-Derivate der allgemeinen Formel (I) gemäß Anspruch 1, in welcher

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,

Z für Methyl, Ethyl, i-Propyl, Butyl, i-Butyl, tert.-Butyl, Fluor, Chlor, Brom, Methoxy und Ethoxy steht,

n für 0 oder 1 steht,

R für Wasserstoff (Ia) oder für die Gruppen der Formel

-CO-$R^1$ (Ib) oder -CO-O-$R^2$ (Ic)

steht, in welcher

$R^1$ für gegebenenfalls durch Fluor oder Chlor substituiertes: $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann steht,

für gegebenenfalls durch Fluor-, Chlor, Brom-, Methyl-, Ethyl-, Propyl, i-Propyl, Methoxy, Ethoxy, Trifluormethyl-, Trifluormethoxy-, Nitro- substituiertes Phenyl steht,

für gegebenenfalls durch Fluor-, Chlor-, Brom-, Methyl-, Ethyl-, Propyl-, i-Propyl, Methoxy, Ethoxy, Trifluormethyl, Trifluormethoxy-substituiertes Phenyl-$C_1$-$C_3$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht

oder

für gegebenenfalls durch Fluor-, Chlor-, Nitro-, Methyl-, Ethyl-, Propyl-, i-Propyl-, Methoxy-, Ethoxy-, Trifluormethyl-substituiertes Phenyl oder Cycloalkyl mit 3 bis 6 Ringatomen steht,

A für gegebenenfalls durch Halogen substituiertes geradkettiges oder verzweigtes $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen, das durch 1-2 Sauerstoff- und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro susbtituiertes Aryl-$C_1$-$C_3$-alkyl steht,

B, C* unabhängig voneinander für Wasserstoff, geradkettiges oder verzweigtes $C_1$-$C_8$-Alkyl, $C_1$-$C_4$-Alkoxyalkyl steht,

sowie die enantiomerenreinen Formen von Verbindungen der Formel I.

**4.** Verfahren zur Herstellung von 3-Aryl-pyrrolidin-2,4-dion-Derivaten der allgemeinen Formel (I) gemäß Anspruch 1

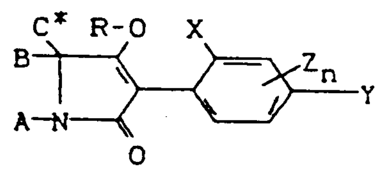

(I)

in welcher

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für 0 oder 1 steht,

R für Wasserstoff (Ia) oder für die Gruppen der Formel

$-CO-R^1$ (Ib) oder $-CO-O-R^2$ (Ic)

steht, in welchen

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,
für gegebenenfalls durch Halogen-, Nitro-, $C_1$-$C_6$-Alkyl-, $C_1$-$C_6$-Alkoxy-, $C_1$-$C_6$-Halogenalkyl-, $C_1$-$C_6$-Halogenalkoxy-substituiertes Phenyl;
für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht,

$R^2$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen-, Nitro-, $C_1$-$C_6$-Alkyl-, $C_1$-$C_6$-Alkoxy-, $C_1$-$C_6$-Halogenalkyl-substituiertes Phenyl oder Cycloalkyl mit 3-8 Ringatomen steht,

A für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann
oder gegebenenfalls durch Halogen, $C_1$-$C_6$-Alkyl-$C_1$-$C_6$-Haloalkyl-, $C_1$-$C_6$-Alkoxy-, Nitro substituiertes Aryl-$C_1$-$C_6$-alkyl steht,

B, C* unabhängig voneinander für Wasserstoff, geradkettiges oder verzweigtes $C_1$-$C_{12}$-Alkyl, $C_1$-$C_8$-Alkoxyalkyl steht,

dadurch gekennzeichnet,

(A) daß man zum Erhalt der Verbindungen der Formel (Ia)

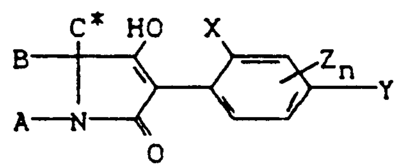

(Ia)

N-Acylaminosäureester der Formel (II)

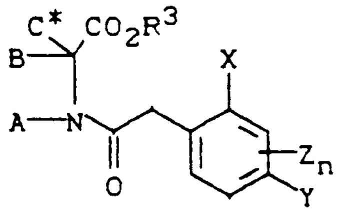

(II)

in welcher
A, B, C*, X, Y, Z und n die oben angegebene Bedeutung haben
und
$R^3$ für Alkyl steht,
in Gegenwart eines Verdünnungsmittels und in Gegenwart einer Base intramolekular kondensiert,
(B) oder, daß man zum Erhalt von Verbindungen der Formel (Ib)

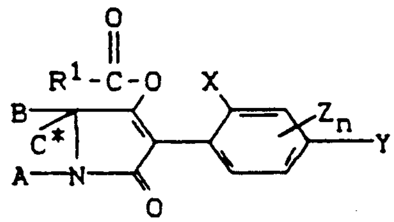

(Ib)

Verbindungen der Formel (Ia),

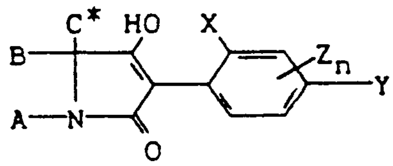

(Ia)

in welcher
A, B, C*, X, Y, Z und n die oben angegebene Bedeutung haben,
entweder
α) mit Säurehalogeniden der allgemeinen Formel (III)

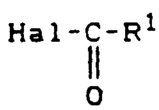

(III)

in welcher
$R^1$ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels,
oder
β) mit Carbonsäureanhydriden der allgemeinen Formel (IV)

$R^1$-CO-O-CO-$R^1$ (IV)

in welcher
$R^1$ die oben angegebene Bedeutung hat,
gegebenenfalls in Gegenwart eines Verdünnungsmittels und gegebenenfalls in Gegenwart eines

Säurebindemittels,
umsetzt,
(C) oder, daß man zum Erhalt von Verbindungen der Formel (Ic)

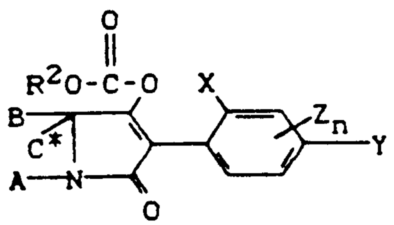

(Ic)

in welcher
A, B, C*, X, Y, Z, $R^2$ und n die oben angegebene Bedeutung haben,
Verbindungen der Formel (Ia)

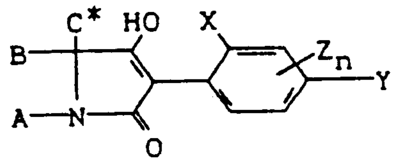

(Ia)

in welcher
A, B, C*, X, Y, Z und n die oben angegebene Bedeutung haben
mit Chlorameisensäureester der allgemeinen Formel (V)

$R^2$-O-CO-Cl (V)

in welcher

$R^2$ die oben angegebene Bedeutung hat,
gegebenenfals in Gegenwart eines Verdünnungsmittels und gegebenenfalls in Gegenwart eines Säurebindemittels umsetzt.

**5.** Insektizide und/oder akarizide und/oder herbizide Mittel, gekennzeichnet durch einen Gehalt an mindestens einem 3-Aryl-pyrrolidin-2,4-dion-Derivat der Formel (I) gemäß Ansprüchen 1-3.

**6.** Verwendung von 3-Aryl-pyrrolidin-2,4-dion-Derivaten der Formel (I) gemäß Ansprüchen 1-3 zur Bekämpfung von Insekten und/oder Spinnentieren und/oder Unkräutern.

**7.** Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) gemäß Ansprüchen 1-3 mit Streckmitteln und/oder oberflächenaktiven Mitteln vermischt.

**8.** 3-Aryl-pyrrolidin-2,4-dion-Derivate gemäß Ansprüchen 1 bis 3 zur Bekämpfung von Mykosen.

**9.** Verwendung von 3-Aryl-pyrrolidin-2,4-dion-Derivaten gemäß Ansprüchen 1 bis 3 bei der Herstellung von Arzneimitteln zur Bekämpfung von Mykosen.

## Claims

**1.** 3-Aryl-pyrrolidine-2,4-dione derivatives of the general formula (I)

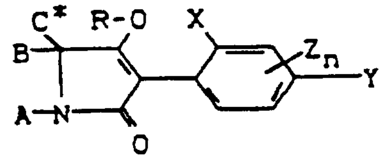

(I)

in which

X represents $C_1$-$C_6$-alkyl, halogen or $C_1$-$C_6$-alkoxy,

Y represents hydrogen, $C_1$-$C_6$-alkyl, halogen, $C_1$-$C_6$-alkoxy or $C_1$-$C_3$-halogenoalkyl,

Z represents $C_1$-$C_6$-alkyl, halogen or $C_1$-$C_6$-alkoxy,

n represents 0 or 1,

R represents hydrogen (Ia) or the groups of the formula

-CO-$R^1$ (Ib) or -CO-O-$R^2$ (Ic)

in which

$R^1$ represents optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-alkylthio-$C_2$-$C_8$-alkyl, $C_1$-$C_8$-polyalkoxy-$C_2$-$C_8$-alkyl and cycloalkyl having 3-8 ring atoms, which can be interrupted by oxygen and/or sulphur,
or represents optionally halogen-, nitro-, $C_1$-$C_6$-alkyl-, $C_1$-$C_6$-alkoxy-, $C_1$-$C_6$-halogenoalkyl- or $C_1$-$C_6$-halogenoalkoxy-substituted phenyl;
or represents optionally halogen-, $C_1$-$C_6$-alkyl-, $C_1$-$C_6$-alkoxy-, $C_1$-$C_6$-halogenoalkyl-or $C_1$-$C_6$-halogenoalkoxy-substituted phenyl -$C_1$-$C_6$-alkyl,

$R^2$ represents optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-polyalkoxy-$C_2$-$C_8$-alkyl,
or represents optionally halogen-, nitro-, $C_1$-$C_6$-alkyl-, $C_1$-$C_6$-alkoxy- or $C_1$-$C_6$-halogenoalkyl-substituted phenyl or cycloalkyl having 3-8 ring atoms,

A represents optionally halogen-substituted, straight-chain or branched $C_1$-$C_{12}$-alkyl, $C_3$-$C_8$-alkenyl, $C_3$-$C_8$-alkinyl, $C_1$-$C_{10}$-alkoxy-$C_2$-$C_8$-alkyl, $C_1$-$C_8$-polyalkoxy-$C_2$-$C_8$-alkyl, $C_1$-$C_{10}$-alkylthio-$C_2$-$C_8$-alkyl, cycloalkyl having 3-8 ring atoms, which can be interrupted by oxygen and/or sulphur,
or represents optionally halogen-, $C_1$-$C_6$-alkyl-$C_1$-$C_6$-haloalkyl-, $C_1$-$C_6$-alkoxy- or nitro-substituted aryl-$C_1$-$C_6$-alkyl,

B and C* independently of one another represent hydrogen or straight-chain or branched $C_1$-$C_{12}$-alkyl or $C_1$-$C_8$-alkoxyalkyl,
and the enantiomerically pure forms of compounds of the formula (I).

**2.** 3-Aryl-pyrrolidine-2,4-dione derivative of the general formula (I) according to Claim 1, in which

X represents $C_1$-$C_4$-alkyl, halogen or $C_1$-$C_4$-alkoxy,

Y represents hydrogen, $C_1$-$C_6$-alkyl, halogen, $C_1$-$C_4$-alkoxy or $C_1$-$C_2$-halogenoalkyl,

Z represents $C_1$-$C_4$-alkyl, halogen or $C_1$-$C_4$-alkoxy,

n represents 0 or 1,

R represents hydrogen (Ia) or the groups of the formula

-CO-$R^1$ (Ib) or -CO-O-$R^2$ (Ic)

in which

$R^1$ represents optionally halogen-substituted $C_1$-$C_{16}$-alkyl, $C_2$-$C_{16}$-alkenyl, $C_1$-$C_6$-alkoxy-$C_2$-$C_6$-alkyl, $C_1$-$C_6$-alkylthio-$C_2$-$C_6$-alkyl, $C_1$-$C_6$-polyalkoxy-$C_2$-$C_6$-alkyl and cycloalkyl having 3-7 ring atoms, which can be interrupted by 1-2 oxygen and/or sulphur atoms,
or represents optionally halogen-, nitro-, $C_1$-$C_4$-alkyl-, $C_1$-$C_4$-alkoxy-, $C_1$-$C_3$-halogenoalkyl- or $C_1$-$C_3$-halogenoalkoxy-substituted phenyl,
or represents optionally halogen-, nitro-, $C_1$-$C_4$-alkyl-, $C_1$-$C_4$-alkoxy-, $C_1$-$C_3$-

halogenoalkyl- or $C_1$-$C_3$-halogenoalkoxy-substituted phenyl-

$R^2$ represents optionally halogen-substituted $C_1$-$C_{16}$-alkyl, $C_2$-$C_{16}$-alkenyl, $C_1$-$C_{16}$-alkoxy-$C_2$-$C_6$-alkyl, $C_1$-$C_6$-polyalkoxy-$C_2$-$C_6$-alkyl, or represents optionally halogen-, nitro-, $C_1$-$C_4$-alkyl-, $C_1$-$C_3$-alkoxy- or $C_1$-$C_3$-halogenoalkyl-substituted phenyl or cycloalkyl having 3-7 ring atoms,

A represents optionally halogen-substituted, straight-chain or branched $C_1$-$C_{10}$-alkyl, $C_3$-$C_6$-alkenyl, $C_3$-$C_6$-alkinyl, $C_1$-$C_8$-alkoxy-$C_2$-$C_6$-alkyl, $C_1$-$C_6$-polyalkoxy-$C_2$-$C_6$-alkyl,$C_1$-$C_8$-alkylthio-$C_2$-$C_6$-alkyl, cycloalkyl having 3-7 ring atoms, which can be interrupted by 1-2 oxygen and/or sulphur atoms, or represents optionally halogen-, $C_1$-$C_4$-alkyl-, $C_1$-$C_4$-halogenoalkyl-$C_1$-$C_4$-alkoxy- or nitro-substituted aryl-$C_1$-$C_4$-alkyl,

B and C* independently of one another represent hydrogen or straight-chain or branched $C_1$-$C_{10}$-alkyl or $C_1$-$C_6$-alkoxyalkyl,

and the enantiomerically pure forms of compounds of the formula (I).

**3.** 3-Aryl-pyrrolidine-2,4-dione derivatives of the general formula (I) according to Claim 1, in which

X represents methyl, ethyl, propyl, i-propyl, fluorine, chlorine, bromine, methoxy and ethoxy,

Y represents hydrogen, methyl, ethyl, propyl, i-propyl, butyl, i-butyl, tert.-butyl, fluorine, chlorine, bromine, methoxy, ethoxy and trifluoromethyl,

Z represents methyl, ethyl, i-propy, butyl, i-butyl, tert.-butyl, fluorine, chlorine, bromine, methoxy and ethoxy,

n represents 0 or 1

R represents hydrogen (Ia) or the groups of the formula

-CO-$R^1$ (Ib) or -CO-O-$R^2$ (Ic)

in which

$R^1$ represents optionally fluorine- or chlorine-substituted: $C_1$-$C_{14}$-alkyl, $C_2$-$C_{14}$-alkenyl, $C_1$-$C_4$-alkoxy-$C_2$-$C_6$-alkyl, $C_1$-$C_4$-alkylthio-$C_2$-$C_6$-alkyl, $C_1$-$C_4$-polyalkoxyl-$C_2$-$C_4$-alkyl and cycloalkyl having 3-6 ring atoms, which can be interrupted by 1-2 oxygen and/or sulphur atoms, or represents optionally fluorine-, chlorine-, bromine-, methyl-, ethyl-, propyl-, i-propyl-, methoxy-, ethoxy-, trifluoromethyl-, trifluoromethoxy- or nitro-substituted phenyl, or represents optionally fluorine-, chlorine-, bromine-, methyl-, ethyl-, propyl-, i-propyl-, methoxy-, ethoxy-, trifluoromethyl-, or trifluoromethoxy-substituted phenyl-$C_1$-$C_3$-alkyl,

$R^2$ represents optionally fluorine- or chlorine-substituted $C_1$-$C_{14}$-alkyl, $C_2$-$C_{14}$-alkenyl, $C_1$-$C_4$-alkoxy-$C_2$-$C_6$-alkyl or $C_1$-$C_4$-polyalkoxy-$C_2$-$C_6$-alkyl, or represents optionally fluorine-, chlorine-, nitro-, methyl-, ethyl-, propyl-, i-propyl-, methoxy-, ethoxy- or trifluoromethyl-substituted phenyl or cycloalkyl having 3 to 6 ring atoms,

A represents optionally halogen-substituted, straight-chain or branched $C_1$-$C_8$-alkyl, $C_3$-$C_4$-alkenyl, $C_3$-$C_4$-alkinyl, $C_1$-$C_6$-alkoxy-$C_2$-$C_4$-alkyl, $C_1$-$C_4$-polyalkoxy-$C_2$-$C_4$-alkyl, $C_1$-$C_6$-alkylthio-$C_2$-$C_4$-alkyl, cycloalkyl having 3-6 ring atoms, which can be interrupted by 1-2 oxygen and/or sulphur atoms, or represents optionally fluorine-, chlorine-, bromine-, methyl-, ethyl-, propyl-, i-propyl-, methoxy-, ethoxy-, trifluoromethyl- or nitro-substituted aryl-$C_1$-$C_3$-alkyl,

B and C* independently of one another represent hydrogen or straight-chain or branched $C_1$-$C_8$-alkyl or $C_1$-$C_4$-alkoxyalkyl, and the enantiomerically pure forms of compounds of the formula I.

**4.** Process for the preparation of 3-aryl-pyrrolidine-2,4-dione derivatives of the general formula (I) according to Claim 1

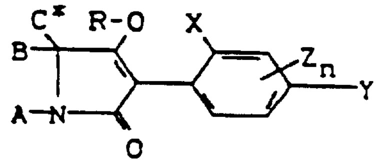 (I)

in which

X represents $C_1$-$C_6$-alkyl, halogen or $C_1$-$C_6$-alkoxy,

Y represents hydrogen, $C_1$-$C_6$-alkyl, halogen, $C_1$-$C_6$-alkoxy or $C_1$-$C_3$-halogenoalkyl,

Z represents $C_1$-$C_6$-alkyl, halogen or $C_1$-$C_6$-alkoxy,

n represents 0 or 1

R represents hydrogen (Ia) or the groups of the formula

-CO-$R^1$ (Ib) or -CO-O-$R^2$ (Ic)

in which

$R^1$ represents optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-alkylthio-$C_2$-$C_8$-alkyl, $C_1$-$C_8$-polyalkoxy-$C_2$-$C_8$-alkyl and cycloalkyl having 3-8 ring atoms, which can be interrupted by oxygen and/or sulphur,
or represents optionally halogen-, nitro-, $C_1$-$C_6$-alkyl-, $C_1$-$C_6$-alkoxy-, $C_1$-$C_6$-halogenoalkyl- or $C_1$-$C_6$-halogenoalkoxy-substituted phenyl;
or represents optionally halogen-, $C_1$-$C_6$-alkyl-, $C_1$-$C_6$-alkoxy-, $C_1$-$C_6$-halogenoalkyl- or $C_1$-$C_6$-halogenoalkoxy-substituted phenyl -$C_1$-$C_6$-alkyl,

$R^2$ represents optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-polyalkoxy-$C_2$-$C_8$-alkyl,
or represents optionally halogen-, nitro-, $C_1$-$C_6$-alkyl-, $C_1$-$C_6$-alkoxy- or $C_1$-$C_6$-halogenoalkyl-substituted phenyl or cycloalkyl having 3-8 ring atoms,

A represents optionally halogen-substituted, straight-chain or branched $C_1$-$C_{12}$-alkyl, $C_3$-$C_8$-alkenyl, $C_3$-$C_8$-alkinyl, $C_1$-$C_{10}$-alkoxy-$C_2$-$C_8$-alkyl, $C_1$-$C_8$-polyalkoxy-$C_2$-$C_8$-alkyl, $C_1$-$C_{10}$-alkylthio-$C_2$-$C_8$-alkyl, cycloalkyl having 3-8 ring atoms, which can be interrupted by oxygen and/or sulphur,
or represents optionally halogen-, $C_1$-$C_6$-alkyl-$C_1$-$C_6$-haloalkyl-, $C_1$-$C_6$-alkoxy- or nitro-substituted aryl-$C_1$-$C_6$-alkyl, and

B and C* independently of one another represent hydrogen or straight-chain or branched $C_1$-$C_{12}$-alkyl or $C_1$-$C_8$-alkoxyalkyl,

characterized in that,

(A) to obtain the compounds of the formula (Ia),

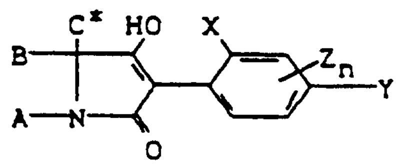 (Ia)

N-acylamino acid esters of the formula (II)

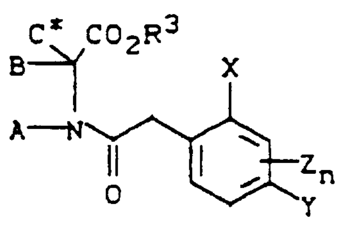 (II)

in which

A, B, C*, X, Y, Z and n have the abovementioned meaning,

and

$R^3$ represents alkyl,

are subjected to an intramolecular condensation reaction in the presence of a diluent and in the presence of a base,

(B) or in that, to obtain compounds of the formula (Ib)

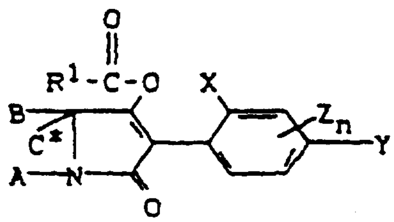

(Ib)

compounds of the formula (Ia)

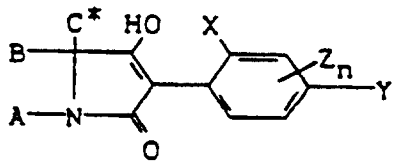

(Ia)

in which

A, B, C*, X, Y, Z and n have the abovementioned meaning,

are reacted either

α) with acid halides of the general formula (III)

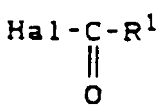

(III)

in which

$R^1$ has the abovementioned meaning

and

Hal represents halogen, in particular chlorine and bromine,

if appropriate in the presence of a diluent and if appropriate in the presence of an acid-binding agent,

or

β) with carboxylic anhydrides of the general formula (IV)

$R^1$-CO-O-CO-$R^1$ (IV)

in which

$R^1$ has the abovementioned meaning,

if appropriate in the presence of a diluent, and if appropriate in the presence of an acid-binding agent,

(C) or in that, to obtain compounds of the formula (Ic)

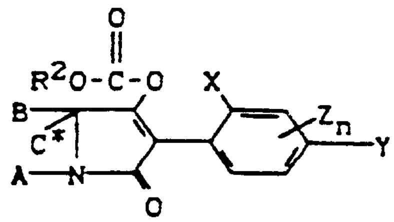

(Ic)

in which
A, B, C*, X, Y, Z, $R^2$ and n have the abovementioned meaning,
compounds of the formula (Ia)

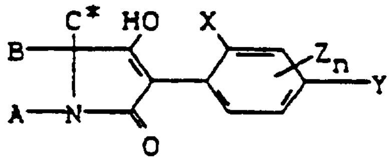

(Ia)

in which
A, B, C*, X, Y, Z and n have the abovementioned meaning,
are reacted with chloroformic ester of the general formula (V)

$R^2$-O-CO-Cl (V)

in which
$R^2$ has the abovementioned meaning,
if appropriate in the presence of a diluent and if appropriate in the presence of an acid-binding agent.

**5.** Insecticidal and/or acaricidal and/or herbicidal agents, characterized in that they contain at least one 3-aryl-pyrrolidine-2,4-dione derivative of the formula (I) according to Claims 1-3.

**6.** Use of 3-aryl-pyrrolidine-2,4-dione derivatives of the formula (I) according to Claims 1-3 for combating insects and/or arachnids and/or weeds.

**7.** Process for the preparation of insecticidal and/or acaricidal and/or herbicidal agents, characterized in that 3-aryl-pyrrolidine-2,4-dione derivatives of the formula (I) according to Claims 1-3 are mixed with extenders and/or surface-active agents.

**8.** 3-Aryl-pyrrolidine-2,4-dione derivatives according to Claims 1 to 3 for combating mycoses.

**9.** Use of 3-aryl-pyrrolidine-2,4-dione derivatives according to Claims 1 to 3 in the preparation of medicaments for combating mycoses.

## Revendications

**1.** Dérivés de 3-aryl-pyrrolidine-2,4-diones répondant à la formule I

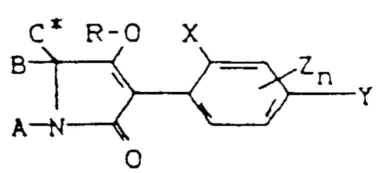

(I)

dans laquelle

X représente un groupe alkyle en $C_1$-$C_6$, un halogène un groupe alcoxy en $C_1$-$C_6$,

Y représente l'hydrogène, un groupe alkyle en $C_1$-$C_6$, un halogène, un groupe alcoxy en $C_1$-$C_6$, halogénoalkyle en $C_1$-$C_3$,

Z représente un groupe alkyle en $C_1$-$C_6$, un halogène, un groupe alcoxy en $C_1$-$C_6$,

n est égal à 0 ou 1

R représente l'hydrogène (Ia) ou les groupes de formules

$-CO-R^1$ (Ib) ou $-CO-O-R^2$ (Ic)

dans lesquelles

$R^1$ représente les groupes suivants, éventuellement substitués par des halogènes : alkyle en $C_1$-$C_{20}$, alcényle en $C_2$-$C_{20}$, (alcoxy en $C_1$-$C_8$)-alkyle en $C_2$-$C_8$, (alkylthio en $C_1$-$C_8$)-alkyle en $C_2$-$C_8$, (polyalcoxy en $C_1$-$C_8$)-alkyle en $C_2$-$C_8$, et cycloalkyle contenant 3 à 8 atomes cycliques et éventuellement interrompu par l'oxygène et/ou le soufre,
un groupe phényle éventuellement substitué par des halogènes, des groupes nitro, alkyle en $C_1$-$C_6$, alcoxy en $C_1$-$C_6$, halogénoalkyle en $C_1$-$C_6$ , halogénalcoxy en $C_1$-$C_6$;
un groupe phényl-alkyle en $C_1$-$C_6$ éventuellement substitué par des halogènes, des groupes alkyle en $C_1$-$C_6$, alcoxy en $C_1$-$C_6$, halogénoalkyle en $C_1$-$C_6$, halogénoalcoxy en $C_1$-$C_6$,

$R^2$ représente les groupes suivants, éventuellement substitués par des halogènes : alkyle en $C_1$-$C_{20}$, alcényle en $C_2$-$C_{20}$, (alcoxy en $C_1$-$C_8$)-alkyle en $C_2$-$C_8$, (polyalcoxy en $C_1$-$C_8$)-alkyle en $C_2$-$C_8$, un groupe phényle ou cycloalkyle de 3 à 8 atomes cycliques éventuellement substitué par des halogènes, des groupes nitro, alkyle en $C_1$-$C_6$, alcoxy en $C_1$-$C_6$, halogénoalkyle en $C_1$-$C_6$,

A représente un groupe alkyle en $C_1$-$C_{12}$ à chaîne droite ou ramifiée éventuellement substitué par des halogènes, alcényle en $C_3$-$C_8$, alcynyle en $C_3$-$C_8$, (alcoxy en $C_1$-$C_{10}$)-alkyle en $C_2$-$C_8$, (polyalcoxy en $C_1$-$C_8$)-alkyle en $C_2$-$C_8$, (alkylthio en $C_1$-$C_{10}$)-alkyle en $C_2$-$C_8$, cycloalkyle contenant 3 à 8 atomes cycliques et éventuellement interrompu par l'oxygène et/ou le soufre, ou bien un groupe aryl-alkyle en $C_1$-$C_6$ éventuellement substitué par des halogènes, des groupes alkyle en $C_1$-$C_6$, halogénoalkyle en $C_1$-$C_6$, alcoxy en $C_1$-$C_6$, nitro,

B, C* représentent chacun, indépendamment l'un de l'autre, l'hydrogène, un groupe alkyle en $C_1$-$C_{12}$ à chaîne droite ou ramifiée, un groupe alcoxyalkyle en $C_1$-$C_8$,

ainsi que les formes énantiomères pures des composés de formule I.

**2.** Dérivé de 3-aryl-pyrrolidine-2,4-dione de formule générale I de la revendication 1, dans laquelle

X représente un groupe alkyle en $C_1$-$C_4$, un halogène un groupe alcoxy en $C_1$-$C_4$,

Y représente l'hydrogène, un groupe alkyle en $C_1$-$C_6$, un halogène, un groupe alcoxy en $C_1$-$C_4$, halogénoalkyle en $C_1$-$C_2$,

Z représente un groupe alkyle en $C_1$-$C_4$, un halogène, un groupe alcoxy en $C_1$-C4,

n est égal à 0 ou 1

R représente l'hydrogène (Ia) ou les groupes de formules

$-CO-R^1$ (Ib) ou $-CO-O-R^2$ (Ic)

dans lesquelles

$R^1$ représente les groupes suivants, éventuellement substitués par des halogènes : alkyle en $C_1$-$C_{16}$, alcényle en $C_2$-$C_{16}$, (alcoxy en $C_1$-$C_6$)-alkyle en $C_2$-$C_6$, (alkylthio en $C_1$-$C_6$)-alkyle en $C_2$-$C_6$, (polyalcoxy en $C_1$-$C_6$)-alkyle en $C_2$-$C_6$, et cycloalkyle contenant 3 à 7 atomes cycliques et éventuellement interrompu par 1 à 2 atomes d'oxygène et/ou de soufre,
un groupe phényle éventuellement substitué par des halogènes, des groupes nitro, alkyle en $C_1$-$C_4$, alcoxy en $C_1$-$C_4$, halogénoalkyle en $C_1$-$C_3$,halogénalcoxy en $C_1$-$C_3$;
un groupe phényl-alkyle en $C_1$-$C_4$ éventuellement substitué par des halogènes, des groupes alkyle en $C_1$-$C_4$, alcoxy en $C_1$-$C_4$, halogénoalkyle en $C_1$-$C_3$, halogénoalcoxy en $C_1$-$C_3$,

$R^2$ représente un groupe alkyle en $C_1$-$C_{16}$, éventuellement substitué par des halogènes, alcényle en $C_2$-$C_{16}$, (alcoxy en $C_1$-$C_{16}$)-alkyle en $C_2$-$C_6$, (polyalcoxy en $C_1$-$C_6$)-alkyle en

$C_2$-$C_6$, un groupe phényle ou cycloalkyle contenant 3 à 7 atomes cycliques éventuellement substitué par des halogènes, des groupes nitro, alkyle en $C_1$-$C_4$, alcoxy en $C_1$-$C_3$, halogénoalkyle en $C_1$-$C_3$,

A représente les groupes suivants, éventuellement substitués par des halogènes : alkyle à chaîne droite ou ramifiée en $C_1$-$C_{10}$, alcényle en $C_3$-$C_6$, alcynyle en $C_3$-$C_6$, (alcoxy en $C_1$-$C_8$)-alkyle en $C_2$-$C_6$, (polyalcoxy en $C_1$-$C_6$)-alkyle en $C_2$-$C_6$, (alkylthio en $C_1$-$C_8$)-alkyle en $C_2$-$C_6$, cycloalkyle contenant 3 à 7 atomes cycliques et éventuellement interrompu par 1 ou 2 atomes d'oxygène et/ou de soufre,
ou un groiupe aryl-alkyle en $C_1$-$C_4$ éventuellement substitué par des halogènes, des groupes alkyle en $C_1$-$C_4$, halogénoalkyle en $C_1$-$C_4$, alcoxy en $C_1$-$C_4$, nitro,

B, C* représentent chacun, indépendamment l'un de l'autre, l'hydrogène, un groupe alkyle à chaîne droite ou ramifiée en $C_1$-$C_{10}$, alcoxyalkyle en $C_1$-$C_6$,

ainsi que les formes énantiomères pures des composés de formule I.

**3.** Dérivés de 3-aryl-pyrrolidine-2,4-diones de formule générale I de la revendication 1, dans laquelle

X représente un groupe méthyle, éthyle, propyle, isopropyle, le fluor, le chlore, le brome, un groupe méthoxy ou éthoxy,

Y représente l'hydrogène, un groupe méthyle, éthyle, propyle, isopropyle, butyle, isobutyle, tert-butyle, le fluor, le chlore, le brome, un groupe méthoxy, éthoxy ou trifluorométhyle,

Z représente un groupe méthyle, éthyle, isopropyle, butyle, isobutyle, tert-butyle, le fluor, le chlore, le brome, un groupe méthoxy ou éthoxy,

n est égal à 0 ou 1,

R représente l'hydrogène (Ia) ou les groupes de formules

$-CO-R^1$ (Ib) ou $-CO-O-R^2$ (Ic)

dans lesquelles

$R^1$ représente les groupes suivants, éventuellement substitués par le fluor ou le chlore : alkyle en $C_1$-$C_{14}$) alcényle en $C_2$-$C_{14}$, (alcoxy en $C_1$-$C_4$)-alkyle en $C_2$-$C_6$, (alkylthio en $C_1$-$C_4$)-alkyle en $C_2$-$C_6$, (polyalcoxy en $C_1$-$C_4$)-alkyle en $C_2$-$C_4$, et cycloalkyle contenant 3 à 6 atomes cycliques et éventuellement interrompu par 1 à 2 atomes d'oxygène et/ou de soufre,
un groupe phényle éventuellement substitué par le fluor, le chlore, le brome, des groupes méthyle, éthyle, propyle, isopropyle, méthoxy, éthoxy, trifluorométhyle, trifluorométhoxy, nitro,
un groupe phényl-alkyle en $C_1$-$C_3$ éventuellement substitué par le fluor, le chlore, le brome, des groupes méthyle, éthyle, propyle, isopropyle, méthoxy, éthoxy, trifluorométhyle, trifluorométhoxy,

$R^2$ représente un groupe alkyle en $C_1$-$C_{14}$, éventuellement substitué par le fluor ou le chlore, un groupe alcényle en $C_2$-$C_{14}$, (alcoxy en $C_1$-$C_4$)-alkyle en $C_2$-$C_6$, (polyalcoxy en $C_1$-$C_4$)-alkyle en $C_2$-$C_6$,
ou bien un groupe phényle ou cycloalkyle de 3 à 6 atomes cycliques, éventuellement substitué par le fluor, le chlore, des groupes nitro, méthyle, éthyle, propyle, isopropyle, méthoxy, éthoxy, trifluorométhyle,

A représente un groupe alkyle en $C_1$-$C_8$ à chaîne droite ou ramifiée éventuellement substitué par des halogènes, un groupe alcényle en $C_3$-$C_4$, alcynyle en $C_3$-$C_4$, (alcoxy en $C_1$-$C_6$)-alkyle en $C_2$-$C_4$, (polyalcoxy en $C_1$-$C_4$)alkyle en $C_2$-$C_4$, (alkylthio en $C_1$-$C_6$)-alkyle en $C_2$-$C_4$, cycloalkyle contenant 3 à 6 atomes cycliques et éventuellement interrompu par 1 à 2 atomes d'oxygène et/ou de soufre, ou bien un groupe aryl-alkyle en $C_1$-$C_3$ éventuellement substitué par le fluor, le chlore, le brome, des groupes méthyle, éthyle, propyle, isopropyle, méthoxy, éthoxy, trifluorométhyle, nitro,

B,C* représentent chacun, indépendamment l'un de l'autre, l'hydrogène, un groupe alkyle à chaîne droite ou ramifiée en $C_1$-$C_8$, un groupe alcoxyalkyle en $C_1$-$C_4$,

ainsi que les formes énantiomères pures des composés de formule I.

**4.** Procédé de préparation des dérivés de 3-aryl-pyrrolidine-2,4-diones de formule générale I de la revendication 1

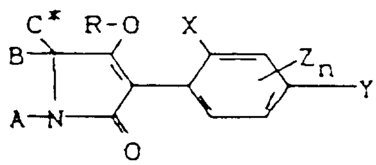

(I)

dans laquelle

X représente un groupe alkyle en $C_1$-$C_6$, un halogène un groupe alcoxy en $C_1$-$C_6$,

Y représente l'hydrogène, un groupe alkyle en $C_1$-$C_6$, un halogène, un groupe alcoxy en $C_1$-$C_6$, halogénoalkyle en $C_1$-$C_3$,

Z représente un groupe alkyle en $C_1$-$C_6$, un halogène, un groupe alcoxy en $C_1$-$C_6$,

n est égal à 0 ou 1

R représente l'hydrogène (Ia) ou les groupes de formules

$-CO-R^1$ (Ib) ou $-CO-O-R^2$ (Ic)

dans lesquelles

$R^1$ représente les groupes suivants, éventuellement substitués par des halogènes : alkyle en $C_1$-$C_{20}$, alcényle en $C_2$-$C_{20}$, (alcoxy en $C_1$-$C_8$)-alkyle en $C_2$-$C_8$, (alkylthio en $C_1$-$C_8$)-alkyle en $C_2$-$C_8$, (polyalcoxy en $C_1$-$C_8$)-alkyle en $C_2$-$C_8$, et cycloalkyle contenant 3 à 8 atomes cycliques et éventuellement interrompu par l'oxygène et/ou le soufre,
un groupe phényle éventuellement substitué par des halogènes, des groupes nitro, alkyle en $C_1$-$C_6$, alcoxy en $C_1$-$C_6$, halogénoalkyle en $C_1$-$C_6$ , halogénalcoxy en $C_1$-$C_6$;
un groupe phényl-alkyle en $C_1$-$C_6$ éventuellement substitué par des halogènes, des groupes alkyle en $C_1$-$C_6$, alcoxy en $C_1$-$C_6$, halogénoalkyle en $C_1$-$C_6$, halogénoalcoxy en $C_1$-$C_6$,

$R^2$ représente les groupes suivants, éventuellement substitués par des halogènes : alkyle en $C_1$-$C_{20}$, alcényle en $C_2$-$C_{20}$, (alcoxy en $C_1$-$C_8$)-alkyle en $C_2$-$C_8$, (polyalcoxy en $C_1$-$C_8$)-alkyle en $C_2$-$C_8$,
un groupe phényle ou cycloalkyle de 3 à 8 atomes cycliques éventuellement substitué par des halogènes, des groupes nitro, alkyle en $C_1$-$C_6$, alcoxy en $C_1$-$C_6$, halogénoalkyle en $C_1$-$C_6$,

A représente un groupe alkyle en $C_1$-$C_{12}$ à chaîne droite ou ramifiée éventuellement substitué par des halogènes, alcényle en $C_3$-$C_8$, alcynyle en $C_3$-$C_8$, (alcoxy en $C_1$-$C_{10}$)-alkyle en $C_2$-$C_8$, (polyalcoxy en $C_1$-$C_8$)-alkyle en $C_2$-$C_8$, (alkylthio en $C_1$-$C_{10}$)-alkyle en $C_2$-$C_8$, cycloalkyle contenant 3 à 8 atomes cycliques et éventuellement interrompu par l'oxygène et/ou le soufre, ou bien un groupe aryl-alkyle en $C_1$-$C_6$ éventuellement substitué par des halogènes, des groupes alkyle en $C_1$-$C_6$, halogénoalkyle en $C_1$-$C_6$, alcoxy en $C_1$-$C_6$, nitro,

B, C* représentent chacun, indépendamment l'un de l'autre, l'hydrogène, un groupe alkyle en $C_1$-$C_{12}$ à chaîne droite ou ramifiée, un groupe alcoxyalkyle en $C_1$-$C_8$,

caractérisé en ce que :

A) pour obtenir les composés de formule Ia

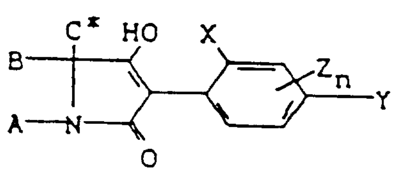

(Ia)

on soumet des esters de N-acylaminoacides de formule II

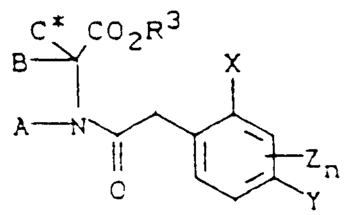

(II)

dans laquelle A, B, C*, X, Y, Z et n ont les significations indiquées ci-dessus, et $R^3$ représente un groupe alkyle,
à condensation intramoléculaire en présence d'un diluant et d'une base,
B) ou bien, pour obtenir les composés de formule Ib

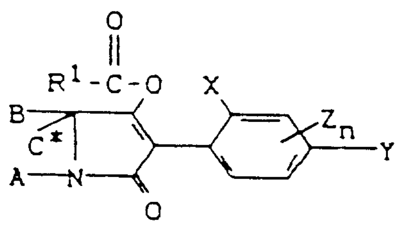

(Ib)

on fait réagir des composés de formule Ia

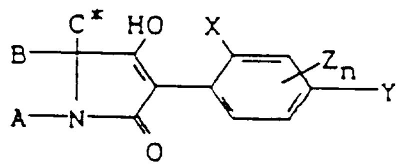

(Ia)

dans laquelle A, B, C*, X, Y, Z et n ont les significations indiquées ci-dessus,
α) soit avec des halogénures d'acides de formule générale III

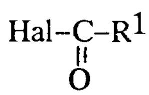

(III)

dans laquelle $R^1$ a les significations indiquées ci-dessus, et
Hal représente un halogène, en particulier le chlore ou le brome,
éventuellement en présence d'un diluant et éventuellement en présence d'un accepteur d'acide,
β) soit avec des anhydrides d'acides carboxyliques de formule IV

$R^1$-CO-O-CO-$R^1$ (IV)

dans laquelle $R^1$ a les significations indiquées ci-dessus, éventuellement en présence d'un diluant et éventuellement en présence d'un accepteur d'acide,

C) ou bien, pour obtenir des composés de formule Ic

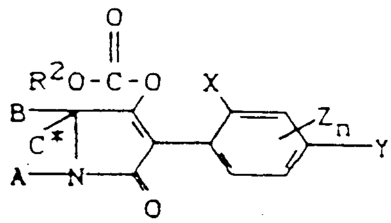

(Ic)

dans laquelle A, B, C*, X, Y, Z, $R^2$ et n ont les significations indiquées ci-dessus, on fait réagir des composés de formule Ia

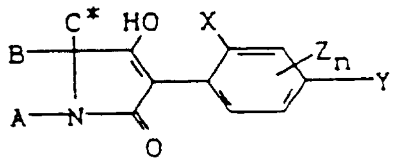

(Ia)

dans laquelle A, B, C*, X, Y, Z et n ont les significations indiquées ci-dessus, avec des esters chloroformiques de formule générale V

$R^2$-O-CO-Cl (V)

dans laquelle $R^2$ a les significations indiquées ci-dessus,
éventuellement en présence d'un diluant et éventuellement en présence d'un accepteur d'acide.

**5.** Produits insecticides et/ou acaricides et/ou herbicides, caractérisés en ce qu'ils contiennent au moins un dérivé de 3-aryl-pyrrolidine-2,4-dione de formule I selon les revendications 1 à 3.

**6.** Utilisation des dérivés de 3-aryl-pyrrolidine-2,4-diones de formule I selon les revendications 1 à 3 pour la lutte contre les insectes et/ou les arachnides et/ou les végétaux adventices.

**7.** Procédé de préparation de produits insecticides et/ou acaricides et/ou herbicides, caractérisé en ce que l'on mélange des dérivés de 3-aryl-pyrrolidine-2,4-diones de formule I selon les revendications 1 à 3 avec des diluants et/ou des agents tensioactifs.

**8.** Les dérivés de 3-aryl-pyrrolidine-2,4-diones selon les revendications 1 à 3, pour le traitement des mycoses.

**9.** Utilisation des dérivés de 3-aryl-pyrrolidine-2,4-diones selon les revendications 1 à 3 pour la préparation de médicaments servant eux-mêmes au traitement des mycoses.